(19) Europäisches Patentamt / European Patent Office / Office européen des brevets

(11) **EP 4 265 732 A1**

(12) **EUROPEAN PATENT APPLICATION**
published in accordance with Art. 153(4) EPC

(43) Date of publication:
**25.10.2023 Bulletin 2023/43**

(21) Application number: **21908808.5**

(22) Date of filing: **21.10.2021**

(51) International Patent Classification (IPC):
***C12Q 1/68*** $^{(2018.01)}$

(52) Cooperative Patent Classification (CPC):
**C12Q 1/68; C12Q 1/6858; G16B 15/30;
G16B 20/20; G16B 20/40**

(86) International application number:
**PCT/CN2021/125359**

(87) International publication number:
**WO 2022/134807 (30.06.2022 Gazette 2022/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **21.12.2020 CN 202011514641**

(71) Applicant: **Gao, Song
Wuhan, Hubei 430079 (CN)**

(72) Inventor: **Gao, Song
Wuhan, Hubei 430079 (CN)**

(74) Representative: **Kutzenberger Wolff & Partner
Waidmarkt 11
50676 Köln (DE)**

(54) **METHOD FOR DETECTING FETAL GENETIC VARIATIONS BY SEQUENCING POLYMORPHIC SITES AND TARGET SITES**

(57) Provided is a method for the non-invasive detection of fetal genetic variations, comprising: firstly, estimating the percentage of fetal genetic material in a plasma sample of a pregnant woman by means of carrying out targeted sequencing on polymorphic sites on a reference genome and then performing allelic copy counting for each polymorphic site; and then, carrying out allelic copy counting on the polymorphic sites on a target genome or a target to be detected, and using a goodness-of-fit test or a relative distribution diagram of allele counts to detect whether the target to be detected in the sample has any variation at the chromosomal level, sub-chromosomal level or single genetic site level. The method is suitable for simultaneously detecting chromosomal euploidy variations, micro-deletion and micro-duplication variations at the sub-chromosomal level and variations at the short sequence level in the plasma sample of the pregnant woman, and has good development and application prospects.

EP 4 265 732 A1

**Description**

**Technical Field**

The invention relates to the field of genetic variation detection, especially aneuploidy variations at the chromosome level, micro-deletion/micro-duplication variations at the sub-chromosomal level or indel and single-nucleotide site variations at the short-sequence level.

**Background Technique**

In 1997, cell-free DNA of fetal origin was found in maternal plasma (Lo, Corbetta et al. 1997, Lancet 350:485-487). Based on this discovery and massively parallel sequencing, multiple research groups have developed methods based on sequencing analysis of maternal plasma DNA (cfDNA) to detect chromosomal aneuploidy variations, micro-deletion/micro-duplication variations at the sub-chromosomal level, or short sequence indel and single-nucleotide site variations at the single-gene level (Advani, Barrett et al. 2017, Prenat Diagn 37:1067-1075; Breveglieri, D'Aversa et al. 2019, Mol Diagn Ther 23:291-299; Andari, Bussamra et al. 2020, Ceska Gynekol 85:41-48; Guseh 2020, Hum Genet 139:1141-1148).

At present, the detection of chromosomal aneuploidy abnormalities using next-generation sequencing has been recognized and industrialized in many countries around the world due to its high sensitivity and specificity (Chiu, Chan et al. 2008, Proc Natl Acad Sci U S A 105:20458-20463; Fan, Blumenfeld et al. 2008, Proc Natl Acad Sci U S A 105:16266-16271; Liao, Chan et al. 2012, PLoS One 7:e38154; Zimmermann, Hill et al. 2012, Prenat Diagn 32:1233-1241). However, for micro-deletion/micro-duplication variations at the sub-chromosomal level, the sensitivity and specificity of noninvasive detection methods are not very high, especially for micro-deletion/micro-duplication variations of small fragments (Advani, Barrett et al. 2017, Prenat Diagn 37:1067-1075; Hu, Wang et al. 2019, Human Genomics 13:14; Srebniak, Knapen et al. 2020, Mol Genet Genomic Med 8:e1062). Although a variety of non-invasive detection methods for monogenic diseases based on next-generation sequencing have been developed (Lun, Tsui et al. 2008, Proc Natl Acad Sci U S A 105:19920-19925; Lo, Chan et al. 2010, Sci Transl Med 2:61ra91; Lv, Wei et al. 2015, Clinical Chemistry 61:172-181; Vermeulen, Geeven et al. 2017, Am J Hum Genet 101:326-339; Allen, Young et al. 2018, Noninvasive Prenatal Testing (NIPT) 157-177; Yin, Du et al. 2018, J Hum Genet 63:1129-1137; Cutts, Vavoulis et al. 2019, Blood 134:1190-1193; Zhang, Li et al. 2019, Nat Med 25:439-447), these methods have not been widely used in clinical practice, mainly because these methods use methods different from those of detection of variations at the chromosomal or sub-chromosomal level, so they cannot be used to detect variations both at the chromosome and sub-chromosomal levels. At the same time, the cost of these methods for the detection of each monogenic disease is very high, resulting in low cost-effectiveness of using these methods to screen low-prevalence monogenic diseases.

Therefore, a general method that can use maternal plasma DNA to simultaneously detect fetal chromosomal, sub-chromosomal, and single-gene short-sequence variations will be of great benefit to the non-invasive detection of fetal genetic variation.

**Summary of the Invention**

The present invention aims to provide a method for simultaneously detecting chromosomal aneuploidy genetic diseases, micro-deletion/micro-duplication genetic diseases at the sub-chromosomal level and monogenic diseases caused by short sequence variations.

In order to achieve the above aim, the present invention designs a method for genetic variation screening based on high-throughput sequencing technology, including obtaining test samples and extracting DNA, selectively amplifying target sites, performing high-throughput sequencing on target sites, and analyzing the sequencing data to obtain the detection result.

The present invention provides a method for detecting genetic variations, which comprises the following steps:

(1) receiving a biological sample to be tested and preparing nucleic acids;
(2) enriching or amplifying target DNA sites, wherein at least one of the target DNA sites has more than one allele in the sample;
(3) sequencing the amplified target DNA sites;
(4) for each target DNA site, counting the counts of its individual alleles; and
(5) determining the karyotype or genotype or wild-mutant type of the target to be detected in the sample by using a goodness-of-fit test of allele counts and/or a relative distribution diagram of allele counts for target DNA sites.

The present invention provides the detection of aneuploidy at the chromosome level, the detection of micro-deletions/micro-duplications at the sub-chromosomal level, and the detection of variation in short sequence fragments in mixed samples through the amplification and sequencing of specific target DNA sites, wherein at least one of said specific target DNA sites has more than one allele in the sample.

The target DNA site in the present invention refers to a specific DNA sequence, in which the bases may vary in different individuals, and which can be amplified by techniques such as PCR, multiplex PCR, or enriched by techniques such as nucleic acid hybridization. In the present invention, the terms "target DNA sequence" and "target DNA site" can be used interchangeably, and the term "site" when referring to a target does not limit the length of the target, i.e. the length of the target can be a single nucleotide acid to the length of the entire chromosome.

In another aspect, the present invention provides the detection of aneuploidy at the chromosome level, and micro-deletions/micro-duplications at the sub-chromosomal level in a single genome sample through the amplification and sequencing of specific DNA sites (target sites), wherein at least one of said specific target DNA sites has more than one allele in the sample.

The biological sample in the present invention includes fetal and maternal nucleic acids from the biological sample of a pregnant female (such as cell-free DNA in maternal plasma) or from a single genomic sample (such as an embryonic nucleic acid from preimplantation diagnosis).

The enrichment or amplification of target DNA sites described in the present invention can be carried out by any method known in the art to enrich or amplify target DNA sites, including but not limited to using PCR, multiplex PCR, whole genome amplification (WGA), multiple substitution amplification (MDA), rolling circle amplification (RCA), circular amplification (RCR), hybrid capture and other methods to enrich or amplify target DNA sites. Among the enriched or amplified target DNA sites, some are derived from regions of one or more chromosomes that are assumed to be normal euploid, and some are derived from regions of one or more chromosomes that are suspected to have variations at the chromosomal, sub-chromosomal, or short-sequence level to be assayed. A chromosome or region or site assumed to be normal euploid is also designated herein as a "reference chromosome or reference region or reference sequence or reference site"; and a chromosome or region or site assumed to be the one for which the genetic variation status is to be detected is also designated herein as a "target chromosome or target region or target sequence or target site". In the present invention, a set consisting of not less than a or one reference chromosome or reference region or reference sequence or reference site is called a reference group. In the present invention, a set consisting of not less than a or one target chromosome or target region or target sequence or target site is called a target group.

According to the present invention, for each target DNA site, counting the counts of individual alleles means that for each amplified sequence, it is first mapped to the position of the chromosome or genome, and finally the number of sequences mapped to each chromosome or genome region is counted. If there are different alleles in a certain chromosome or genome region, the number of sequences mapped to each allele in the region will be counted at the same time. Various in silico methods are available for mapping individual sequence reads to chromosome or genome locations/regions. Non-limiting examples of computer algorithms that can be used to map sequences include, but are not limited to, search for specific sequences, BLAST, BLITZ, FASTA, BOWTIE, BOWTIE 2, BWA, NOVOALIGN, GEM, ZOOM, ELAN, MAQ, MATCH, SOAP, STAR, SEGEMEHL, MOSAIK or SEQMAP or variants or combinations thereof.

In the present invention, for ease of understanding, a micro-deletion fragment at the sub-chromosomal level is considered as one chromosome, and a micro-duplication fragment at the sub-chromosomal level is considered as two chromosomes. Therefore, for a single-genome sample, chromosomes with heterozygous micro-deletions at the sub-chromosomal level are marked as monosomy, chromosomes with homozygous micro-deletions are marked as nullisomy, chromosomes with heterozygous micro-duplications are marked as trisomy, and chromosomes with homozygous micro-duplications are marked as tetrasomy. Correspondingly, in a mixed sample, such as in a plasma sample of a pregnant woman, a chromosome wherein both the mother and the fetus are normal is marked as disomy-disomy, a chromosome wherein the mother is normal, while the fetus has a micro-deletion in one chromosome is marked as disomy-monosomy, and a chromosome wherein the mother is normal, while the fetus has a micro-duplication in one chromosome is marked as a disomy-trisomy. In the present invention, chromosomes and/or chromosome fragments involving variations at the chromosome level or sub-chromosomal level are marked according to a similar principle.

In the present invention, micro-deletion/micro-duplication at the sub-chromosomal level refers to a chromosomal aberration wherein a fragment that is deleted or added in a chromosome is not very long and difficult to find through traditional cytogenetic analysis. Chromosomal micro-deletion-micro-duplication syndrome is another major type of neonatal birth defects besides chromosomal aneuploidy. In the present invention, some sections also use the copy number variation of chromosomal fragments to refer to chromosomal micro-deletion/micro-duplication variation.

In the present invention, karyotype is used to refer to variation at the chromosomal or sub-chromosomal level, and genotype is used to refer to variation at the short sequence level. For example, for a plasma sample of a pregnant woman, if the chromosome 21 of the mother is a normal disomy and that of the fetus is a trisomy, the present invention will mark the chromosome 21 karyotype in the sample as a disomy-trisomy karyotype. For example, for a plasma sample of a pregnant woman, if the mother has a 22q11 micro-deletion on one chromosome 22 and no 22q11 micro-deletion on the other chromosome 22, and the fetus has a 22q11 micro-deletion on one chromosome 22 and no 22q11 micro-deletion on the other chromosome 22, the present invention will mark the karyotype of the 22q11 chromosome fragment in the sample as monosomy-monosomy karyotype. For example, for a plasma sample of a pregnant woman, if the mother has a 22q11 micro-duplication on one chromosome 22 and no 22q11 micro-duplication on the other chromosome 22, and the fetus has a 22q11 micro-duplication on one chromosome 22 and no 22q11 micro-duplication on the other chromosome 22, the present invention will mark the karyotype of the 22q11 chromosome fragment in the sample as trisomy-trisomy karyotype. For example, for a plasma sample of a pregnant woman, if the alleles of the position 6 amino acid of the mother's hemoglobin β subunit are A and S, respectively, and the alleles of the position 6 amino acid of the fetal hemoglobin β subunit are S and C, respectively, the present invention will mark the genotype of the position 6 amino acid of the hemoglobin β subunit in this sample as AS|SC type, where the part before the vertical line represents the genotype of the mother and the part after the vertical line represents the genotype of the fetus. In the present invention, wild-type is used to refer to the genotype with the highest frequency observed at a target locus in a normal population without a diseased phenotype. Wild-type, on the other hand, refers to a genotype that does not contain a pathogenic or likely pathogenic variant at the target site. In the present invention, mutant type is used to refer to a genotype whose target site is different from that of wild-type.

In the present invention, the concentration of the least component DNA in the sample is estimated by using the allele counts of individual target sites in the reference group for some samples to be tested. Among them, the concentration of the least component DNA in the sample to be tested can be estimated by any method that has been reported so far. Preferably, a relative ratio method using allele counts of individual target sites in the reference group is used to estimate the concentration of the least component DNA in the sample to be tested; preferably, the iterative fitting genotype method of allele counts of individual target sites in the reference group is used to estimate the concentration of the least component DNA in the sample; preferably, the concentration of the least component DNA in the sample is calculated by using the mean and/or median of FC and TC.

In the present invention, the concentration of the least component DNA in the sample is calculated by using a relative ratio method of allele counts. For example, for a maternal plasma DNA sample, the least component DNA is fetal DNA, while the most component DNA is maternal DNA. In a plasma DNA sample of a normal pregnant woman, the fetus inherits one chromosome from the mother, so the genotype of each target site can only be one of the following five possible genotypes, namely AA|AA, AA|AB, AB|AA, AB|AB, or AB|AC, where A, B, and C represent the individual alleles of the target site. Among the five genotypes, if the target site is of AA|AA or AB|AB genotype, the fetal DNA concentration does not affect the relative count of each allele, while if the target site is of AA|AB, AB|AA or AB|AC genotype, the count of each allele is affected by the concentration of fetal DNA. Thus, the genotypes AA|AB, AB|AA and AB|AC can be used to estimate the fetal DNA-derived count (FC) at each target DNA site.

The present invention provides a method for calculating a concentration of the least component DNA in a sample using a relative ratio of allele counts of individual target sites in the reference group, the method comprising:

(a1) setting a noise threshold $\alpha$ of the sample;
(a2) for each target DNA site, firstly using counts of its individual alleles to estimate its genotype, and then estimating the count (FC) derived from the least component DNA and total count (TC) based on its estimated genotype;
(a3) using the count (FC) of the least component DNA and total count (TC) for each target site to estimate the concentration of the least component DNA.

Further, setting the noise threshold $\alpha$ of the sample in the above step (a1) is to set the threshold for distinguishing the count signal of the real allele from the false allele count signal; preferably, the noise threshold $\alpha$ as set is any value not greater than 0.05; preferably, the noise threshold $\alpha$ as set is 0.05, 0.04, 0.03, 0.02, 0.01, 0.0075, 0.005, 0.0025 or 0.001.

Further, in the above step (a2), for each target DNA site, firstly using counts of its individual alleles to estimate its genotype, and then estimating the count (FC) derived from the least component DNA and total count (TC) based on its estimated genotype, comprises the following steps:

(a2-i) sorting the allele counts of the target DNA site in descending order, wherein the maximal three allele counts are marked as R1, R2 and R3 in sequence;
(a2-ii) estimating the genotype of the target DNA site using counts of individual alleles for the target DNA site;
(a2-iii) based on the estimated genotype of the target DNA site and the individual allele counts for the target DNA site, estimating the count (FC) derived from the least component DNA and total count (TC).

Further, in the above step (a2-ii), estimating the genotype of the target DNA site using counts of individual alleles for the target DNA site, wherein the maximal three allele counts are marked as R1, R2, and R3 in sequence, comprises the following steps:

(a2-ii-1) using the counts of individual alleles for the target DNA site to determine the number of alleles that are detected to be higher than the noise threshold in the target DNA site; if the determination result is 1, performing the following step (a2-ii-2); if the determination result is 2, performing the following step (a2-ii-3); if the determination result is greater than 2, performing the following step (a2-ii-4);

(a2-ii-2) estimating the genotype of the target DNA site as AA|AA, and then performing the following step (a2-ii-5);

(a2-ii-3) estimating the genotype of the target DNA site based on the number, that is 2, of alleles detected to be higher than the noise threshold and the maximal two allele counts for the target DNA site, and then performing the following step (a2-ii-5);

(a2-ii-4) estimating the genotype of the target DNA site based on the number, that is greater than 2, of alleles detected to be higher than the noise threshold and at least two maximal allele counts for the target DNA site, and then performing the following step (a2-ii-5);

(a2-ii-5) outputting the estimated genotype of the target site.

Further, in the above step (a2-ii-1), using the counts of individual alleles for the target DNA site to determine the number of alleles that are detected to be higher than the noise threshold in the target DNA site, comprises the following steps in sequence:

(a2-ii-1-1) calculating relative counts for each allele at target DNA sites;

(a2-ii-1-2) determining whether the relative count of each allele is higher than the noise threshold as set, and then counting the number of alleles which are higher than the noise threshold as set.

Among them, the relative count for an allele is the quotient of the count for that allele and the counts for all alleles at that target site. Preferably, the noise threshold $\alpha$ as set is any value not greater than 0.05; preferably, the predetermined noise threshold is 0.05, 0.04, 0.02, 0.01, 0.0075, 0.005, 0.0025 or 0.001.

Further, in the above step (a2-ii-3), estimating the genotype of the target DNA site based on the number, that is 2, of alleles detected to be higher than the noise threshold and the maximal two allele counts for the target DNA site, wherein the maximal two allele counts are marked as R1 and R2, respectively, comprises the following steps:

(a2-ii-3-1) determining whether the value of R1/(R1+R2) is less than $0.5+\alpha$, and if the determination result is yes, estimating the genotype of the target DNA site as AB|AB, and then performing the following step (a2-ii-3-3); if the determination result is no, then performing the following step (a2-ii-3-2);

(a2-ii-3-2) determining whether the value of R1/(R1+R2) is less than 0.75, and if the determination result is yes, estimating the genotype of the target DNA site as ABIAA, and then performing the following step (a2-ii-3-3); if the determination result is no, estimating the genotype of the target DNA site as AA|AB, and then performing the following step (a2-ii-3-3);

(a2-ii-3-3) outputting the estimated genotype of the target site.

Further, in the above step (a2-ii-4), estimating the genotype of the target DNA site based on the number, that is greater than 2, of alleles detected to be higher than the noise threshold and at least two maximal allele counts for the target DNA site, wherein the maximal two allele counts are marked as R1 and R2, respectively, comprises the following steps:

(a2-ii-4-1) determining whether R2/R1 is greater than or equal to 0.5 and/or whether R1/(R1+R2) is greater than or equal to 1/2 and less than or equal to 2/3 and/or whether R2/(R1+R2) is a value that is greater than or equal to 1/3 and less than or equal to 1/2, and if the determination result is yes, estimating the genotype of the target DNA site as AB|AC, and then performing the following step (a2-ii-4-3); if the determination result is no, then performing the following step (a2-ii-4-2);

(a2-ii-4-2) marking the allele count for this site an an outlier, and then either estimating the genotype of this target site to be NA and performing the following step (a2-ii-4-3), or setting the number of alleles detected to be higher than the noise threshold in the target DNA site to be 2, then estimating the genotype of the target site as described in step (a2-ii-3), and performing the following step (a2-ii-4-3);

(a2-ii-4-3) outputting the estimated genotype of the target site.

Among them, genotype NA represents that the genotype for a target site cannot be estimated.

Further, in the above step (a2-iii), based on the estimated genotype of the target DNA site and the individual allele counts for the target DNA site, estimating the count (FC) derived from the least component DNA and total count (TC), wherein the maximal three allele counts are marked as R1, R2 and R3 in sequence, comprises the following steps:

(a2-iii-1) if the estimated genotype of the target site is AA|AA, estimating the count (FC) derived from the least component DNA as NA, and the total count (TC) as R1 or R1+R2 or R1+R2+R3, and then performing the following step (a2-iii-7);

(a2-iii-2) if the estimated genotype of the target site is AB AB, estimating the count (FC) derived from the least component DNA as NA, and the total count (TC) as R1+R2 or R1+R2 +R3, and then performing the following step (a2-iii-7);

(a2-iii-3) if the estimated genotype of the target site is AB|AA, estimating the count (FC) derived from the least component DNA as R1-R2, and the total count (TC) as R1+R2 or R1+R2+R3, and then performing the following step (a2-iii-7);

(a2-iii-4) if the estimated genotype of the target site is AA|AB, estimating the count (FC) derived from the least component DNA as 2 times R2, and the total count (TC) as R1+R2 or R1+R2+R3, and then performing the following step (a2-iii-7);

(a2-iii-5) if the estimated genotype of the target site is AB|AC, estimating the count (FC) derived from the least component DNA as R1-R2+R3 or 2 times R3 or 2 times (R1-R2), and the total count (TC) as R1+R2+R3, and then performing the following step (a2-iii-7);

(a2-iii-6) if the estimated genotype of the target site is not one of the above-mentioned genotypes, estimating the count (FC) derived from the least component DNA as NA, and the total count (TC) as R1 or R1+R2 or R1+R2+R3, and then performing the following step (a2-iii-7);

(a2-iii-7) outputting the estimated count (FC) derived from the least component DNA and total count (TC).

Among them, estimating the count (FC) derived from the least component DNA as NA means that the count (FC) derived from the least component DNA cannot be estimated.

Further, in the above step (a3), the count (FC) of the least component DNA and total count (TC) for each target site of the reference group is used to estimate the concentration of the least component DNA, wherein linear regression or robust linear regression is used to calculate the concentration of the least component DNA in the sample, and/or the mean or median of FC and TC is used to calculate the concentration of the least component DNA in the sample.

Further, in the above step (a3), the count (FC) of the least component DNA and total count (TC) for each target site of the reference group is used to estimate the concentration of the least component DNA, wherein the concentration of the least component DNA is estimated by fitting a regression model.

Further, in the above steps, the concentration of the least component DNA is estimated by fitting a regression model, wherein the regression model is selected from: linear regression model, robust linear regression model, simple regression model, ordinary least squares regression model, multiple regression model, general multiple regression model, polynomial regression model, general linear model, generalized linear model, discrete choice regression model, logistic regression model, multinomial logit mode, mixed logit model, probit model, polynomial probabilistic unit model, ordinal logit model, ordered probit model, Poisson model, multiple response regression mode, multilevel mode, fixed effects mode, random effects mode, mixed mode, nonlinear regression mode, nonparametric mode, semiparametric mode, robust mode, quantile mode, isotonic mode, principal component mode, minimum angle mode, local mode, segmental mode, and variable error mode.

Further, in the above steps, the concentration of the least component DNA is estimated by fitting a regression model, wherein in the fitted model, the total count (TC) of each target site in the reference group is an independent variable, and the count (TC) of the least component DNA of each target site DNA count (FC) is a dependent variable.

Further, in the above steps, the concentration of the least component DNA is estimated by fitting a regression model, wherein the concentration of the least component DNA is estimated as the regression coefficient of the model parameter total count (TC).

Preferably, the fitted regression model is a linear regression model; preferably, the fitted regression model is a robust linear regression model; preferably, the fitted regression model is a general linear model.

The present invention provides a method for calculating a concentration of the least component DNA in a sample by using an iterative fitting genotype method of allele counts of individual target sites in the reference group, the method comprising:

(b1) setting a noise threshold $\alpha$, an initial concentration estimation value $f_0$ and an iteration error precision value $\varepsilon$ of the sample;

(b2) for each target DNA site, using counts of its individual alleles and the concentration value $f_0$ of the least component DNA in the sample to estimate its genotype;

(b3) for each target DNA site, estimating the count (FC) derived from the least component DNA and total count (TC) based on its estimated genotype;

(b4) using the count (FC) of the least component DNA and total count (TC) to estimate the concentration f of the least component DNA;

(b5) determining whether the absolute value of $f-f_0$ is less than $\varepsilon$, and if the determination result is no, then setting

$f_0=f$, and then performing step (b2); if the determination result is yes, estimating the least component DNA concentration in the sample as f.

Further, setting the noise threshold $\alpha$ of the sample in the above step (b1) is to set the threshold for distinguishing the count signal of the real allele from the false allele count signal; preferably, the noise threshold $\alpha$ as set is any value not greater than 0.05; preferably, the noise threshold $\alpha$ as set is 0.05, 0.04, 0.03, 0.02, 0.01, 0.0075, 0.005, 0.0025 or 0.001.

Further, setting the initial concentration estimation value $f_0$ in the above-mentioned step (b1) is to set $f_0$ as the value of any possible least component DNA concentration; preferably, the set initial concentration estimation value $f_0$ is less than 0.5; preferably, the set initial concentration estimation value $f_0$ is less than 0.5 and greater than the set noise threshold $\alpha$; preferably, the set initial concentration estimation value $f_0$ is any value that is not only less than 0.5 but also greater than the set noise threshold $\alpha$; preferably, the set initial concentration estimation value $f_0$ is 0.50, 0.45, 0.40, 0.35, 030, 0.25, 0.20, 0.15, 0.10, 0.05, 0.04, 0.03, 0.02, 0.01 or 0.005.

Further, setting the iteration error precision value $\varepsilon$ in the above-mentioned step (b1) is to set $\varepsilon$ as a very small cut-off threshold for iterative calculation; preferably, the set $\varepsilon$ value is less than 0.01; preferably, the set $\varepsilon$ value is any value less than 0.01; preferably, the set $\varepsilon$ value is less than 0.001; preferably, the set $\varepsilon$ value is less than 0.0001; preferably, the set $\varepsilon$ value is 0.01, 0.001, 0.0001 or 0.00001.

Further, in the above-mentioned step (b2), for each target DNA site, using counts of its individual alleles and the concentration value $f_0$ of the least component DNA in the sample to estimate its genotype, comprises the following steps:

(b2-i) listing all possible genotypes of the target DNA site according to the source of the sample;
(b2-ii) for each possible genotype of the target DNA site, using the concentration value $f_0$ of the least component DNA in the sample and the total count (TC) of individual alleles of the target DNA site to calculate theoretical counts of individual alleles thereof;
(b2-iii) for each possible genotype of the target DNA site, using the counts of individual alleles of the target DNA site and theoretical counts of individual alleles thereof to perform a goodness-of-fit test;
(b2-iv) analyzing results of the goodness-of-fit test of the target DNA site for all possible genotypes, and selecting the genotype with the best fit for each allele count of the target DNA site as the estimated target DNA site genotype.

In the present invention, the goodness-of-fit test refers to one or more statistical testing methods that can be used to test the consistency between observed numbers and theoretical numbers; preferably, the goodness-of-fit test is chi-square test; preferably, the goodness-of-fit test is a G test; preferably, the goodness-of-fit test is Fisher's exact test; preferably, the goodness-of-fit test is a binomial distribution test; preferably, the goodness-of-fit test is a chi-square test and/or G test and/or Fisher's exact test and/or binomial distribution test and/or variants thereof and/or combinations thereof; preferably, the goodness-of-fit test is the goodness-of-fit test that is performed by using calculated values, G values and /or AIC values, and/or corrected G values and/or corrected AIC values, and/or variants of G values or AIC values, and/or combinations thereof, of the G test.

Further, in the above step (b3), for each target DNA site, estimating the count (FC) derived from the least component DNA and total count (TC) based on its estimated genotype, wherein the maximal four allele counts are marked as R1, R2, R3, and R4 in sequence, comprises the following steps:

(b3-1) if the estimated genotype of the target site is AA|AA, estimating the count (FC) derived from the least component DNA as NA, and the total count (TC) as R1 or R1+R2 or R1+R2+R3 or R1+R2+R3+R4, and then performing the following step (b3-11);
(b3-2) if the estimated genotype of the target site is AB|AB, estimating the count (FC) derived from the least component DNA as NA, and the total count (TC) as R1+R2 or R1+R2+R3 or R1+R2+R3+R4, and then performing the following step (b3-11);
(b3-3) if the estimated genotype of the target site is ABIAA, estimating the count (FC) derived from the least component DNA as R1-R2, and the total count (TC) as R1+R2 or R1+R2+R3 or R1+R2+R3+R4, and then performing the following step (b3-11);
(b3-4) if the estimated genotype of the target site is AA|AB, estimating the count (FC) derived from the least component DNA as 2 times R2, and the total count (TC) as R1+R2 or R1+R2+R3 or R1+R2+R3+R4, and then performing the following step (b3-11);
(b3-5) if the estimated genotype of the target site is AB|AC, estimating the count (FC) derived from the least component DNA as R1-R2+R3 or 2 times R3 or 2 times (R1-R2), and the total count (TC) as R1+R2+R3 or R1+R2+R3+R4, and then performing the following step (b3-11);
(b3-6) if the estimated genotype of the target site is AA|BB, estimating the count (FC) derived from the least component DNA as R2, and the total count (TC) as R1+R2 or R1+R2+R3 or R1+R2+R3+R4, and then performing the following step (b3-11);

(b3-7) if the estimated genotype of the target site is AA|BC, estimating the count (FC) derived from the least component DNA as R2+R3 or 2 times R2 or 2 times R3, and the total count (TC) as R1+R2+R3 or R1+R2+R3+R4, and then performing the following step (b3-11);

(b3-8) if the estimated genotype of the target site is AB|CC, then determining whether the current estimated value $f_0$ is greater than or equal to 1/3, and if the determination result is yes, estimating the count (FC) derived from the least component DNA as R1, and the total count (TC) as R1+R2+R3 or R1+R2+R3+R4, and then performing the following step (b3-11); if the determination result is no, estimating the count (FC) derived from the least component DNA as R3, and the total count (TC) as R1+R2+R3 or R1+R2+R3+R4, and then performing the following step (b3-11);

(b3-9) if the estimated genotype of the target site is AB|CD, estimating the count (FC) derived from the least component DNA as R3+R4 or 2 times R3 or 2 times R4, and the total count (TC) as R1+R2+R3+R4, and then performing the following step (b3-11);

(b3-10) if the estimated genotype of the target site is not one of the above-mentioned genotypes, estimating the count (FC) derived from the least component DNA as NA, and the total count (TC) as R1 or R1+R2 or R1+R2+R3 or R1+R2+R3+R4, and then performing the following step (b3-11);

(b3-11) outputting the estimated count (FC) derived from the least component DNA and total count (TC).

Further, in the above-mentioned step (b4), using the count (FC) of the least component DNA and total count (TC) to estimate the concentration f of the least component DNA, is to estimate the concentration f of the least component DNA by using the method described in step (a3).

In the present invention, the concentration of the least component DNA in a sample is calculated by using an iterative fitting genotype method of allele counts of individual target sites in the reference group. This method can be used not only to estimate the concentration of the least component DNA in mixed samples with biological relationship, but also to estimate the concentration of the least component DNA in mixed samples without biological relationship. Further, the method is not only suitable for calculating the concentration of fetal DNA in the plasma DNA samples of pregnant women who are biological genetic mothers, but also suitable for calculating the concentration of fetal DNA in the plasma DNA of pregnant women who are legally permitted to accept egg donation. Furthermore, this method can be used to estimate the concentration of the least component DNA in two independent mixed DNA samples. Furthermore, the method described above can be used to estimate concentrations of several components in a mixture of more than two samples.

For example, for multiple pregnancy, a fetal DNA concentration value that needs to be iterated can be set for each fetus; for example, for twin pregnancy, fetal DNA concentration values that need to be iterated can be set as f1 and f2, respectively; for triplet pregnancy, fetal DNA concentration values that need to be iterated can be set as f1, f2, and f3; and so on. In order to estimate the concentration of multiple sample components, one can first set an initial value for the concentration of each sample, and then use the individual allele counts of individual target DNA sites and all possible genotypes of the site to estimate the estimated counts for each sample component at the target site, and then iteratively calculate the concentration of each sample component by using the goodness-of-fit test, until the change of the calculated concentration of each sample component is less than the set precision value.

In the present invention, the target to be detected in the sample includes a single target DNA site, an entire chromosome containing one or more target DNA sites, and a sub-chromosomal fragment containing one or more target DNA sites.

The present invention provides a method for determining the karyotype or genotype or wild-mutant type of a target to be detected in a sample by using a goodness-of-fit test of allele counts for a target DNA site, the method comprising:

(c1) dividing each target DNA site into reference sites or target sites according to its location on a chromosome, wherein reference sites form a reference group, and target sites forms a target group;

(c2) calculating the concentration of the least component DNA in the sample using the allele counts for individual target DNA sites in the reference group;

(c3) estimating the karyotype or genotype or wild-mutant type of the target to be detected in the sample by means of the goodness-of-fit test using the allele counts for individual target DNA sites in the target group and the concentration of the least component DNA in the sample.

Further, in the above step (c3), the genotype of the target to be detected in the sample is estimated by means of the goodness-of-fit test using the allele counts for individual target DNA sites in the target group and the concentration of the least component DNA in the sample, and the method includes:

(c3-a1) for each target DNA site in the target group, listing all possible genotypes thereof;

(c3-a2) for each target DNA site in the target group, calculating theoretical counts of individual alleles for each possible genotype thereof, according to the least component DNA concentration in the sample and the total count of individual alleles at this site;

(c3-a3) for each target DNA site in the target group, using the individual allele counts and theoretical counts of the target DNA site to perform the goodness-of-fit test for each possible genotype thereof;

(c3-a4) for each target DNA site in the target group, selecting the best-fitting genotype as the genotype of the target DNA site, according to the goodness-of-fit test results for all possible genotypes thereof.

Further, in the above step (c3), the karyotype of the target to be detected in the sample is estimated by means of the goodness-of-fit test using the allele counts for individual target DNA sites in the target group and the concentration of the least component DNA in the sample, and the method includes:

(c3-b1) analyzing the sample to be tested, and listing all possible karyotypes of the target chromosomal or sub-chromosomal fragment to be detected;

(c3-b2) for each possible karyotype, listing all possible genotypes for each target DNA site in the target group;

(c3-b3) for each target DNA site in the target group, firstly using the individual allele counts thereof to perform the goodness-of-fit test for all possible genotypes thereof, and then for each possible karyotype, selecting a genotype with the best fit for such karyotype;

(c3-b4) comprehensively analyzing the goodness-of-fit test results of all target DNA sites for each karyotype, and selecting a karyotype with the best comprehensive fit for all target DNA sites as the karyotype of the target chromosomal or sub-chromosomal fragment to be detected.

Further, in the above step (c3), the wild-mutant type of the target to be detected in the sample is estimated by means of the goodness-of-fit test using the allele counts for individual target DNA sites in the target group and the concentration of the least component DNA in the sample, and the method includes:

(c3-c1a) for each target DNA site in the target group, listing all possible wild-mutant genotypes thereof;

(c3-c2a) for each target DNA site in the target group, calculating theoretical counts of individual alleles for each possible wild-mutant genotype thereof, according to the least component DNA concentration in the sample and the total count of individual alleles at this site;

(c3-c3a) for each target DNA site in the target group, using the individual allele counts and theoretical counts of the target DNA site to perform the goodness-of-fit test for each possible wild-mutant genotype thereof;

(c3-c4a) comprehensively analyzing all target DNA sites in the target group, and selecting a wild-mutant genotype with the best fit for all target sites as the wild-mutant genotype of the target to be detected.

Further, in the above step (c3), the wild-mutant type of the target to be detected in the sample is estimated by means of the goodness-of-fit test using the allele counts for individual target DNA sites in the target group and the concentration of the least component DNA in the sample, and the method includes:

(c3-c1b) for each target DNA site in the target group, estimating its genotype by means of the goodness-of-fit test, according to the individual allele counts thereof and the least component DNA concentration in the sample;

(c3-c2b) determining the wild-mutant type of each allele of the target to be detected in each component of the sample, according to the genotype and the sequence of each allele for each target DNA site in the target group.

Further, in the above step (c3), the genotype or wild-mutant type of the target to be detected in the sample is estimated by means of the goodness-of-fit test using the allele counts for individual target DNA sites in the target group and the concentration of the least component DNA in the sample, wherein the target group can be one target site, or multiple independent replicates of one target site. Preferably, the independent replicates of the target site are obtained by using the same primers and independent PCR and/or multiple PCR amplification reactions; preferably, the independent replicates of the target site are obtained by using different primers and independent PCR and/or multiple PCR amplification reactions.

Further, in the above step (c3), the genotype or wild-mutant type of the target to be detected in the sample is estimated by means of the goodness-of-fit test using the allele counts for individual target DNA sites in the target group and the concentration of the least component DNA in the sample, wherein said goodness-of-fit test method adopts one or more statistical testing methods that can be used to test the consistency between observed numbers and theoretical numbers; preferably, the goodness-of-fit test is chi-square test; preferably, the goodness-of-fit test is a G test; preferably, the goodness-of-fit test is Fisher's exact test; preferably, the goodness-of-fit test is a binomial distribution test; preferably, the goodness-of-fit test is a chi-square test and/or G test and/or Fisher's exact test and/or binomial distribution test and/or variants thereof and/or combinations thereof; preferably, the goodness-of-fit test is the goodness-of-fit test that is performed by using calculated values, G values and /or AIC values, and/or corrected G values and /or corrected AIC values, and/or variants of G values or AIC values, and/or combinations thereof, of the G test.

Further, in the above step (c3), the genotype or wild-mutant type of the target to be detected in the sample is estimated by means of the goodness-of-fit test using the allele counts for individual target DNA sites in the target group and the concentration of the least component DNA in the sample, wherein the goodness-of-fit test method is the goodness-of-fit test using the method described in step (b2-i) to step (b2-iv).

In the present invention, the karyotype at the chromosome level refers to the euploidy or aneuploidy state of certain chromosome No. in mixed components in a mixed sample. For example, in a plasma sample of a pregnant woman, the chromosome karyotype wherein the mother is normal while the fetus is monosomy is disomy-monosomy, the chromosome karyotype wherein the mother is normal while the fetus is trisomy is disomy-trisomy, and the chromosome karyotype wherein both the mother and fetus are normal is disomy-disomy.

In the present invention, each fragment at the sub-chromosomal level is considered as a chromosome, so in a plasma sample of a pregnant woman, the sub-chromosomal karyotype wherein both the mother and fetus have a homozygous micro-deletion is nullisomy-nullisomy, the sub-chromosomal karyotype wherein the mother has a homozygous micro-deletion and the fetus has a heterozygous micro-deletion is nullisomy-monosomy, the sub-chromosomal karyotype wherein the mother has a heterozygous micro-deletion and the fetus is normal is monosomy-disomy, the sub-chromosomal karyotype wherein the mother and fetus have a heterozygous micro-deletion is monosomy-monosomy, the sub-chromosomal karyotype wherein the mother has a heterozygous micro-deletion and the fetus has a homozygous micro-deletion is monosomy-nullisomy, the sub-chromosomal karyotype wherein the mother is normal and the fetus has a heterozygous micro-deletion is disomy-monosomy, the sub-chromosomal karyotype wherein both the mother and fetus are normal is disomy-disomy, the sub-chromosomal karyotype wherein both the mother and fetus have a homozygous micro-duplication is tetrasomy-tetrasomy, the sub-chromosomal karyotype wherein the mother has a homozygous micro-duplication and the fetus has a heterozygous micro-duplication is tetrasomy-trisomy, the sub-chromosomal karyotype wherein the mother has a heterozygous micro-duplication and the fetus is normal is trisomy-disomy, the sub-chromosomal karyotype wherein both the mother and fetus have a heterozygous micro-duplication is trisomy-trisomy, the sub-chromosomal karyotype wherein the mother has a heterozygous micro-duplication and the fetus has a homozygous micro-duplication is trisomy-tetrasomy, and the sub-chromosomal karyotype wherein the mother is normal and the fetus has a heterozygous micro-duplication is disomy-trisomy.

In the present invention, genotype refers to the combination of genotypes of a target DNA site in mixed components in a mixed sample, where 0 or 1 allele may be detected at this site on each chromosome. For example, in a plasma sample of a pregnant woman, there are 4 possible genotypes (not including the genotypes where the mother and/or fetus are chimera) at the site whose karyotype is disomy-monosomy, which are AA|AØ, AA|BØ, ABIAO and ABICO, respectively; and possible genotypes of disomy-trisomy (not including genotypes where the mother and/or fetus are chimera and/or the genotypes where the fetus has not inherited not less than one allele from the mother due to *de novo* mutation, etc.) are AA|AAA, AA|AAB, AB|AAA, AB|AAB, AB|AAC, AB ABC, AA|ABB, AA|ABC, AB|ACC and AB|ACD, wherein A, B, C and D represent alleles that are different at target DNA sites, while Ø represents a deletion. In general, the genotype of a site in a mixed sample is all possible combinations of alleles of the site on each chromosome in each sample. Similarly, for variation at the sub-chromosomal level, 0 (micro-deletion), 1 (normal), or 2 (micro-duplication) alleles may be detected at this site on each chromosome, so all possible genotypes corresponding to the sub-chromosomal karyotype in a mixed sample are all possible combinations of all alleles for each site on each chromosome in the mixed sample. For example, in a plasma sample of a pregnant woman, there are 22 possible genotypes (not including genotypes where the mother and/or fetus are chimera and/or the genotypes where the fetus has not inherited not less than one allele from the mother due to *de novo* mutation, etc.) at the site where the sub-chromosomal karyotype is trisomy-trisomy, are AAA|AAA, AAAIAAB, AAAIABB, AAA|ABC, AABIAAA, AABIAAB, AABIAAC, AABIABB, AABIABC, AABIACC, AABIACD, AABIBBB, AABBBC, AABBCC, AABBCD, ABC|AAA, ABC|AAB, ABC|AAD, ABC|ABC, ABC|ABD, ABC|ADD and ABC|ADE, wherein A, B, C, D, and E represent alleles that are different at target DNA sites.

The present invention provides a method for determining the karyotype or genotype or wild-mutant type of a target to be detected in a sample by using a relative distribution diagram of allele counts of individual target sites, the method comprising:

(d1) dividing each target DNA site into reference sites or target sites according to its location on the chromosome, wherein reference sites forms a reference group, and target sites forms a target group;
(d2) calculating the concentration of the least component DNA in the sample using the allele counts for individual target DNA sites in the reference group;
(d3) estimating the karyotype or genotype or wild-mutant type of the target to be detected in the sample by means of the relative distribution diagram of allele counts, using the allele counts for individual target DNA sites in the target group and the concentration of the least component DNA in the sample.

Further, in the above step (d3), the genotype of the target to be detected in the sample is estimated by means of the relative distribution diagram of allele counts, using the allele counts for individual target DNA sites in the target group and the concentration of the least component DNA in the sample, and the method comprises:

(d3-a1) for each target DNA site in the target group, listing all possible genotypes thereof;

(d3-a2) for each possible genotype of the target DNA sites in the target group, firstly calculating theoretical values of relative counts of alleles thereof according to the concentration of the least component DNA in the sample, and then selecting at least one non-maximal theoretical value of relative counts of alleles to be plotted against the maximal theoretical value of relative counts of alleles to mark a theoretical position of the genotype;

(d3-a3) for each target DNA site in the target group, firstly calculating relative counts of alleles thereof, and then selecting at least one non-maximal relative count of alleles to be plotted against the maximal relative count of alleles to mark the actual position of the target DNA site on the relative count map of alleles;

(d3-a4) inferring the genotype of the target to be detected according to the theoretical position distribution and actual position distribution of each target DNA site in the target group in the relative count map of alleles.

Further, in the above step (d3), the karyotype of the target to be detected in the sample is estimated by means of the relative distribution diagram of allele counts, using the allele counts for individual target DNA sites in the target group and the concentration of the least component DNA in the sample, and the method comprises:

(d3-b1) analyzing the sample to be tested, and listing all possible karyotypes of the target chromosomal or sub-chromosomal fragment to be detected;

(d3-b2) for each possible karyotype, listing all possible genotypes for each target DNA site in the target group;

(d3-b3) for each possible genotype of the target DNA sites in the target group, firstly calculating theoretical values of relative counts of alleles thereof according to the concentration of the least component DNA in the sample, and then selecting at least one non-maximal theoretical value of relative counts of alleles to be plotted against the maximal theoretical value of relative counts of alleles to mark a theoretical position of the genotype;

(d3-b4) for each target DNA site in the target group, firstly calculating relative counts of alleles thereof, and then selecting at least one non-maximal relative count of alleles to be plotted against the maximal relative count of alleles to mark the actual position of the target DNA site on the relative count map of alleles;

(d3-b5) inferring the karyotype of the target to be detected according to the theoretical position distribution and actual position distribution in each karyotype of each target DNA site in the target group in the relative count map of alleles.

Further, in the above step (d3), the wild-mutant type of the target to be detected in the sample is estimated by means of the relative distribution diagram of allele counts, using the allele counts for individual target DNA sites in the target group and the concentration of the least component DNA in the sample, and the method comprises:

(d3-c1) for each target DNA site in the target group, listing the wild-type sequence and all possible wild-mutant genotypes thereof;

(d3-c2) for each possible wild-mutant genotype, calculating theoretical values of relative counts of its wild-type allele and other non-wild-type alleles, and selecting at least one theoretical value of relative counts of non-wild-type alleles to be plotted against the theoretical value of the relative count of the wild-type allele to mark a theoretical position of its wild-mutant genotype;

(d3-c3) for each target DNA site in the target group, calculating relative count values of its wild-type allele and other non-wild-type alleles, and selecting at least one relative count of non-wild-type alleles to be plotted against the relative count of the wild-type allele to mark the actual position of the target DNA site on the relative count map of alleles;

(d3-c4) inferring the wild-mutant type thereof according to the theoretical position distribution and actual position distribution of all target DNA sites in the target group in the relative count map of alleles.

The invention provides a method for determining the karyotype or genotype or wild-mutant type of a target to be detected in a sample by using a goodness-of-fit test of allele counts and/or the relative distribution diagram of allele counts for target DNA sites, characterized in that calculating the concentration of the least component DNA in the sample using the allele counts for individual target DNA sites in the reference group in step (c2) or step (d2), is to use the method described in step (a1) to step (a3) and/or step (b1) to step (b5) to calculate the concentration of the least component DNA in the sample.

The present invention provides a method for determining the karyotype of a target to be detected in a single genome sample by using a relative distribution diagram of allele counts, the method comprising:

(e1) calculating the relative counts of individual alleles of each target DNA site;

(e2) for each target DNA site, plotting a distribution diagram A of its second maximal relative count of alleles to its maximal relative count of alleles or plotting a distribution diagram B of its maximal relative count of alleles to the relative position of the target DNA site on the chromosome or sub-chromosome;

(e3) estimating the karyotype of the target to be detected in a single genome sample by using the relative distribution diagram A and/or distribution diagram B of allele counts of individual target DNA sites.

The present invention can not only detect genetic changes of each component in mixed genomes, e.g. detect genetic changes of a single site or variations at the chromosomal and sub-chromosomal level in a mother and/or fetus by counting each allele of polymorphic sites in plasma DNA samples of pregnant women, but also be applied to karyotype or genotype detection of single-genome samples, e.g. for use in preimplantation diagnosis of genetic diseases in embryos. The method can detect genetic changes in samples both at the nucleotide level and at the chromosomal or sub-chromosomal level, and has good development and application prospects for the screening of fetal genetic diseases.

The present invention relates to detecting whether a target to be tested has genetic abnormality using a mixture of mother and fetus genetic materials. Accordingly, in one aspect, the invention provides a method for determining the presence or absence of fetal aneuploidy in a biological sample comprising nucleic acids of fetus and mother in the form of free-floating DNA from a biological sample of said mother, amplifying target DNA sites in a PCR or multiplex PCR reaction (i.e., amplifying template DNA such that the amplified DNA reproduces the ratio of the original template DNA), and then determining the presence or absence of the fetal aneuploidy according to the relative count distribution of individual alleles of each target DNA site for the target to be detected as amplified.

In another aspect, the present invention provides a method for determining the presence or absence of copy number variation of a fetal chromosomal fragment in a biological sample comprising nucleic acids of fetus and mother in the form of free-floating DNA from a biological sample of said mother, amplifying target DNA sites in a PCR or multiplex PCR reaction (i.e., amplifying template DNA such that the amplified DNA reproduces the ratio of the original template DNA), and then determining the presence or absence of the copy number variation of the fetal chromosomal fragment according to the relative count distribution of individual alleles of each target DNA site for the target to be detected as amplified.

In another aspect, the present invention provides a method for determining the presence or absence of a variation in a fetal monogenic disease-causing genetic site in a biological sample comprising nucleic acids of fetus and mother in the form of free-floating DNA from a biological sample of said mother, amplifying target DNA sites in a PCR or multiplex PCR reaction (i.e., amplifying template DNA such that the amplified DNA reproduces the ratio of the original template DNA), and then determining the presence or absence of the variation in the fetal monogenic disease-causing genetic site according to the relative count distribution of individual alleles of the target DNA site (the monogenic disease-causing genetic site) to be tested as amplified

In another aspect, the invention provides a diagnostic kit for implementing the present methods, comprising at least one set of primers to amplify a target DNA site. The at least one set of primers amplifies at least one target DNA site in a reference group and/or at least one target DNA site in a target group. Among them, the target DNA site in the target group is selected from chromosomes with possible chromosomal aneuploidy abnormalities and/or chromosome fragments with possible copy number variations and/or possible pathogenic variation sites of monogenic diseases. Among them, the nucleic acid sequence of the target DNA site in the target group generally has polymorphisms in the population to be tested and/or the target DNA site in the target group is a possible pathogenic variation site of a monogenic disease. Among them, the target DNA site in the reference group is selected from chromosomes that usually have no chromosomal aneuploidy abnormality and/or chromosome fragments that usually have no copy number variation. Among them, the nucleic acid sequence of the target DNA site in the reference group generally has polymorphisms in the population to be tested.

In another aspect, the invention provides a diagnostic kit for implementing the present methods. The diagnostic kit includes primers for performing step (2) and/or step (3). Other reagents that may be optionally included in the diagnostic kit are instructions for use, polymerases and buffers for performing PCR and/or multiplex PCR reactions and reagents required for constructing a high-throughput sequencing library of the amplified fragments.

In another aspect, the invention provides a system for implementing the present methods. The system is used to implement one or more steps, such as one or more of steps (4) to (5), in the methods of predicting the karyotype or genotype or wild-mutant type of a target to be detected from a biological test sample. In another aspect, the present invention provides a device and/or computer program product and/or system and/or module for implementing the present methods, for carrying out any step of the above-mentioned step (1) to step (5), the above-mentioned step (a1) to step (a3), the above-mentioned step (b1) to step (b5), the above-mentioned step (c1) to step (c3), the above-mentioned step (d1) to step (d3) and/or the above-mentioned step (e1) to step (e3).

In some embodiments, the methods of the invention are performed *in vitro* or *ex vivo.* In some embodiments, samples of the invention are *in vitro* or *ex vivo* samples.

In one aspect, the invention relates to a device for implementing the present methods. For example, in some embodiments, the present invention relates to a device for detecting genetic variation in a sample, characterized by comprising:

(1) a module configured to receive a biological sample to be tested and prepare nucleic acid;
(2) a module configured to enrich or amplify target DNA sites, wherein at least one target DNA site has more than one allele in the sample;
(3) a module configured to sequence the amplified target DNA sites;
(4) a statistics module, which is configured to count the counts of individual alleles for each target DNA site;
(5) determination module, which is configured to determine the karyotype or genotype or wild-mutant type of a target to be detected in a sample by using a goodness-of-fit test of allele counts and/or the relative distribution diagram of allele counts for target DNA sites.

In some embodiments, the statistics module is configured to count the counts of individual alleles for each target DNA site, and the counting include the following steps in sequence: (4-1) for each amplified sequence, mapping it to a chromosome or genome position; (4-2) count the number of sequences mapped in each chromosome or genome region; wherein if a certain chromosome or genome region has different alleles, then the number of sequences mapped for each allele in the region is counted at the same time. In some embodiments, any in silico method is used to map each sequence read to a chromosome or genome location/region. In some embodiments, the computer algorithm used in step (4-1) to map sequences includes, but is not limited to, search for specific sequences, BLAST, BLITZ, FASTA, BOWTIE, BOWTIE 2, BWA, NOVOALIGN, GEM, ZOOM, ELAN, MAQ, MATCH, SOAP, STAR, SEGEMEHL, MOSAIK or SEQMAP or variants thereof or combinations thereof. In some embodiments, specific sequences (uniquely mapped sequences) are extracted from the chromosome or genome sequences corresponding to each target DNA site, and then used to map reads to chromosome or genome locations/regions. In some embodiments, sequence reads can be aligned to the sequence of a chromosome or genome location/region. In some embodiments, sequence reads can be aligned to the sequence of a chromosome or genome. In some embodiments, sequence reads can be obtained from, and/or aligned to sequences in, nucleic acid databases known in the art, including, for example, GenBank, dbEST, dbSTS, EMBL (European Molecular Biology Laboratory) and DDBJ (Japan DNA database). BLAST or similar tools can be used to search for the same sequence against a sequence database. Then, for example, search hits can be used to sort identical sequences into appropriate chromosome or genome locations/regions. In some embodiments, reads can be uniquely or non-uniquely mapped to portions in a reference genome. A read is said to be "uniquely mapped" if it aligns to a single sequence in the genome. A read is said to be "non-uniquely mapped, if it aligns to two or more sequences in the genome. In some embodiments, non-uniquely mapped reads are removed from further analysis (e.g., quantification).

In some embodiments, the determination module is configured to determine the karyotype or genotype or wild-mutant type of the target to be detected in the sample by using a goodness-of-fit test of allele counts for target DNA sites, and the determination comprises the following steps in sequence:

(c1) dividing each target DNA site into reference sites or target sites according to its location on a chromosome;
(c2) calculating the concentration of the least component DNA in the sample using the allele counts for individual target DNA sites in the reference group;
(c3) estimating the karyotype or genotype or wild-mutant type of the target to be detected in the sample by means of the goodness-of-fit test using the allele counts for individual target DNA sites in the target group and the concentration of the least component DNA in the sample.

In some embodiments, the determination module is configured to determine the karyotype or genotype or wild-mutant type of a target to be detected in a sample by using a relative distribution diagram of allele counts for target DNA site, and the determination comprises the following steps in sequence:

(d1) dividing each target DNA site into reference sites or target sites according to its location on the chromosome;
(d2) calculating the concentration of the least component DNA in the sample using the allele counts for individual target DNA sites in the reference group;
(d3) estimating the karyotype or genotype or wild-mutant type of the target to be detected in the sample by means of the relative distribution diagram of allele counts, using the allele counts for individual target DNA sites in the target group and the concentration of the least component DNA in the sample.

In some embodiments, one or more statistical testing methods, goodness-of-fit tests, are used to test the consistency between observed numbers and theoretical numbers. In some embodiments, the goodness-of-fit test is chi-square test. In some embodiments, the goodness-of-fit test is a G test. In some embodiments, the goodness-of-fit test is Fisher's exact test. In some embodiments, the goodness-of-fit test is a binomial distribution test. In some embodiments, the goodness-of-fit test is a chi-square test, G test, Fisher's exact test, binomial distribution test, variants thereof or combinations thereof. In some embodiments, the goodness-of-fit test is the goodness-of-fit test that is performed by using calculated values, G values, AIC values, corrected G values, corrected AIC values, variants of G values or AIC values, or combinations thereof, of the G test.

In some embodiments, the determination module is configured to determine the karyotype of a target to be detected in a sample by using a relative distribution diagram of allele counts for target DNA sites, wherein the sample to be tested is a single genome sample, and the determination comprises the following steps in sequence:

(e1) calculating the relative counts of individual alleles of each target DNA site in a target group;
(e2) for each target DNA site in the target group, plotting a distribution diagram A of its second maximal relative count of alleles to its maximal relative count of alleles or plotting a distribution diagram B of its maximal relative count of alleles to the relative position of the target DNA site on the chromosome or sub-chromosome;
(e3) estimating the karyotype of the target to be detected in a single genome sample by using the relative distribution diagram A and/or distribution diagram B of allele counts of individual target DNA sites in the target group.

In some embodiments, in step (c2) or step (d2), the concentration of the least component DNA in the sample is calculated by using a relative ratio method of allele counts, and the calculation comprises the following steps in sequence:

(a1) setting a noise threshold $\alpha$ of the sample;
(a2) for each target DNA site, firstly using counts of individual alleles at the target site to estimate its genotype, and then estimating the count (FC) derived from the least component DNA and total count (TC) based on its estimated genotype;
(a3) using the count (FC) of the least component DNA and total count (TC) for each target site in the reference group, to estimate the concentration of the least component DNA.

In some embodiments, the concentration of the least component DNA in the sample is calculated by using an iterative fitting genotype method of allele counts in step (c2) or step (d2), and the calculation comprises the following steps in sequence:

(b1) setting a noise threshold $\alpha$, an initial concentration estimation value $f_0$ and an iteration error precision value $\varepsilon$ of the sample;
(b2) for each target DNA site, using counts of its individual alleles and the concentration value $f_0$ of the least component DNA in the sample to estimate its genotype;
(b3) for each target DNA site, estimating the count (FC) derived from the least component DNA and total count (TC) based on its estimated genotype;
(b4) using the count (FC) of the least component DNA and total count (TC) to estimate the concentration f of the least component DNA;
(b5) determining whether the absolute value of $f-f_0$ is less than $\varepsilon$, and if the determination result is no, then setting $f_0=f$, and then performing step (b2); if the determination result is yes, estimating the least component DNA concentration in the sample as f.

In some embodiments, in step (c3), the genotype of the target to be detected in the sample is estimated by means of the goodness-of-fit test using the allele counts for individual target DNA sites in the target group and the concentration of the least component DNA in the sample, and said estimation comprises the following steps in sequence:

(c3-a1) for each target DNA site in the target group, listing all possible genotypes thereof;
(c3-a2) for each target DNA site in the target group, calculating theoretical counts of individual alleles for each possible genotype thereof, according to the least component DNA concentration in the sample and the total count of individual alleles at this site;
(c3-a3) for each target DNA site in the target group, using the individual allele counts and theoretical counts of the target DNA site to perform the goodness-of-fit test for each possible genotype thereof;
(c3-a4) for each target DNA site in the target group, selecting the best-fitting genotype as the genotype of the target DNA site, according to the goodness-of-fit test results for all possible genotypes thereof.

In some embodiments, in step (c3), the karyotype of the target to be detected in the sample is estimated by means of the goodness-of-fit test using the allele counts for individual target DNA sites in the target group and the concentration of the least component DNA in the sample, and said estimation comprises the following steps in sequence:

(c3-b 1) analyzing the sample to be tested, and listing all possible karyotypes of the target chromosomal or sub-chromosomal fragment to be detected;

(c3-b2) for each possible karyotype, listing all possible genotypes for each target DNA site in the target group;

(c3-b3) for each target DNA site in the target group, firstly using the individual allele counts thereof to perform the goodness-of-fit test for all possible genotypes thereof, and

then for each possible karyotype, selecting a genotype with the best fit for such karyotype;

(c3-b4) comprehensively analyzing the goodness-of-fit test results of all target DNA sites for each karyotype, and selecting a karyotype with the best comprehensive fit for all target DNA sites as the karyotype of the target chromosomal or sub-chromosomal fragment to be detected.

In some embodiments, in step (c3), the wild-mutant type of the target to be detected in the sample is estimated by means of the goodness-of-fit test using the allele counts for individual target DNA sites in the target group and the concentration of the least component DNA in the sample, and the estimation comprises the following steps in sequence:

(c3-c1a) for each target DNA site in the target group, listing all possible wild-mutant genotypes thereof;

(c3-c2a) for each target DNA site in the target group, calculating theoretical counts of individual alleles for each possible wild-mutant genotype thereof, according to the least component DNA concentration in the sample and the total count of individual alleles at this site;

(c3-c3a) for each target DNA site in the target group, using the individual allele counts and theoretical counts of the target DNA site to perform the goodness-of-fit test for each possible wild-mutant genotype thereof;

(c3-c4a) comprehensively analyzing all target DNA sites in the target group, and selecting a wild-mutant genotype with the best fit for all target sites as the wild-mutant genotype of the target to be detected.

In some embodiments, in step (c3), the wild-mutant type of the target to be detected in the sample is estimated by means of the goodness-of-fit test using the allele counts for individual target DNA sites in the target group and the concentration of the least component DNA in the sample, and the estimation comprises the following steps in sequence:

(c3-c1b) for each target DNA site in the target group, estimating its genotype by means of the goodness-of-fit test, according to the individual allele counts thereof and the least component DNA concentration in the sample;

(c3-c2b) determining the wild-mutant type of each allele of the target to be detected in each component of the sample, according to the genotype and the sequence of each allele for each target DNA site in the target group.

In some embodiments, one or more statistical testing methods, that can be used to test the consistency between observed numbers and theoretical numbers, are used to perform a goodness-of-fit test. In some embodiments, the goodness-of-fit test is chi-square test. In some embodiments, the goodness-of-fit test is a G test. In some embodiments, the goodness-of-fit test is Fisher's exact test. In some embodiments, the goodness-of-fit test is a binomial distribution test. In some embodiments, the goodness-of-fit test is a chi-square test, and/or G test, and/or Fisher's exact test, and/or binomial distribution test. In some embodiments, the goodness-of-fit test is the goodness-of-fit test that is performed by using calculated values, G values, and/or AIC values, and/or corrected G values, and/or corrected AIC values, and/or values derived from G values or AIC values, of the G test.

In some embodiments, in step (d3), the genotype of the target to be detected in the sample is estimated by means of the relative distribution diagram of allele counts, using the allele counts for individual target DNA sites in the target group and the concentration of the least component DNA in the sample, and the estimation comprises the following steps in sequence:

(d3-a1) for each target DNA site in the target group, listing all possible genotypes thereof;

(d3-a2) for each possible genotype of the target DNA sites in the target group, firstly calculating theoretical values of relative counts of alleles thereof according to the concentration of the least component DNA in the sample, and then selecting at least one non-maximal theoretical value of relative counts of alleles to be plotted against the maximal theoretical value of relative counts of alleles to mark a theoretical position of the genotype;

(d3-a3) for each target DNA site in the target group, firstly calculating relative counts of alleles thereof, and then selecting at least one non-maximal relative count of alleles to be plotted against the maximal relative count of alleles to mark the actual position of the target DNA site on the relative count map of alleles;

(d3-a4) inferring the genotype of the target to be detected according to the theoretical position distribution and actual position distribution of each target DNA site in the target group in the relative count map of alleles.

In some embodiments, in step (d3), the karyotype of the target to be detected in the sample is estimated by means of the relative distribution diagram of allele counts, using the allele counts for individual target DNA sites in the target group and the concentration of the least component DNA in the sample, and the estimation comprises the following steps in sequence:

(d3-b 1) analyzing the sample to be tested, and listing all possible karyotypes of the target chromosomal or sub-chromosomal fragment to be detected;
(d3-b2) for each possible karyotype, listing all possible genotypes for each target DNA site in the target group;
(d3-b3) for each possible genotype of the target DNA sites in the target group, firstly calculating theoretical values of relative counts of alleles thereof according to the concentration of the least component DNA in the sample, and then selecting at least one non-maximal theoretical value of relative counts of alleles to be plotted against the maximal theoretical value of relative counts of alleles to mark a theoretical position of the genotype;
(d3-b4) for each target DNA site in the target group, firstly calculating relative counts of alleles thereof, and then selecting at least one non-maximal relative count of alleles to be plotted against the maximal relative count of alleles to mark the actual position of the target DNA site on the relative count map of alleles;
(d3-b5) inferring the karyotype of the target to be detected according to the theoretical position distribution and actual position distribution in each karyotype of each target DNA site in the target group in the relative count map of alleles.

In some embodiments, in step (d3), the wild-mutant type of the target to be detected in the sample is estimated by means of the relative distribution diagram of allele counts, using the allele counts for individual target DNA sites in the target group and the concentration of the least component DNA in the sample, and the estimation comprises the following steps in sequence:

(d3-c1) for each target DNA site in the target group, listing the wild-type sequence and all possible wild-mutant genotypes thereof;
(d3-c2) for each possible wild-mutant genotype, calculating theoretical values of relative counts of its wild-type allele and other non-wild-type alleles, and selecting at least one theoretical value of relative counts of non-wild-type alleles to be plotted against the theoretical value of the relative count of the wild-type allele to mark a theoretical position of its wild-mutant genotype;
(d3-c3) for each target DNA site in the target group, calculating relative count values of its wild-type allele and other non-wild-type alleles, and selecting at least one relative count of non-wild-type alleles to be plotted against the relative count of the wild-type allele to mark the actual position of the target DNA site on the relative count map of alleles;
(d3-c4) inferring the wild-mutant type thereof according to the theoretical position distribution and actual position distribution of all target DNA sites in the target group in the relative count map of alleles.

In some embodiments, with respect to for each target DNA site, firstly using counts of its individual alleles to estimate its genotype, and then estimating the count (FC) derived from the least component DNA and total count (TC) based on its estimated genotype, as performed in step (a2), said estimating comprises the following steps in sequence:

(a2-i) sorting the allele counts of the target DNA site in descending order, wherein the maximal three allele counts are marked as R1, R2 and R3 in sequence;
(a2-ii) estimating the genotype of the target DNA site using counts of individual alleles for the target DNA site;
(a2-iii) based on the estimated genotype of the target DNA site and the individual allele counts for the target DNA site, estimating the count (FC) derived from the least component DNA and total count (TC).

In some embodiments, with respect to estimating the genotype of the target DNA site using counts of individual alleles for the target DNA site, as performed in step (a2-ii), said estimating comprises the following steps in sequence:

(a2-ii-1) using the counts of individual alleles for the target DNA site to determine the number of alleles that are detected to be higher than the noise threshold in the target DNA site; if the determination result is 1, performing the following step (a2-ii-2); if the determination result is 2, performing the following step (a2-ii-3); if the determination result is greater than 2, performing the following step (a2-ii-4);
(a2-ii-2) estimating the genotype of the target DNA site as AA|AA, and then performing the following step (a2-ii-5);
(a2-ii-3) estimating the genotype of the target DNA site based on the number, that is 2, of alleles detected to be higher than the noise threshold and the maximal two allele counts for the target DNA site, and then performing the following step (a2-ii-5);

(a2-ii-4) estimating the genotype of the target DNA site based on the number, that is greater than 2, of alleles detected to be higher than the noise threshold and at least two maximal allele counts for the target DNA site, and then performing the following step (a2-ii-5);

(a2-ii-5) outputting the estimated genotype of the target site.

In some embodiments, with respect to estimating the genotype of the target DNA site based on the number, that is 2, of alleles detected to be higher than the noise threshold and the maximal two allele counts for the target DNA site, as performed in step (a2-ii-3), said estimating comprises the following steps in sequence:

(a2-ii-3-1) determining whether the value of R1/(R1+R2) is less than 0.5+$\alpha$, and if the determination result is yes, estimating the genotype of the target DNA site as AB|AB, and then performing the following step (a2-ii-3-3); if the determination result is no, then performing the following step (a2-ii-3-2);

(a2-ii-3-2) determining whether the value of R1/(R1+R2) is less than 0.75, and if the determination result is yes, estimating the genotype of the target DNA site as AB|AA, and then performing the following step (a2-ii-3-3); if the determination result is no, estimating the genotype of the target DNA site as AA|AB, and then performing the following step (a2-ii-3-3);

(a2-ii-3-3) outputting the estimated genotype of the target site.

In some embodiments, with respect to estimating the genotype of the target DNA site based on the number, that is greater than 2, of alleles detected to be higher than the noise threshold and at least two maximal allele counts for the target DNA site, as performed in step (a2-ii-4), said estimating comprises the following steps:

(a2-ii-4-1) determining whether R2/R1 is greater than or equal to 0.5 and/or whether R1/(R1+R2) is greater than or equal to 1/2 and less than or equal to 2/3 and/or whether R2/(R1+R2) is a value that is greater than or equal to 1/3 and less than or equal to 1/2, and if the determination result is yes, estimating the genotype of the target DNA site as AB|AC, and then performing the following step (a2-ii-4-3); if the determination result is no, then performing the following step (a2-ii-4-2);

(a2-ii-4-2) marking the allele count for this site an an outlier, and then either estimating the genotype of this target site to be NA and performing the following step (a2-ii-4-3), or setting the number of alleles detected to be higher than the noise threshold in the target DNA site to be 2, then estimating the genotype of the target site as described in step (a2-ii-3), and performing the following step (a2-ii-4-3);

(a2-ii-4-3) outputting the estimated genotype of the target site.

In some embodiments, with respect to based on the estimated genotype of the target DNA site and the individual allele counts for the target DNA site, estimating the count (FC) derived from the least component DNA and total count (TC), wherein the maximal three allele counts are marked as R1, R2 and R3 in sequence, as performed in step (a2-iii), said estimating comprises the following steps:

(a2-iii-1) if the estimated genotype of the target site is AA|AA, estimating the count (FC) derived from the least component DNA as NA, and the total count (TC) as R1 or R1+R2 or R1+R2+R3, and then performing the following step (a2-iii-7);

(a2-iii-2) if the estimated genotype of the target site is AB AB, estimating the count (FC) derived from the least component DNA as NA, and the total count (TC) as R1+R2 or R1+R2 +R3, and then performing the following step (a2-iii-7);

(a2-iii-3) if the estimated genotype of the target site is AB|AA, estimating the count (FC) derived from the least component DNA as R1-R2, and the total count (TC) as R1+R2 or R1+R2+R3, and then performing the following step (a2-iii-7);

(a2-iii-4) if the estimated genotype of the target site is AA|AB, estimating the count (FC) derived from the least component DNA as 2 times R2, and the total count (TC) as R1+R2 or R1+R2+R3, and then performing the following step (a2-iii-7);

(a2-iii-5) if the estimated genotype of the target site is AB|AC, estimating the count (FC) derived from the least component DNA as R1-R2+R3 or 2 times R3 or 2 times (R1-R2), and the total count (TC) as R1+R2+R3, and then performing the following step (a2-iii-7);

(a2-iii-6) if the estimated genotype of the target site is not one of the above-mentioned genotypes, estimating the count (FC) derived from the least component DNA as NA, and the total count (TC) as R1 or R1+R2 or R1+R2+R3, and then performing the following step (a2-iii-7);

(a2-iii-7) outputting the estimated count (FC) derived from the least component DNA and total count (TC).

In some embodiments, with respect to for each target DNA site, using counts of its individual alleles and the concentration value $f_0$ of the least component DNA in the sample to estimate its genotype, as performed in step (b2), said estimating comprises the following steps:

(b2-i) listing all possible genotypes of the target DNA site according to the source of the sample;
(b2-ii) for each possible genotype of the target DNA site, using the concentration value $f_0$ of the least component DNA in the sample and the total count (TC) of individual alleles of the target DNA site to calculate theoretical counts of individual alleles thereof;
(b2-iii) for each possible genotype of the target DNA site, using the counts of individual alleles of the target DNA site and theoretical counts of individual alleles thereof to perform a goodness-of-fit test;
(b2-iv) analyzing results of the goodness-of-fit test of the target DNA site for all possible genotypes, and selecting the genotype with the best fit for each allele count of the target DNA site as the estimated target DNA site genotype.

In some embodiments, with respect to for each target DNA site, estimating the count (FC) derived from the least component DNA and total count (TC) based on its estimated genotype, wherein the maximal four allele counts are marked as R1, R2, R3, and R4 in sequence, as performed in step (b3), said estimating comprises the following steps:

(b3-1) if the estimated genotype of the target site is AA|AA, estimating the count (FC) derived from the least component DNA as NA, and the total count (TC) as R1 or R1+R2 or R1+R2+R3 or R1+R2+R3+R4, and then performing the following step (b3-11);
(b3-2) if the estimated genotype of the target site is AB AB, estimating the count (FC) derived from the least component DNA as NA, and the total count (TC) as R1+R2 or R1+R2+R3 or R1+R2+R3+R4, and then performing the following step (b3-11);
(b3-3) if the estimated genotype of the target site is AB|AA, estimating the count (FC) derived from the least component DNA as R1-R2, and the total count (TC) as R1+R2 or R1+R2+R3 or R1+R2+R3+R4, and then performing the following step (b3-11);
(b3-4) if the estimated genotype of the target site is AA|AB, estimating the count (FC) derived from the least component DNA as 2 times R2, and the total count (TC) as R1+R2 or R1+R2+R3 or R1+R2+R3+R4, and then performing the following step (b3-11);
(b3-5) if the estimated genotype of the target site is AB|AC, estimating the count (FC) derived from the least component DNA as R1-R2+R3 or 2 times R3 or 2 times (R1-R2), and the total count (TC) as R1+R2+R3 or R1+R2+R3+R4, and then performing the following step (b3-11);
(b3-6) if the estimated genotype of the target site is AA|BB, estimating the count (FC) derived from the least component DNA as R2, and the total count (TC) as R1+R2 or R1+R2+R3 or R1+R2+R3+R4, and then performing the following step (b3-11);
(b3-7) if the estimated genotype of the target site is AA|BC, estimating the count (FC) derived from the least component DNA as R2+R3 or 2 times R2 or 2 times R3, and the total count (TC) as R1+R2+R3 or R1+R2+R3+R4, and then performing the following step (b3-11);
(b3-8) if the estimated genotype of the target site is AB|CC, then determining whether the current estimated value $f_0$ is greater than or equal to 1/3, and if the determination result is yes, estimating the count (FC) derived from the least component DNA as R1, and the total count (TC) as R1+R2+R3 or R1+R2+R3+R4, and then performing the following step (b3-11); if the determination result is no, estimating the count (FC) derived from the least component DNA as R3, and the total count (TC) as R1+R2+R3 or R1+R2+R3+R4, and then performing the following step (b3-11);
(b3-9) if the estimated genotype of the target site is AB|CD, estimating the count (FC) derived from the least component DNA as R3+R4 or 2 times R3 or 2 times R4, and the total count (TC) as R1+R2+R3+R4, and then performing the following step (b3-11);
(b3-10) if the estimated genotype of the target site is not one of the above-mentioned genotypes, estimating the count (FC) derived from the least component DNA as NA, and the total count (TC) as R1 or R1+R2 or R1+R2+R3 or R1+R2+R3+R4, and then performing the following step (b3-11);
(b3-11) outputting the estimated count (FC) derived from the least component DNA and total count (TC).

In some embodiments, the present invention relates to a device for calculating a concentration of the least component DNA in a sample, said device comprising:

(a1) a module for setting a noise threshold $\alpha$ of the sample;
(a2) a module for with respect to each target DNA site, firstly using counts of individual alleles at the target site to estimate its genotype, and then estimating the count (FC) derived from the least component DNA and total count (TC) based on its estimated genotype;

(a3) a calculation module, which estimates the concentration of the least component DNA by using the count (FC) of the least component DNA and total count (TC) for each target site in a reference group.

In some embodiments, for each target DNA site, firstly using counts of its individual alleles to estimate its genotype, and then estimating the count (FC) derived from the least component DNA and total count (TC) based on its estimated genotype, as performed in step (a2) of the present invention, comprises the following steps:

(a2-i) sorting the allele counts of the target DNA site in descending order, wherein the maximal three allele counts are marked as R1, R2 and R3 in sequence;
(a2-ii) estimating the genotype of the target DNA site using counts of individual alleles for the target DNA site;
(a2-iii) based on the estimated genotype of the target DNA site and the individual allele counts for the target DNA site, estimating the count (FC) derived from the least component DNA and total count (TC).

In some embodiments, estimating the genotype of the target DNA site using counts of individual alleles for the target DNA site, wherein the maximal three allele counts are marked as R1, R2, and R3 in sequence, comprises the following steps:

(a2-ii-1) using the counts of individual alleles for the target DNA site to determine the number of alleles that are detected to be higher than the noise threshold in the target DNA site; if the determination result is 1, performing the following step (a2-ii-2); if the determination result is 2, performing the following step (a2-ii-3); if the determination result is greater than 2, performing the following step (a2-ii-4);
(a2-ii-2) estimating the genotype of the target DNA site as AA|AA, and then performing the following step (a2-ii-5);
(a2-ii-3) estimating the genotype of the target DNA site based on the number, that is 2, of alleles detected to be higher than the noise threshold and the maximal two allele counts for the target DNA site, and then performing the following step (a2-ii-5);
(a2-ii-4) estimating the genotype of the target DNA site based on the number, that is greater than 2, of alleles detected to be higher than the noise threshold and at least two maximal allele counts for the target DNA site, and then performing the following step (a2-ii-5);
(a2-ii-5) outputting the estimated genotype of the target site.

In some embodiments, estimating the genotype of the target DNA site based on the number, that is 2, of alleles detected to be higher than the noise threshold and the maximal two allele counts for the target DNA site, wherein the maximal two allele counts are marked as R1 and R2, respectively, comprises the following steps:

(a2-ii-3-1) determining whether the value of R1/(R1+R2) is less than $0.5+\alpha$, and if the determination result is yes, estimating the genotype of the target DNA site as AB|AB, and then performing the following step (a2-ii-3-3); if the determination result is no, then performing the following step (a2-ii-3-2);
(a2-ii-3-2) determining whether the value of R1/(R1+R2) is less than 0.75, and if the determination result is yes, estimating the genotype of the target DNA site as AB|AA, and then performing the following step (a2-ii-3-3); if the determination result is no, estimating the genotype of the target DNA site as AA|AB, and then performing the following step (a2-ii-3-3);
(a2-ii-3-3) outputting the estimated genotype of the target site.

In some embodiments, estimating the genotype of the target DNA site based on the number, that is greater than 2, of alleles detected to be higher than the noise threshold and at least two maximal allele counts for the target DNA site, wherein the maximal two allele counts are marked as R1 and R2, respectively, comprises the following steps:

(a2-ii-4-1) determining whether R2/R1 is greater than or equal to 0.5 and/or whether R1/(R1+R2) is greater than or equal to 1/2 and less than or equal to 2/3 and/or whether R2/(R1+R2) is a value that is greater than or equal to 1/3 and less than or equal to 1/2, and if the determination result is yes, estimating the genotype of the target DNA site as AB|AC, and then performing the following step (a2-ii-4-3); if the determination result is no, then performing the following step (a2-ii-4-2);
(a2-ii-4-2) marking the allele count for this site an an outlier, and then either estimating the genotype of this target site to be NA and performing the following step (a2-ii-4-3), or setting the number of alleles detected to be higher than the noise threshold in the target DNA site to be 2, then estimating the genotype of the target site as described in step (a2-ii-3), and performing the following step (a2-ii-4-3);
(a2-ii-4-3) outputting the estimated genotype of the target site.

In some embodiments, based on the estimated genotype of the target DNA site and the individual allele counts for the target DNA site, estimating the count (FC) derived from the least component DNA and total count (TC), wherein the maximal three allele counts are marked as R1, R2 and R3 in sequence, comprises the following steps:

(a2-iii-1) if the estimated genotype of the target site is AA|AA, estimating the count (FC) derived from the least component DNA as NA, and the total count (TC) as R1 or R1+R2 or R1+R2+R3, and then performing the following step (a2-iii-7);

(a2-iii-2) if the estimated genotype of the target site is AB AB, estimating the count (FC) derived from the least component DNA as NA, and the total count (TC) as R1+R2 or R1+R2 +R3, and then performing the following step (a2-iii-7);

(a2-iii-3) if the estimated genotype of the target site is AB|AA, estimating the count (FC) derived from the least component DNA as R1-R2, and the total count (TC) as R1+R2 or R1+R2+R3, and then performing the following step (a2-iii-7);

(a2-iii-4) if the estimated genotype of the target site is AA|AB, estimating the count (FC) derived from the least component DNA as 2 times R2, and the total count (TC) as R1+R2 or R1+R2+R3, and then performing the following step (a2-iii-7);

(a2-iii-5) if the estimated genotype of the target site is AB|AC, estimating the count (FC) derived from the least component DNA as R1-R2+R3 or 2 times R3 or 2 times (R1-R2), and the total count (TC) as R1+R2+R3, and then performing the following step (a2-iii-7);

(a2-iii-6) if the estimated genotype of the target site is not one of the above-mentioned genotypes, estimating the count (FC) derived from the least component DNA as NA, and the total count (TC) as R1 or R1+R2 or R1+R2+R3, and then performing the following step (a2-iii-7);

(a2-iii-7) outputting the estimated count (FC) derived from the least component DNA and total count (TC).

In some embodiments, the calculation module in step (a3) uses linear regression or robust linear regression to calculate the concentration of the least component DNA in the sample, or uses the mean or median of FC and TC to calculate the concentration of the least component DNA in the sample, according to FC and TC counts.

In some embodiments, the invention relates to a device for calculating a concentration of the least component DNA in a sample, said device comprising:

(b1) a setting module, which sets a noise threshold $\alpha$, an initial concentration estimation value $f_0$ and an iteration error precision value $\varepsilon$ of the sample;

(b2) a module for with respect to each target DNA site, using counts of its individual alleles and the concentration value $f_0$ of the least component DNA in the sample to estimate its genotype;

(b3) an estimation module, which for each target DNA site, estimates the count (FC) derived from the least component DNA and total count (TC) based on its estimated genotype;

(b4) a module for using the count (FC) of the least component DNA and total count (TC) to estimate the concentration f of the least component DNA;

(b5) a determination module, which determines whether the absolute value of f-$f_0$ is less than $\varepsilon$, and if the determination result is no, then sets $f_0$=f, and then performs step (b2); if the determination result is yes, estimates the least component DNA concentration in the sample as f.

In some embodiments, with respect to each target DNA site, using counts of its allele and the concentration value $f_0$ of the least component DNA in the sample to estimate its genotype comprises the steps of:

(b2-i) listing all possible genotypes of the target DNA site according to the source of the sample;

(b2-ii) for each possible genotype of the target DNA site, using the concentration value $f_0$ of the least component DNA in the sample and the total count (TC) of individual alleles of the target DNA site to calculate theoretical counts of individual alleles thereof;

(b2-iii) for each possible genotype of the target DNA site, using the counts of individual alleles of the target DNA site and theoretical counts of individual alleles thereof to perform a goodness-of-fit test;

(b2-iv) analyzing results of the goodness-of-fit test of the target DNA site for all possible genotypes, and selecting the genotype with the best fit for each allele count of the target DNA site as the estimated target DNA site genotype.

In some embodiments, for each target DNA site, estimating the count (FC) derived from the least component DNA and total count (TC) based on its estimated genotype, wherein the maximal four allele counts are marked as R1, R2, R3, and R4 in sequence, comprises the steps of:

(b3-1) if the estimated genotype of the target site is AA|AA, estimating the count (FC) derived from the least component DNA as NA, and the total count (TC) as R1 or R1+R2 or R1+R2+R3 or R1+R2+R3+R4, and then performing the following step (b3-11);

(b3-2) if the estimated genotype of the target site is AB AB, estimating the count (FC) derived from the least component DNA as NA, and the total count (TC) as R1+R2 or R1+R2+R3 or R1+R2+R3+R4, and then performing the following step (b3-11);

(b3-3) if the estimated genotype of the target site is AB|AA, estimating the count (FC) derived from the least component DNA as R1-R2, and the total count (TC) as R1+R2 or R1+R2+R3 or R1+R2+R3+R4, and then performing the following step (b3-11);

(b3-4) if the estimated genotype of the target site is AA|AB, estimating the count (FC) derived from the least component DNA as 2 times R2, and the total count (TC) as R1+R2 or R1+R2+R3 or R1+R2+R3+R4, and then performing the following step (b3-11);

(b3-5) if the estimated genotype of the target site is AB|AC, estimating the count (FC) derived from the least component DNA as R1-R2+R3 or 2 times R3 or 2 times (R1-R2), and the total count (TC) as R1+R2+R3 or R1+R2+R3+R4, and then performing the following step (b3-11);

(b3-6) if the estimated genotype of the target site is AA|BB, estimating the count (FC) derived from the least component DNA as R2, and the total count (TC) as R1+R2 or R1+R2+R3 or R1+R2+R3+R4, and then performing the following step (b3-11);

(b3-7) if the estimated genotype of the target site is AA|BC, estimating the count (FC) derived from the least component DNA as R2+R3 or 2 times R2 or 2 times R3, and the total count (TC) as R1+R2+R3 or R1+R2+R3+R4, and then performing the following step (b3-11);

(b3-8) if the estimated genotype of the target site is AB|CC, then determining whether the current estimated value $f_0$ is greater than or equal to 1/3, and if the determination result is yes, estimating the count (FC) derived from the least component DNA as R1, and the total count (TC) as R1+R2+R3 or R1+R2+R3+R4, and then performing the following step (b3-11); if the determination result is no, estimating the count (FC) derived from the least component DNA as R3, and the total count (TC) as R1+R2+R3 or R1+R2+R3+R4, and then performing the following step (b3-11);

(b3-9) if the estimated genotype of the target site is AB|CD, estimating the count (FC) derived from the least component DNA as R3+R4 or 2 times R3 or 2 times R4, and the total count (TC) as R1+R2+R3+R4, and then performing the following step (b3-11);

(b3-10) if the estimated genotype of the target site is not one of the above-mentioned genotypes, estimating the count (FC) derived from the least component DNA as NA, and the total count (TC) as R1 or R1+R2 or R1+R2+R3 or R1+R2+R3+R4, and then performing the following step (b3-11);

(b3-11) outputting the estimated count (FC) derived from the least component DNA and total count (TC).

In some embodiments, the sample is a plasma sample of a pregnant woman, and the least component DNA is fetal DNA. In some embodiments, the sample is an embryonic nucleic acid from preimplantation diagnosis.

In some embodiments, the invention provides a diagnostic kit for implementing the present methods. The diagnostic kit comprises at least one set of primers to amplify target DNA sites in a reference group and/or target DNA sites in a target group. Among them, target DNA sites in the target group are selected from chromosomes with possible chromosomal aneuploidy abnormalities and/or chromosome fragments with possible copy number variations and/or possible pathogenic variation sites of monogenic diseases. Among them, nucleic acid sequences of the target DNA sites in the target group generally have polymorphisms in the population to be tested and/or are possible pathogenic variation sites of monogenic diseases. Among them, the target DNA sites in the reference group are selected from chromosomes that usually have no chromosomal aneuploidy abnormality and/or chromosome fragments that usually have no copy number variation. Among them, the nucleic acid sequences of the target DNA sites in the reference group generally have polymorphisms in the population to be tested. In some embodiments, the target DNA sites in the reference group are selected from chromosomal regions in a sample that are considered to be free of chromosomal aneuploidy abnormalities or copy number variations of chromosomal fragments. In some embodiments, the reference chromosomes or reference chromosomal regions are selected from chromosomes 1, 2, 3, 4, 5, 6, 7, 8, 9, 10, 11, 12, 13, 14, 15, 16, 17, 18, 19, 20, 21, 22, X and Y, and sometimes the reference chromosomes or reference chromosomal regions are selected from an autosome (i.e., not X and Y). In some embodiments, the target DNA sites of interest are selected from chromosomal regions in a sample that are considered to have chromosomal aneuploidy abnormalities or copy number variations of chromosomal fragments. In some embodiments, the target DNA sites of interest are selected from nucleic acid regions in a sample where a pathogenic variation site of a monogenic disease is believed to exist and/or may exist. In some embodiments, chromosomal regions of interest are selected from chromosomes 1, 2, 3, 4, 5, 6, 7, 8, 9, 10, 11, 12, 13, 14, 15, 16, 17, 18, 19, 20, 21, 22, X and Y. Preferably, the target DNA sites in the target group are selected from chromosome 13 and/or chromosome 18 and/or chromosome 21 and/or chromosome X and/or chromosome Y. Preferably, the kit comprises primers for amplifying target nucleic acids derived from chromosome 13, 18, 21, X and/or Y. Preferably, the target DNA sites in the target group are selected from chromosomal regions of 1p36 deletion syndrome, cri du chat syndrome, Charcot-Marie-Tooth disease, Digeorge syndrome, Duchenne muscular dystrophy, Williams-Beuren syndrome, Wolf-Hirschhorn syndrome, 15q13.3 micro-deletion syndrome, Miller-Dieker syndrome, Smith-Magenis syndrome, Angelman syndrome, Langer-Giedion syndrome. Preferably, the kit comprises primers for amplifying nucleic acids of interest derived from chromosomal regions of 1p36 deletion syndrome, cri du chat syndrome, Charcot-Marie-Tooth disease, Digeorge syndrome, Duchenne muscular dystrophy, Williams-Beuren syndrome, Wolf-Hirschhorn syndrome, 15q13.3 micro-deletion syndrome, Miller-Dieker syndrome, Smith-Magenis syndrome, Angelman syndrome, Langer-Giedion syndrome. It is understood that the reference chromosome or portion thereof comprising the region of the target site is a euploid chromosome. Euploid refers to a normal number of chromosomes. Other reagents that may be optionally included in the diagnostic kit are instructions for use, polymerases and buffers for performing PCR and/or multiplex PCR reactions and reagents required for constructing a high-throughput sequencing library of the amplified fragments.

In some embodiments, the invention provides a diagnostic kit for implementing the present methods. The diagnostic kit comprises primers for performing step (2) and/or step (3). Other reagents that may be optionally included in the diagnostic kit are instructions for use, polymerases and buffers for performing PCR and/or multiplex PCR reactions and reagents required for constructing a high-throughput sequencing library of the amplified fragments.

In some embodiments, the present invention provides a system for implementing the present methods, which is used to implement one or more steps, such as one or more of steps (4) to (5), in the methods of predicting the karyotype or genotype or wild-mutant type of a target to be detected from a biological test sample. In some embodiments, the present invention provides a device and/or computer program product and/or system and/or module for implementing the present methods, which is used for carrying out any step of the above-mentioned step (1) to step (5), the above-mentioned step (a1) to step (a3), the above-mentioned step (b1) to step (b5), the above-mentioned step (c1) to step (c3), the above-mentioned step (d1) to step (d3) and/or the above-mentioned step (e1) to step (e3).

In one aspect, the invention relates to the following embodiments:

1. A method for detecting genetic variation of a sample, characterized in that it comprises the following steps in sequence:

(1) receiving a biological sample to be tested and preparing nucleic acid;
(2) enriching or amplifying target DNA sequences, wherein at least one target DNA sequence has more than one allele in the sample;
(3) sequencing the amplified target DNA;
(4) for each target DNA sequence, counting the counts of individual alleles;
(5) determining the karyotype or genotype or wild-mutant type of a target site to be detected in a sample by using a goodness-of-fit test of allele counts and/or a relative distribution diagram of allele counts.

2. The method according to embodiment 1, characterized in that in step (5), the karyotype or genotype or wild-mutant type of the target site to be detected in the sample is determined by using the goodness-of-fit test of allele counts, and said determining comprises the following steps in sequence:

(A1) dividing target DNA sequences into sequences of a reference group and sequences of a target group according to their location on the chromosome;
(A2) calculating a concentration of the least component DNA in the sample using the allele counts of each target DNA sequence in the reference group, by using a relative ratio method of allele counts or an iterative fitting genotype method of allele counts;
(A3) estimating the karyotype or genotype or wild-mutant type of the target site to be detected in the sample by using the goodness-of-fit test of allele counts of each target DNA sequence in the target group.

3. The method according to embodiment 1, characterized in that in step (5), the karyotype or genotype or wild-mutant type of the target site to be detected in the sample is determined by using the relative distribution diagram of allele counts, and the determining comprises the following steps in sequence:

(B1) dividing target DNA sequences into sequences of a reference group and sequences of a target group according to their location on the chromosome;
(B2) calculating a concentration of the least component DNA in the sample using the allele counts of each target DNA sequence in the reference group, by using a relative ratio method of allele counts or an iterative fitting genotype method of allele counts;
(B3) estimating the karyotype or genotype or wild-mutant type of the target site to be detected in the sample by using the relative distribution diagram of allele counts of each target DNA sequence in the target group.

4. The method according to embodiment 1, characterized in that in step (5), the karyotype or genotype or wild-mutant type of the target site to be detected in the sample is determined by using the relative distribution diagram of allele counts, wherein the sample to be detected is a single genome sample, and the determining comprises the following steps in sequence:

(C1) calculating the relative counts of individual alleles of each target DNA sequence in the target group;
(C2) for each target DNA sequence, plotting a distribution diagram A of the second maximal relative count of alleles to the maximal relative count of alleles or plotting a distribution diagram B of the maximal relative count of alleles to the relative position of the target DNA sequence on the chromosome or sub-chromosome;
(C3) estimating the karyotype of the target region to be detected in a single genome sample by using the relative distribution diagram A and/or distribution diagram B of allele counts of each target DNA sequence in the target group.

5. The method according to embodiment 2 or 3, characterized in that in step (A2) or step (B2), the relative ratio method of allele counts is used for calculating the concentration of the least component DNA in the sample, and the calculating comprises the following steps in sequence:

(a1) setting a noise background value $\alpha$ of the sample;
(a2) estimating the count (FC) derived from the least component DNA and total count (TC) for each target DNA sequence;
(a3) estimating the concentration of the least component DNA by using the count (FC) and the total count (TC) of the least component DNA.

6. The method according to embodiment 2 or 3, characterized in that in step (A2) or step (B2), the iterative fitting genotype method of allele counts is used for calculating the concentration of the least component DNA in the sample, and the calculating comprises the following steps in sequence:

(b1) setting a noise background value $\alpha$, an initial concentration estimation value $f_0$ and an iteration error precision value $\varepsilon$ of the sample;
(b2) for each target DNA sequence, using counts of its individual alleles and $f_0$ to estimate its genotype;
(b3) for each target DNA sequence, estimating the count (FC) derived from the least component DNA and total count (TC) based on its estimated genotype;
(b4) using the count (FC) of the least component DNA and total count (TC) to estimate the concentration f of the least component DNA;
(b5) determining whether the absolute value of $f-f_0$ is less than $\varepsilon$, and if the determination result is no, then setting $f_0=f$, and then performing step (b2); if the determination result is yes, estimating the least component DNA concentration in the sample as f.

7. The method according to embodiment 2, characterized in that in step (A3), the goodness-of-fit test of allele counts of each target DNA sequence in the target group is used for estimating the genotype of the target site to be detected in the sample, and the estimating comprises the following steps in sequence:

(A3.a1) analyzing the target DNA sequence site of interest and listing all possible genotypes thereof;
(A3.a2) for each possible genotype, calculating theoretical counts of individual alleles thereof according to the least component DNA concentration as estimated and the total count of the target DNA sequence, and then using the individual allele counts and theoretical counts of the target DNA sequence to perform a goodness-of-fit test;
(A3.a3) selecting a genotype with the best fit as the genotype of the target DNA sequence site, according to the goodness-of-fit test results for all possible genotypes of the target DNA sequence.

8. The method according to embodiment 2, characterized in that in step (A3), the goodness-of-fit test of allele counts of each target DNA sequence in the target group is used for estimating the karyotype of the target site to be detected in the sample, and the estimating comprises the following steps in sequence:

(A3.b1) analyzing the sample to be tested, and listing all possible karyotypes of the sample on the chromosomal or sub-chromosomal fragment;

(A3.b2) for each possible karyotype, listing all possible genotypes for target DNA sequences in the target group on the chromosome or sub-chromosome of that karyotype in the sample;

(A3.b3) for each target DNA sequence in the target group, firstly using the counts of individual alleles to perform a goodness-of-fit test for all possible genotypes, and then for each karyotype, selecting a genotype with the best fit for such karyotype;

(A3.b4) comprehensively analyzing the goodness-of-fit test results of all target DNA sequences for all karyotypes, and selecting the karyotype with the best comprehensive fit for all target DNA sequences as the karyotype of the target chromosomal or sub-chromosomal fragment to be detected.

9. The method according to embodiment 2, characterized in that in step (A3), the goodness-of-fit test of allele counts of each target DNA sequence in the target group is used for estimating the wild-mutant type of the target site to be detected in the sample, and the estimating comprises the following steps in sequence:

(A3.c1) estimating the genotype of the target DNA sequence by using the counts of individual alleles of the target DNA and a goodness-of-fit test;

(A3.c2) determining the wild-mutant type of each allele of the target DNA sequence in each component of the sample, according to the genotype of the target DNA sequence and the wild-mutant sequence of each allele thereof.

10. The method according to embodiment 3, characterized in that in step (B3), the relative distribution of allele counts of each target DNA sequence in the target group is used for estimating the genotype of the target site to be detected in the sample, and the estimating comprises the following steps in sequence:

(B3.a1) analyzing the sample to be tested, and listing all possible genotypes of the target sequence site of interest;

(B3.a2) calculating theoretical values of relative counts of individual alleles in each possible genotype, and for each genotype, selecting at least one non-maximal theoretical value of relative counts of alleles to be plotted against the maximal theoretical value of relative counts of alleles to mark theoretical positions of all possible genotypes;

(B3.a3) calculating relative counts of individual alleles of the target DNA sequence, and selecting at least one non-maximal relative count of alleles to be plotted against the maximal relative count of alleles to mark actual positions of relative counts of alleles of the target DNA sequence;

(B3.a4) inferring the genotype thereof according to the theoretical position distribution and actual position distribution of the target DNA sequence in the relative count map of alleles.

11. The method according to embodiment 3, characterized in that in step (B3), the relative distribution diagram of allele counts of each target DNA sequence in the target group is used for estimating the karyotype of the target site to be detected in the sample, and the estimating comprises the following steps in sequence:

(B3.b1) analyzing the sample to be tested and listing all possible karyotypes of the sample on the target chromosomal or sub-chromosomal fragment;

(B3.b2) for each possible karyotype, listing all possible genotypes of the target DNA sequences in the target group on the chromosome or sub-chromosome for such karyotype in the sample, and then for each genotype, selecting at least one non-maximal theoretical value of relative counts of alleles to be plotted against the maximal theoretical value of relative counts of alleles to mark a theoretical position of such genotype;

(B3.b3) for each target DNA sequence in the target group, calculating relative counts of individual alleles and selecting at least one non-maximal relative count of alleles to be plotted against the maximal relative count of alleles to mark the actual location of such site;

(B3.b4) inferring the karyotype of the target chromosomal or sub-chromosomal fragment to be detected according to the theoretical position distribution and actual position distribution of all target DNA sequences in the relative count map of alleles.

12. The method according to embodiment 3, characterized in that in step (B3), the relative distribution diagram of allele counts of each target DNA sequence in the target group is used for estimating the wild-mutant type of the target site to be detected in the sample, and the estimating comprises the following steps in sequence:

(B3.c1) analyzing the sample to be tested, and listing the wild-type sequence of the target sequence site of interest and all possible genotypes thereof;

(B3.c2) calculating theoretical values of relative counts of the wild-type allele and other non-wild-type alleles for each possible genotype, and for each genotype, selecting at least one theoretical value of relative counts of non-wild-type alleles to be plotted against the theoretical value of the relative count of the wild-type allele to mark theoretical positions of all possible genotypes;

(B3.c3) calculating relative counts of the wild-type allele and other non-wild-type alleles of the target DNA sequence, and selecting at least one relative count of non-wild-type alleles to be plotted against the relative count of the wild-type allele to mark the actual position of relative counts of alleles of the target DNA sequence;

(B3.c4) inferring the wild-mutant type thereof according to the theoretical position distribution and actual position distribution of the target DNA sequence in the relative count map of alleles.

13. The method according to embodiment 5, characterized in that the estimating performed in step (a2) comprises the following steps in sequence:

(i) sorting the allele counts of the target DNA sequence in descending order, wherein the maximal three allele counts are marked as R1, R2 and R3 in sequence;

(ii) determining the number of alleles that are detected to be higher than the noise threshold in the target DNA sequence; if the determination result is 1, estimating the genotype of the target DNA sequence as AA|AA, and performing the following step (vi); if the determination result is 2, performing the following step (iii); if the determination result is greater than 2, performing the following step (v);

(iii) determining whether the value of R1/(R1+R2) is less than $0.5+\alpha$, and if the determination result is yes, estimating the genotype of the target DNA sequence as ABIAB, and then performing the following step (vi); if the determination result is no, then performing the following step (iv);

(iv) determining whether the value of R1/(R1+R2) is less than 0.75, and if the determination result is yes, estimating the genotype of the target DNA sequence as AB|AA, and then performing the following step (vi); if the determination result is no, estimating the genotype of the target DNA sequence as AAIAB, and then performing the following step (vi);

(v) determining whether the value of R2/R1 is less than 0.5, and if the determination result is no, estimating the genotype of the target DNA sequence as AB|AC, and then performing the following step (vi); if the determination result is yes, marking the target DNA sequence as an outlier, and then either estimating the genotype of the target DNA sequence as NA, and then performing the following step (vi), or performing the above step (iii);

(vi) based on the estimated genotype of the target DNA sequence, estimating the count (FC) derived from the least component DNA and total count (TC).

14. The method according to embodiment 6, characterized in that the estimating performed in step (b2) comprises the following steps in sequence:

(i) listing all possible genotypes of the target DNA sequence according to the source of the sample;

(ii) for all possible genotypes of the target DNA sequence, using $f_0$ and the total count (TC) of individual alleles of the target DNA sequence to calculate the theoretical counts of its individual alleles;

(iii) using the counts of individual alleles of the target DNA sequence and theoretical counts of individual alleles thereof to perform a goodness-of-fit test;

(iv) analyzing results of the goodness-of-fit test of the target DNA sequence for all possible genotypes, and selecting the genotype with the best fit for each allele count of the target DNA sequence as the estimated genotype of the target DNA sequence.

## Description of drawings

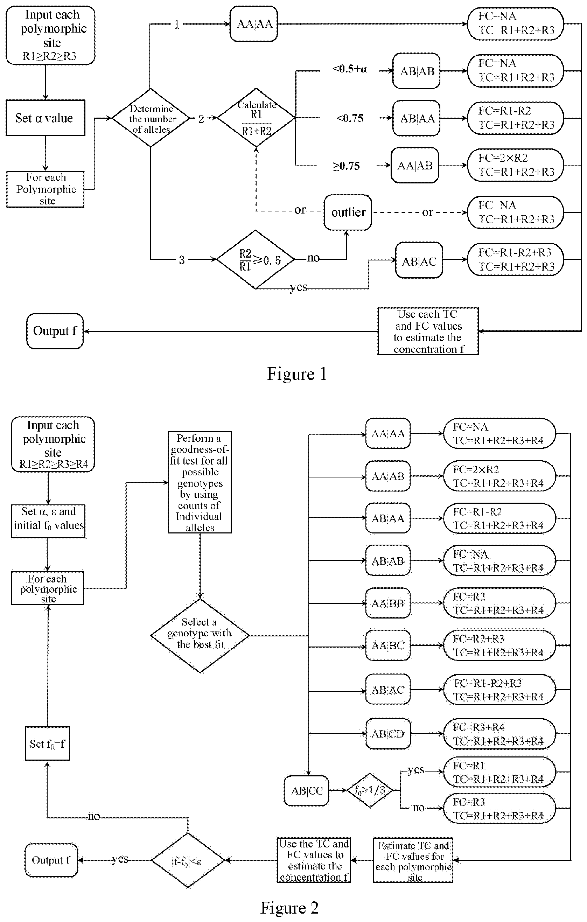

Figure 1 is a schematic flow chart of estimating a fetal DNA concentration by using counts of individual alleles at multiple polymorphic sites in a plasma cfDNA sample of a pregnant woman.

Figure 2 is a schematic flow chart of estimating a DNA concentration of the least component by using counts of individual alleles at multiple polymorphic sites in a mixed sample of two components.

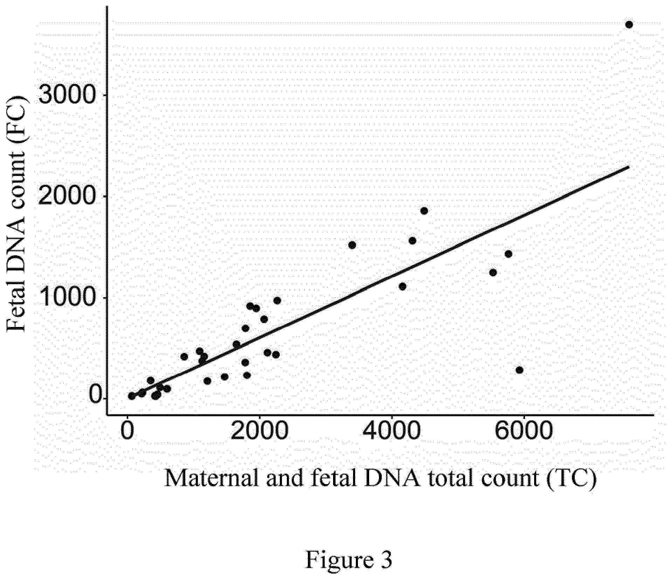

Figure 3 shows the estimation of a fetal DNA concentration by using polymorphic site sequencing in a plasma cfDNA sample of a pregnant woman. Firstly, the individual allele counts of individual polymorphic sites were used to estimate the fetal DNA count (FC) and mother and fetal DNA total count (TC), and then an rlm robust regression fit across the origin was performed for the FC and TC counts of all polymorphic sites, and the fetal DNA concentration was estimated as the slope of this fitted line (model coefficient).

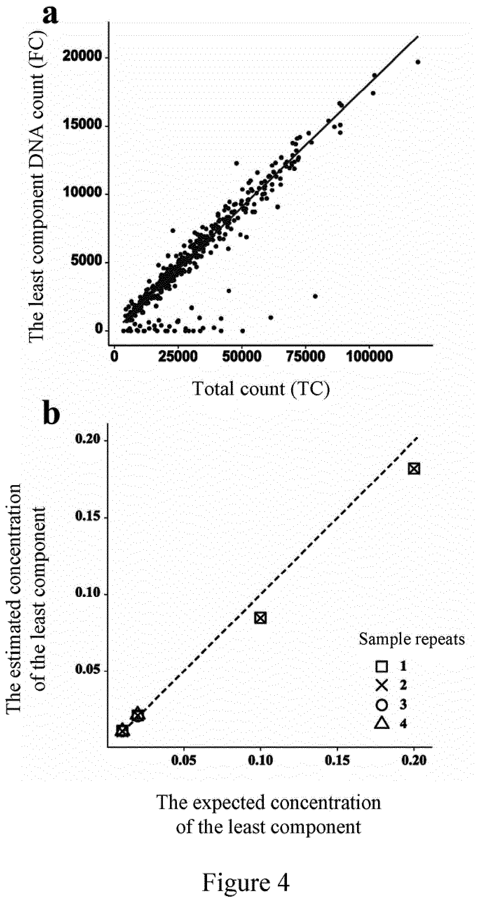

Figure 4 shows the estimation of the least component DNA concentration by using polymorphic site sequencing in a mixed component DNA sample. The individual allele counts at each polymorphic site were used to estimate the count (FC) of its least component DNA and the total count (TC) of all component DNA at that site. In Figure 4a, the rlm robust regression across the origin was performed by using the FC and TC values at each polymorphic site, and the least component DNA concentration was estimated as the slope of the line (model coefficient). Figure 4b is the result of estimating the concentration of the sample having the least component DNA by performing the rlm robust regression on multiple different samples or different biological replicates. Multiple replicates were performed for four mixed samples at the library preparation or sequencing level, and the expected least component DNA concentrations were 0.01, 0.02, 0.10, or 0.20 (x-axis), respectively, and the estimated least component DNA concentration per sample was y-axis. The dotted line in the figure indicates the position of the line y=x.

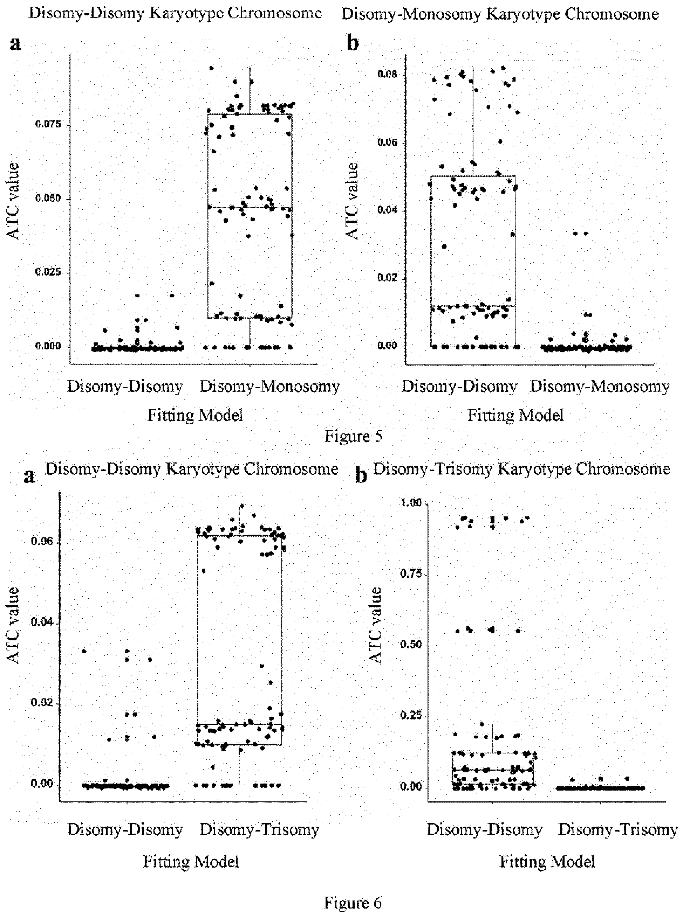

Figure 5 shows the detection of monosomy variations in fetal chromosomes by using individual allele counts at polymorphic sites. Figure 5a shows using the results of a comprehensive goodness-of-fit test to detect whether the disomy-disomy karyotype chromosomes in a simulated plasma cfDNA sample of a pregnant woman is a fetal monosomy abnormality. Figure 5b shows using the result of a comprehensive goodness-of-fit test to detect whether the disomy-monosomy karyotype chromosome in a simulated plasma cfDNA sample of a pregnant woman is a fetal monosomy abnormality. The AIC value on the y-axis is the corrected AIC value, which is obtained by dividing the AIC value of the G test at the site by the fetal concentration and then dividing it by the total count of individual alleles at the site.

Figure 6 shows the detection of trisomy variations in fetal chromosomes by using individual allele counts at polymorphic sites. Figure 6a shows using the results of a comprehensive goodness-of-fit test to detect whether the disomy-disomy karyotype chromosome in a simulated plasma cfDNA sample of a pregnant woman is a fetal trisomy abnormality. Figure 6b shows using the results of a comprehensive goodness-of-fit test to detect whether the disomy-trisomy karyotype chromosome in a simulated plasma cfDNA sample of a pregnant woman is a fetal trisomy abnormality.

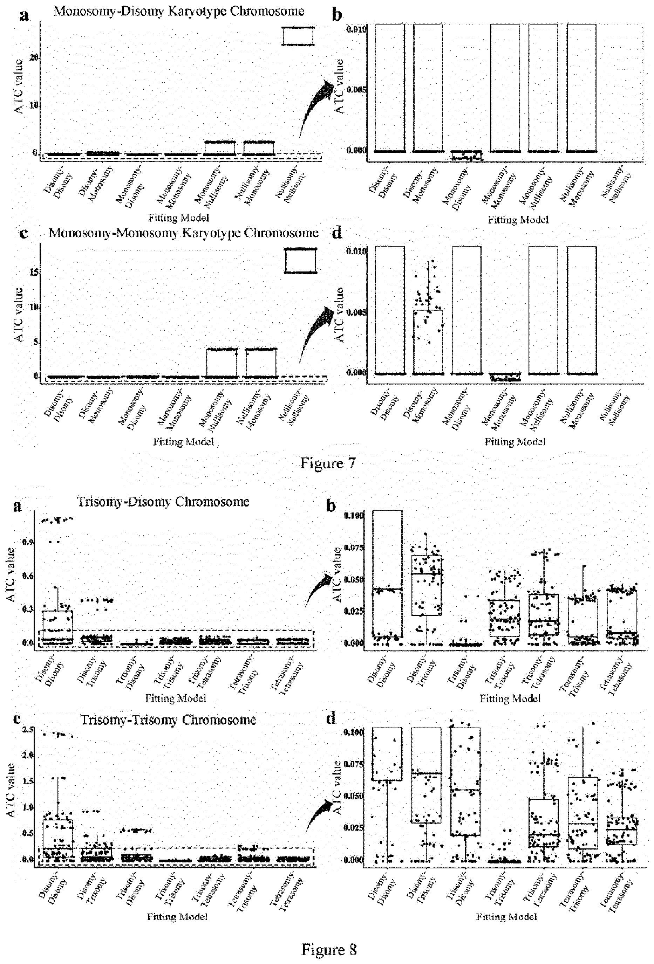

Figure 7 shows the estimation of micro-deletion variations at the sub-chromosomal level of the fetus to be detected by using the counts of individual alleles at polymorphic sites. Figure 7a shows using the results of a comprehensive goodness-of-fit test to detect whether the monosomy-disomy karyotype chromosome in a simulated plasma cfDNA sample of a pregnant woman is a micro-deletion abnormality of the fetal chromosome. Figure 7b is a partial enlargement of Figure 7a. Figure 7c shows using the results of a comprehensive goodness-of-fit test to detect whether the monosomy-monosomy karyotype chromosome in a simulated plasma cfDNA sample of a pregnant woman is a micro-deletion abnormality of the fetal chromosome. Figure 7d is a partial enlargement of Figure 7c.

Figure 8 shows the estimation of micro-duplication variations at the sub-chromosomal level of the fetus to be detected by using the counts of individual alleles at polymorphic sites. Figure 8a shows using the results of a comprehensive goodness-of-fit test to detect whether the trisomy-disomy karyotype chromosome in a simulated plasma cfDNA sample of a pregnant woman is a micro-duplication abnormality of the fetal chromosome. Figure 8b is a partial enlargement of Figure 8a. Figure 8c shows using the results of a comprehensive goodness-of-fit test to detect whether the trisomy-trisomy karyotype chromosome in a simulated plasma cfDNA sample of a pregnant woman is a micro-duplication abnormality of the fetal chromosome. Figure 8d is a partial enlargement of Figure 8c.

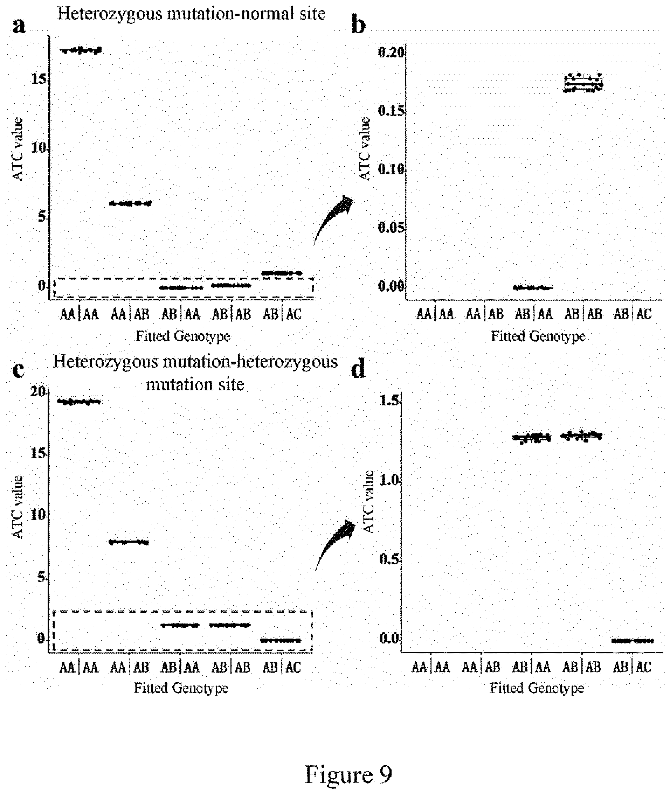

Figure 9 shows the detection of the wild-mutant type of a fetus at the short-sequence level by using the counts of individual alleles at a polymorphic site. Figure 9a shows the detection of the genotype of a site of a simulated short sequence where the mother has a heterozygous mutation and the fetus is normal by using the result of a goodness-of-fit test. Figure 9b is a partial enlargement of Figure 9a. The results showed that the estimated genotype of this genetic site was AB|AA, that is, a genotype wherein the mother was heterozygous and the fetus was homozygous. Further analysis of the allele sequence found that allele A was wild-type and allele B was mutant type, so it was determined that the wild-mutant type at this site was one where the mother had a heterozygous mutation and the fetus was normal (Aa|AA). Figure 9c shows the detection of the genotype of a site of a simulated short sequence where the mother and the fetus both have a heterozygous mutation by using the result of a goodness-of-fit test. Figure 9d is a partial enlargement of Figure 9c. The results showed that the estimated genotype of this genetic site was AB|AC, that is, a genotype wherein both mother and fetus were heterozygous. Further analysis of the allele sequence found that allele A was wild-type and alleles B and C were mutant types, so it was determined that the wild-mutant type at this site was one where both mother and fetus had a heterozygous mutation (Aa|Ab), and the fetus either had a *de novo* mutation or inherited an allelic mutation derived from the father.

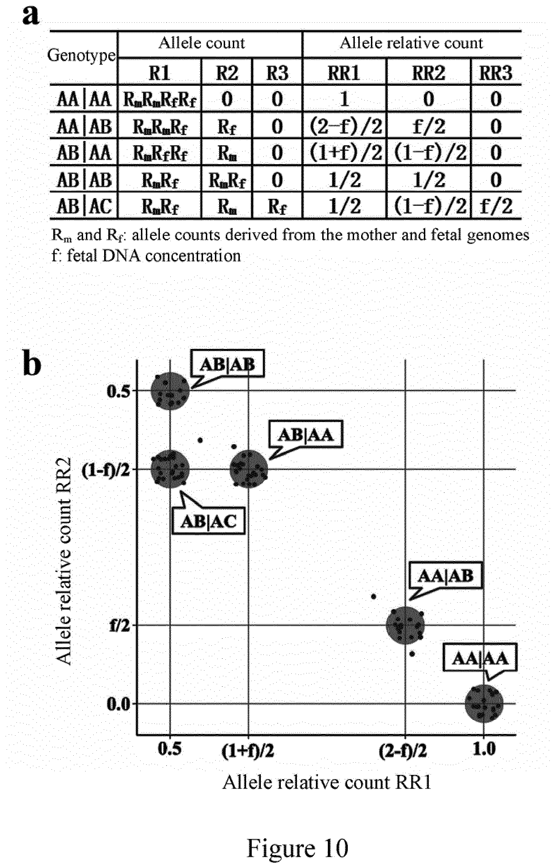

Figure 10 shows the estimated genotypes of a target site by using the relative distribution diagram of allele counts. Figure 10a shows the theoretical distribution of relative counts of individual alleles at polymorphic sites on the chromosome of a normal disomy-disomy karyotype. Figure 10b shows the distribution of the second maximal relative count of alleles relative to the maximal relative count of alleles at polymorphic sites on the chromosome of a normal disomy-disomy karyotype.

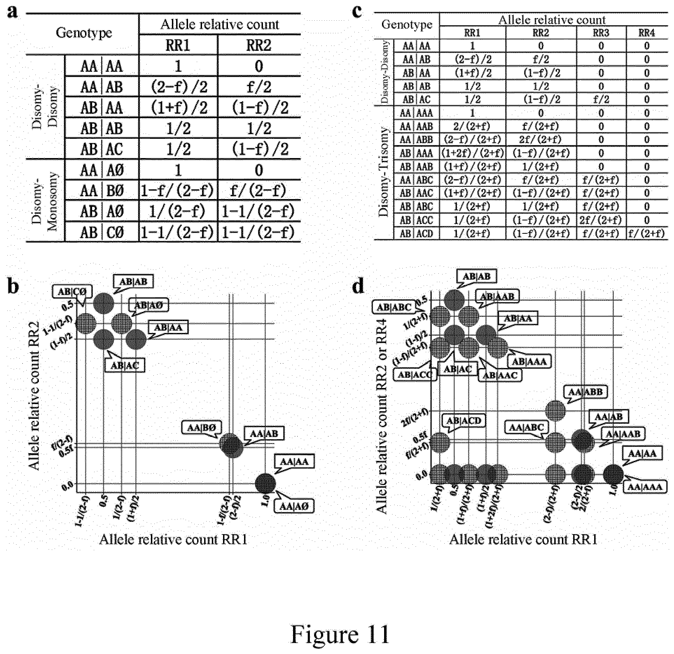

Figure 11 shows the theoretical distribution of relative counts of individual alleles at each polymorphic site on the chromosome where the mother is of a normal karyotype in a plasma cfDNA sample of a pregnant woman. Figure 11a shows all possible genotypes and the theoretical values of relative counts of their respective alleles at each polymorphic site on the chromosome with a disomy-disomy karyotype or a disomy-monosomy karyotype. Figure 11b shows the theoretical distribution of the second maximal relative count of alleles relative to the maximal relative count of alleles at each polymorphic site on chromosomes with a disomy-disomy karyotype and a disomy-monosomy karyotype. Figure 11c shows all possible genotypes and the theoretical values of relative counts of their respective alleles at each polymorphic site on the chromosome with a disomy-disomy karyotype or a disomy-trisomy karyotype. Figure 11d shows the theoretical distribution of the second or fourth maximal relative count of alleles relative to the maximal relative count of alleles at each polymorphic site on chromosomes with a disomy-disomy karyotype and a disomy-trisomy karyotype.

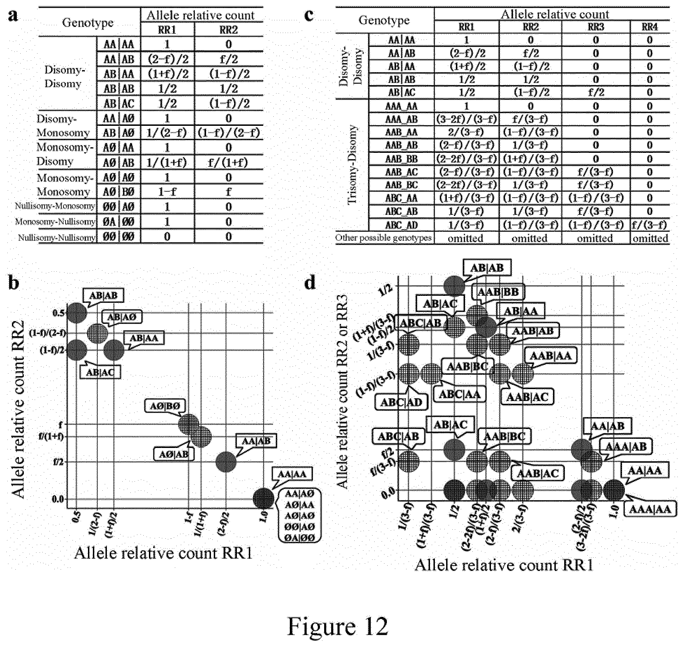

Figure 12 shows the theoretical distribution of relative counts of individual alleles at each polymorphic site at the sub-chromosomal level in the target group in a plasma cfDNA sample of a pregnant woman. Figure 12a shows all possible genotypes and the theoretical values of relative counts of their respective alleles at each polymorphic site on the chromosome wherein the mother or fetus has or doesn't have a micro-deletion karyotype. Figure 12b shows the theoretical distribution of the second maximal relative count of alleles relative to the maximal relative count of alleles at each polymorphic site on chromosomes wherein the mother or fetus has or doesn't have a micro-deletion karyotype. Figure 12c shows all possible genotypes and the theoretical values of relative counts of their respective alleles at each polymorphic site on the sub-chromosome where the mother has or doesn't have a micro-duplication and the fetus is normal. Figure 12d shows the theoretical distribution of the second or third maximal relative count of alleles relative to the maximal relative count of alleles at each polymorphic site on the sub-chromosome where the mother has or doesn't have a micro-duplication and the fetus has a normal karyotype.

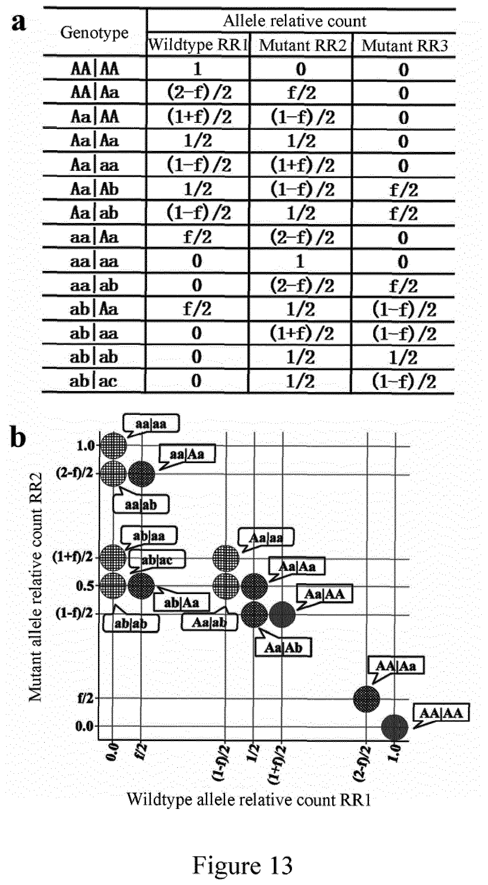

Figure 13 shows all possible genotypes and the theoretical distribution of their respective alleles of the site to be detected on the chromosome of a normal disomy-disomy karyotype in a plasma cfDNA sample of a pregnant woman. Figure 13a shows all possible genotypes and the theoretical values of relative counts of their respective alleles at the site to be detected on the chromosome of a normal disomy-disomy karyotype. Figure 13b shows a theoretical distribution diagram of the maximal relative count of non-wild-type alleles relative to the relative count of the wild-type allele for each possible genotype of the site to be detected on the chromosome of a normal disomy-disomy karyotype.

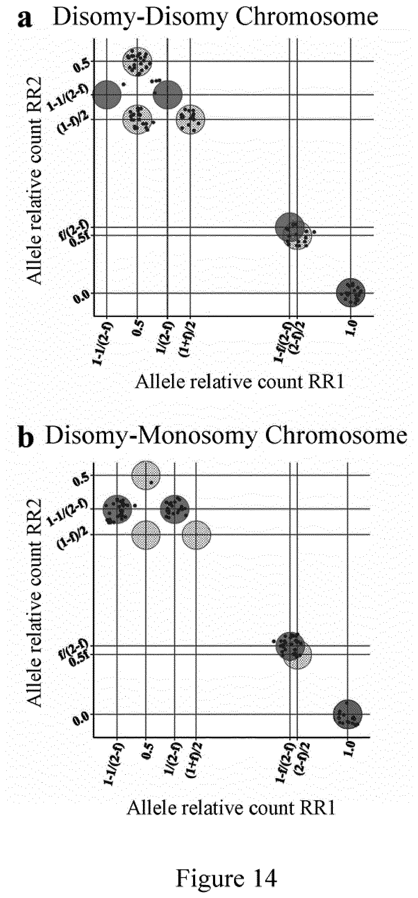

Figure 14 shows the detection of monosomy variations in fetal chromosomes by using the relative distribution diagram of the counts of individual alleles at polymorphic sites. Figure 14a shows the estimation of the karyotype of a normal disomy-disomy chromosome in a simulated plasma cfDNA sample of a pregnant woman by using the relative distribution diagram of the counts of alleles. Figure 14b shows the estimation of the karyotype of a disomy-monosomy chromosome in a simulated plasma cfDNA sample of a pregnant woman by using the relative distribution diagram of the counts of alleles.

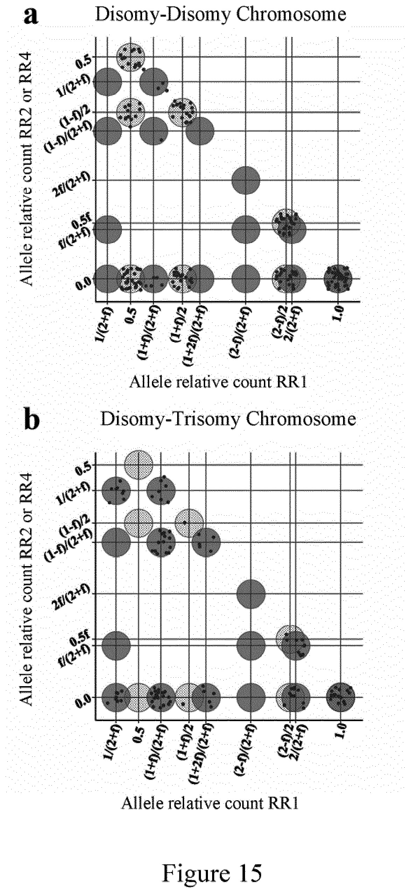

Figure 15 shows the detection of trisomy variations in fetal chromosomes by using the relative distribution diagram of the counts of individual alleles at polymorphic sites. Figure 15a shows the estimation of the karyotype of a normal disomy-disomy chromosome in a simulated plasma cfDNA sample of a pregnant woman by using the relative distribution diagram of the counts of alleles. Figure 15b shows the estimation of the karyotype of a disomy-trisomy chromosome in a simulated plasma cfDNA sample of a pregnant woman by using the relative distribution diagram of the counts of alleles.

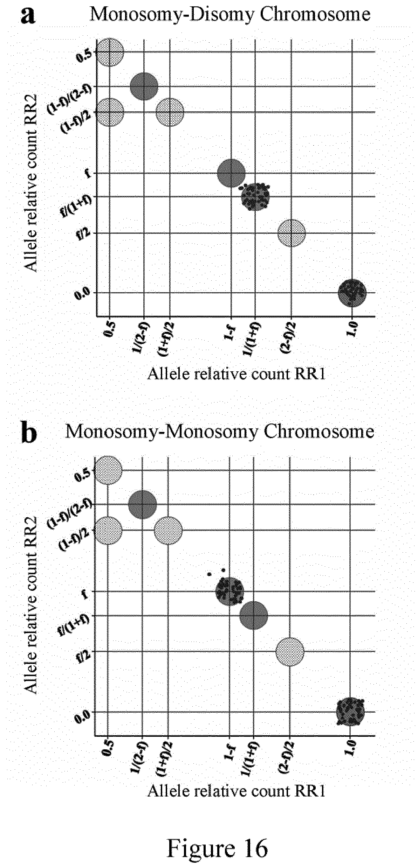

Figure 16 shows the detection of micro-deletion variations at the sub-chromosomal level of the fetus by using the relative distribution diagram of the counts of individual alleles at polymorphic sites. Figure 16a shows the estimation of the micro-deletion karyotype of a monosomy-disomy sub-chromosome in a simulated plasma cfDNA sample of a pregnant woman by using the relative distribution diagram of the counts of alleles. Figure 16b shows the estimation of the micro-deletion karyotype of a monosomy-monosomy sub-chromosome in a simulated plasma cfDNA sample of a pregnant woman by using the relative distribution diagram of the counts of alleles.

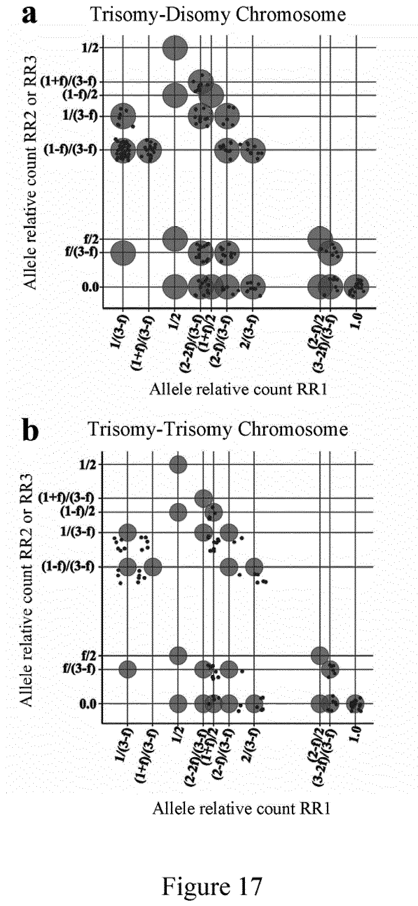

Figure 17 shows the detection of micro-duplication variations at the sub-chromosomal level of the fetus by using the relative distribution diagram of the counts of individual alleles at polymorphic sites. Figure 17a shows the estimation of the micro-duplication karyotype of a trisomy-disomy sub-chromosome in a simulated plasma cfDNA sample of a pregnant woman by using the relative distribution diagram of the counts of alleles. Figure 17b shows the estimation of the micro-duplication karyotype of a trisomy-trisomy sub-chromosome in a simulated plasma cfDNA sample of a pregnant woman by using the relative distribution diagram of the counts of alleles.

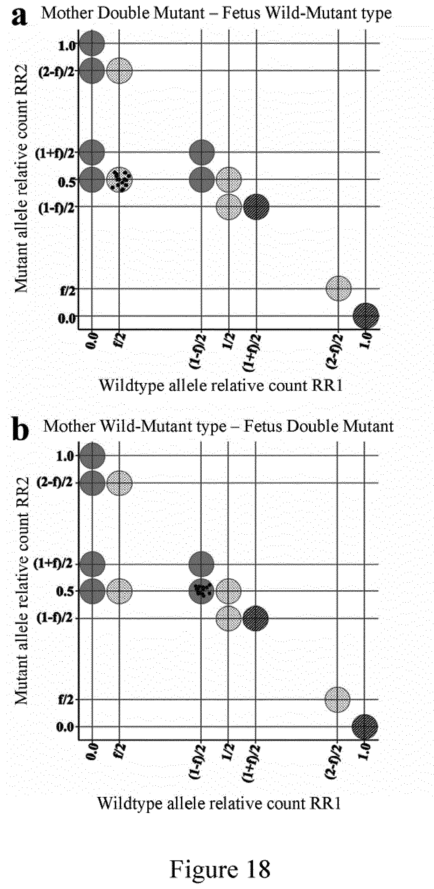

Figure 18 shows the detection of the wild-mutant type of the fetus at the short sequence level by using the relative distribution diagram of the counts of individual alleles at polymorphic sites. Figure 18a shows the estimation of the wild-mutant type of an ablaa genotype site in a simulated plasma cfDNA sample of a pregnant woman by using the relative distribution diagram of the counts of alleles. Figure 18b shows the estimation of the wild-mutant type of an Aalab genotype site in a simulated plasma cfDNA sample of a pregnant woman by using the relative distribution diagram of the counts of alleles.

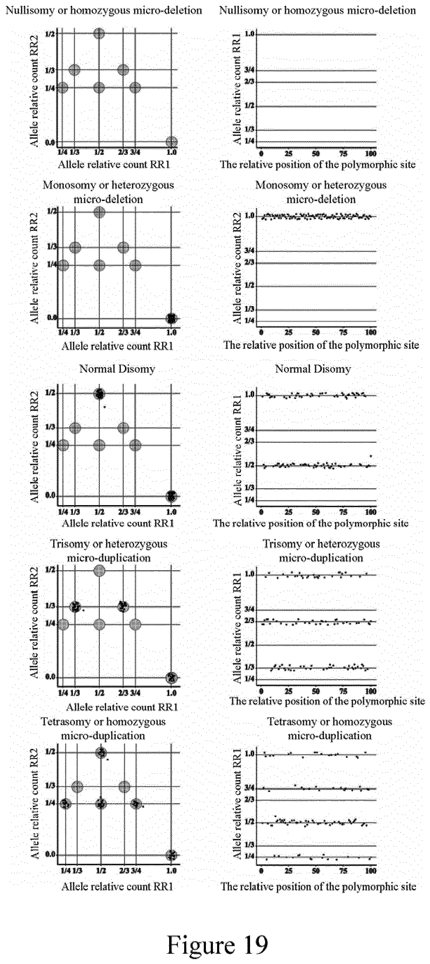

Figure 19 shows the detection of the karyotype of a chromosomal or sub-chromosomal fragment in the target group in a single-genome sample by using the relative counts of individual alleles at polymorphic sites. For each polymorphic site in the target group, the second maximal relative count of alleles is plotted against the maximal relative count of alleles (relative count map), or the maximal relative count of alleles is plotted against the relative position of the site on the simulated chromosome (relative count position map). The karyotype of the target to be detected can be estimated according to the distribution profile of each polymorphic site on the relative count map or the relative count position map.

**Detailed Description**

The present invention is further illustrated below in conjunction with specific Examples. It should be understood that these Examples are only used to illustrate the present invention and are not intended to limit the protection scope of the present invention. Any modifications or substitutions made to the methods, steps or conditions of the present invention without departing from the spirit and essence of the present invention fall within the scope of the present invention.

**Example 1. Analysis and calculation of the counts of individual alleles of each polymorphic site in plasma DNA samples of pregnant women**

In this example, the sequencing result file ((Barrett, Xiong et al. 2017, PLoS One 12:e0186771) came from the NIH SRA database (BioProject ID:PRJNA387652).

**1. Sample collection:** In this example, Barrett et al. collected 10-20 ml of peripheral blood from each pregnant woman, and then used QiaAmp Circulating Nucleic Acid kit (Qiagen) to extract plasma DNA (cfDNA) according to the manufacturer's protocol. In this example, we analyzed 157 plasma cfDNA samples collected using the method described above.

**2. Amplification and sequencing of polymorphic sites:** Barrett et al. selected 44 polymorphic sites with high minor allele frequencies (MAF>0.25) in the population, and then designed 45 pairs of amplification primers, including 44 pairs of sequence-specific amplification primers for polymorphic sites and a pair of amplification primers for the ZFX/ZFY site. Finally, each sample was amplified with 45 pairs of primers and multiplex PCR. The amplified products were used to prepare the sequencing library with TruSeq Nano DNA Sample Preparation kits (Illumina) according to the manufacturer's instructions. Sequencing was then performed using the MiSeq sequencer according to the manufacturer's instructions.

**3. Preparation of data analysis index:**

(3.1) Preparation of reference sequences of polymorphic sites: the forward primer and reverse primer of the 45 amplification sites reported by Barrett et al. and the specific locations of polymorphic sites on chromosomes were used to extract the reference sequence for each amplified product from the human genome sequence database.

(3.2) Preparation of a positioning index of polymorphic sites: for each reference sequence of the amplified products, it was manually divided into three regions, the 5' region, the variable region and the 3' region, wherein the variable region is the nucleic acid sequence region affected by any allele of the polymorphic site in the reference sequence of the amplified products, while the 5' region is the nucleic acid sequence from the beginning of the reference sequence to the beginning of the variable region in the 5' to 3' direction, and the 3' region is the nucleic acid sequence from the end of the variable region to the end of the reference sequence in the 5' to 3' direction. Then, for each polymorphic site, at least one set of unique sequences from the 5' region and the 3' region, respectively, was selected as the positioning index of the polymorphic site, wherein the unique sequence refers to the sequence which is unique in all the reference sequences of the amplified products of the polymorphic site, and the amplified product can be uniquely positioned to a specific polymorphic site by using this sequence.

(3.3) Preparation of a counting index of alleles of polymorphic sites: for each polymorphic site, firstly all its allele sequences were downloaded from the dbSNP database of NCBI, and then for each allele sequence, an unique nucleic acid sequence was selected as the counting index of alleles of the polymorphic site, wherein the unique nucleic acid sequence means that the unique nucleic acid sequence is unique among all possible amplification product reference sequences of the polymorphic site, and for the same allele of the site, the unique nucleic acid sequence is the same, and for different alleles of the site, the unique nucleic acid sequences are different from each other.

**4. Sequencing data analysis:** For each sequencing sequence, low-quality sequences were firstly filtered out, and then the positioning index of each polymorphic site was searched from beginning to end in the filtered sequencing sequences. If at least one positioning index of the polymorphic site was found, the sequence was mapped to a specific polymorphic site, otherwise, the sequence was discarded. Finally, for each sequence mapped to a specific polymorphic site, the counting index of alleles of the polymorphic site was searched from the beginning to the end. If at least one counting index of alleles was found, one of the indices of allele counts was selected and the sequence was marked as that allele for the polymorphic site, otherwise the sequence was discarded.

**5. Counting the counts of individual alleles of each polymorphic site:** For each sample, the number of sequenced sequences of each allele of each polymorphic site was counted, that is, the counts of individual alleles of each polymorphic site were counted.

**Example 2. Analysis and calculation of allele counts of individual polymorphic sites in two independent genome mixed samples**

In this example, the sequencing result file (Kim, Kim et al. 2019, Nat Commun 10: 1047) came from the NIH SRA database (BioProject ID: PRJNA517742).

**1. Sample collection:** In this example, Kim et al. extracted genomic DNA from two independent blood samples, one as the major component and the other as the minor component. The genomic DNA of the two samples was mixed according to a certain ratio, and the mixed samples were obtained with the proportions of the minor component were 0.01, 0.02, 0.10 and 0.20, respectively.

**2. Amplification and sequencing of polymorphic sites:** Kim et al. selected 645 polymorphic sites in two genome samples and designed amplification primers. Each mixed sample was amplified with amplification primers and multiplex PCR. The amplified products were prepared as sequencing libraries according to the process described in the manufacturer's instructions, respectively, and then sequenced using Ion Torrent or Illumina sequencers, respectively. In this example, we analyzed the Illumina sequencing data set (ILA data set) in the sequencing results.

**3. Preparation of data analysis index:** We used information such as the specific locations of 645 amplified sites on the chromosomes as reported by Kim et al., to extract the reference sequence of each amplified product from the human genome sequence database, and then used the process described in step (3.2) and step (3.3) in step 3 of Example 1, to prepare the positioning index of each polymorphic site and the counting index of each allele thereof.

**4. Sequencing data analysis:** For each sequencing sequence, low-quality sequences were firstly filtered out, and then the positioning index of each polymorphic site was searched from beginning to end in the filtered sequencing sequences. If at least one positioning index of the polymorphic site was found, the sequence was mapped to a specific polymorphic site, otherwise, the sequence was discarded. Finally, for each sequence mapped to a specific polymorphic site, the counting index of alleles of the polymorphic site was searched from the beginning to the end. If at least one counting index of alleles was found, one of the indices of allele counts was selected and the sequence was marked as that allele for the polymorphic site, otherwise the sequence was discarded.

**5. Counting the counts of individual alleles of each polymorphic site:** For each sample, the number of sequenced sequences of each allele of each polymorphic site was counted, that is, the counts of individual alleles of each polymorphic site were counted.

**Example 3. Computer simulation of each polymorphic site in a mixed sample and counting each allele thereof**

In this example, we followed the steps below to generate the respective allelic sequences of the simulated polymorphic sites.

**1. Simulation of polymorphic sites:** A 70bp-long unique sequence was firstly randomly generated, and divided into three regions, the 5' region (30bp in length), the variable region (10bp in length) and the 3' region (30bp in length). Then mutations (including nucleic acid sequence changes such as insertions, deletions, point mutations, and multiple site mutations) were randomly generated to the 10 bp sequence of the variable region, and at least six different nucleic sequences with a length greater than or equal to 60 bp, including the 5' region, the variable region, and the 3' region, were obtained, and marked as different alleles of the polymorphic site. Finally, at least one unique sequence of 12 bp length was selected as the positioning index of the polymorphic site according to step (3.2) in Example 1; at least one unique sequence of 12 bp length that includes the variable region was selected as the allele counting index of the polymorphic site according to step (3.3) in Example 1.

**2. Simulation of specific chromosomes or chromosome fragments in the sample:** For each chromosome number, at least 100 polymorphic sites were simulated according to the above step 1, and the number of simulated alleles and individual allele counts were determined according to the simulated genotype for each site.

For example, a polymorphic site on certain chromosome No. of the plasma cfDNA of a pregnant woman, whose karyotype is disomy-disomy, is simulated, and the genotype thereof may be AA|AA, AA|AB, AB|AA, AB|AB and AB|AC. Assuming that the concentration of fetal DNA in the sample is 10%, and the simulated genome copy number is 200, the fetal genome has 20 copies and the maternal genome has 180 copies. Firstly, a polymorphic site is selected, its allele sequences are listed and marked as A, B, C, D, E, F and so on, respectively. Then for genotype AA|AA, 200 copies of allele A are simulated; for genotype AA|AB, 180 copies of maternal allele A and 10 copies of fetal allele A and 10 copies of fetal allele B are simulated, i.e. 190 copies of allele A and 10 copies of allele B are simulated; for genotype ABIAA, 110 copies of allele A and 90 copies of allele B are simulated; for genotype AB|AB, 100 copies of allele A and 100 copies of allele B are simulated; for genotype AB|AC, 100 copies of allele A, 90 copies of allele B and 10 copies of allele C are simulated.

For example, a polymorphic site on certain chromosome No. in the plasma cfDNA of a pregnant woman whose karyotype is disomy-monosomy, or a polymorphic site on the plasma cfDNA of a pregnant woman wherein the karyotype of a fragment of certain chromosome No. is disomy-monosomy, is simulated, and the genotype thereof may be AA|AØ, AB|AØ, AA|BØ or ABICO. Assuming that the concentration of fetal DNA in the sample is 10%, and the simulated normal genome copy number is 200, the genome of the fetus is 20 copies and the genome of the mother is 180 copies. Firstly, a polymorphic site is selected, its allele sequences are listed and marked as A, B, C, D, E, F and so on, respectively. Then for genotype AAIAO, 190 copies of allele A are simulated; for genotype ABIAO, 100 copies of allele A and 90 copies of allele B are simulated; for genotype AA|BØ, 180 copies of allele A and 10 copies of allele B are simulated; for genotype ABICO, 90 copies of allele A, 90 copies of allele B and 10 copies of allele C are simulated.

The number of alleles of polymorphic sites on other chromosomes or chromosome fragments of different karyotypes and the genome copy number of individual alleles thereof can be simulated in a similar way.

**3. Simulation of specific samples:** Different chromosomes were simulated for each sample according to the purpose of the experiment, and at least 100 polymorphic sites were simulated for each chromosome or chromosome fragment according to the above step 2, and genome copy numbers of corresponding different alleles were simulated for each site according to the difference in genotype, where all allele copy numbers at each polymorphic site corresponded to 200 genome copies under a normal disomy-disomy karyotype.

**4. Simulation of high-throughput sequencing results:** The genome copy sequences of different polymorphic sites simulated for each sample were used as the input file, and the ART simulation software (Huang, Li et al. 2012, Bioinformatics 28:593-594) was used to simulate high-throughput sequencing results.

**5. Sequencing data analysis:** For each sequencing sequence, low-quality sequences were firstly filtered out, and then the positioning index of each polymorphic site was searched from beginning to end in the filtered sequencing sequences. If at least one positioning index of the polymorphic site was found, the sequence was mapped to a specific polymorphic site, otherwise, the sequence was discarded. Finally, for each sequence mapped to a specific polymorphic site, the counting index of alleles of the polymorphic site was searched from the beginning to the end. If at least one counting index of alleles was found, one of the indices of allele counts was selected and the sequence was marked as that allele for the polymorphic site, otherwise the sequence was discarded.

**6. Counting the counts of individual alleles of each polymorphic site:** For each sample, the number of sequenced sequences of each allele of each polymorphic site was counted, that is, the counts of individual alleles of each polymorphic site were counted.

**Example 4. Estimation of the number of alleles detected to be higher than the noise threshold in a polymorphic site**

A polymorphic site is elected, and the counts of individual alleles thereof are arranged in descending order, and marked as R1, R2, R3, ..., Rn or $R_1, R_2, R_3, ..., R_n$, and the total count for individual alleles thereof is the sum of the counts for individual alleles, marked as TC($TC = \sum_{i=1}^{n} R_i$).

Assuming that the noise threshold of the sample is $\alpha$, for a polymorphic site, if the count of an allele is less than $TC \times \alpha$, the allele count is marked as noise, and the number of alleles that are not marked as noise for the polymorphic site is the number of alleles that are higher than the noise threshold at that site. For example, the counts of four alleles at the polymorphic site are 27, 3552, 5809 and 11, respectively, then TC=27+3552+5809+11=9399, R1=5809, R2=3552, R3=27 and R4=11. If the noise threshold $\alpha$=0.01 is set, then the cut-off threshold (Th)=TC$\times\alpha$=93.99. Since both R1 and R2 are greater than 93.99 and both R3 and R4 are less than 93.99, the alleles above the noise threshold at this site are R1 and R2, and the number of alleles above the noise threshold at this site is 2.

Preferably, after the counts of individual alleles of a polymorphic site are arranged in descending order and marked as R1, R2, ..., Rn, the number of alleles detected to be higher than the noise threshold for the polymorphic site is estimated according to the following steps:

(1) setting the noise threshold of sequencing to be $\alpha$;

(2) calculating $C_i = R_i / \sum_{j=1}^{i} R_j$

(3) if $C_{i-1} \geq \alpha$ and $C_i < \alpha$, it is estimated that there are i-1 alleles at the polymorphic site.

For example, for a polymorphic site, if i=3, $C_2$=R2/(R1+R2)$\geq\alpha$ and $C_3$=R3/(R1+R2+R3)$<\alpha$, then it is estimated that the site has i-1 = 2 alleles detected to be higher than the noise threshold. For example, the counts of four alleles at the polymorphic site are 27, 3552, 5809 and 11, respectively, then TC=27+3552+5809+11=9399, R1=5809, R2=3552, R3=27 and R4=11. If the noise threshold $\alpha$=0.01 is set, the cut-off threshold $\alpha$=0.01. Since $C_1$=R1/R1=1.0, $C_2$=R2/(R1+R2)=0.38,$C_3$=R3/(R1+R2+R3)=0.003 and $C_4$=R4/(R1+R2+R3+R4)=0.001, $C_2$ is greater than or equal to 0.01 and $C_3$ is less than 0.01, the alleles above the noise threshold at this site are R1 and R2, and the number of alleles above the noise threshold at this site is 2.

**Example 5. Estimation of the total count (TC) of individual alleles in a polymorphic site**

The total count (TC) of individual alleles in a polymorphic site can be calculated by any of the following methods:

(1) for a polymorphic site, the counts of individual alleles are summed to obtain the total count of individual alleles in the polymorphic site;
(2) for a polymorphic site, firstly the number of alleles detected to be higher than the noise threshold is calculated according to the method described in Example 4, then the total count of individual alleles in this polymorphic site is the sum of individual allele counts higher than the noise threshold;
(3) according to the characteristics of the sample, considering that the polymorphic site in the sample may have at most several alleles (set as k), the total count of individual alleles in the polymorphic site is the sum of the maximal k allele counts thereof, i.e. $TC = \sum_{i=1}^{k} R_i$.

**Example 6. Estimation of the possible genotype of a polymorphic site in a plasma cfDNA sample using allele counts**

For a pregnant woman (biological pregnant woman) who is the biological mother of the fetus, the genotype of each polymorphic site on chromosomes where both the mother and the fetus are of normal disomy karyotypes in a plasma cfDNA, can only be one of the five genotypes (not considering cases where the mother and/or fetus are chimera and/or the fetus does not inherit the mother's genotype for various reasons). For each polymorphic site, firstly the number of alleles detected to be higher than the noise threshold is calculated according to the method described in Example 4, and then the possible genotype of the polymorphic site can be estimated according to the following steps:

(1) setting the noise threshold of sequencing to be $\alpha$;
(2) determining the number of alleles higher than the noise threshold, and if the determination result is 1, then performing the following step (3); if the determination result is 2, then performing the following step (4); if the determination result is greater than 2, then performing the following step (8);
(3) estimating the genotype of the polymorphic site as AA|AA, and then performing the following step (11);
(4) determining the value of R1/(R1+R2), and if the determination result is less than 0.5+$\alpha$, then performing the following step (5); if the determination result is greater than or equal to 0.5+$\alpha$ and less than 0.75, then performing the following step (6); if the determination result is greater than or equal to 0.75, then performing the following step (7);
(5) estimating the genotype of the polymorphic site as AB|AB, and then performing the following step (11);
(6) estimating the genotype of the polymorphic site as ABIAA, and then performing the following step (11);
(7) estimating the genotype of the polymorphic site as AA|AB, and then performing the following step (11);
(8) determining the value of R2/R1, and if the determination result is less than 0.5, then performing the following step (9); if the determination result is greater than or equal to 0.5, then performing the following step (10);
(9) marking the polymorphic site as an outlier, and then either estimating the genotype of the polymorphic site as NA, and performing the following step (11); or performing the above step (4);
(10) estimating the genotype of the polymorphic site as AB|AC, and then performing the following step (11);
(11) outputting the estimated genotype of the polymorphic site.

**Example 7. Estimation of the count (FC) derived from fetal DNA of a polymorphic site in a plasma cfDNA sample of a biological pregnant woman**

A polymorphic site is selected, and the total count (TC) derived from the pregnant woman and fetal DNA is firstly estimated according to the method described in Example 5, and then the possible genotype of the polymorphic site is estimated according to the method described in Example 6, and the count (FC) derived from fetal DNA of the polymorphic site is estimated according to the following steps:

(1) if the genotype of the polymorphic site is AA|AA, then FC is estimated to be NA;
(2) if the genotype of the polymorphic site is AA|AB, then FC is estimated to be $2.0 \times R2$;
(3) if the genotype of the polymorphic site is AB|AA, then FC is estimated to be R1-R2;
(4) if the genotype of the polymorphic site is AB|AB, then FC is estimated to be NA;
(5) if the genotype of the polymorphic site is AB|AC, then FC is estimated to be R1-R2+R3 or $2.0 \times R3$;
(6) if the genotype of the polymorphic site is not any of the above genotypes, then FC is estimated to be NA.

**Example 8. Estimation of a concentration (f) of the least component DNA in a mixed sample**

Multiple polymorphic sites are selected, and then the concentration of the least component DNA in the sample is estimated according to the following steps:

(1) according to the method described in Example 5, estimating the total count (TC) derived from all sample DNA of each polymorphic site;
(2) according to the method described in Example 6 and Example 7, estimating the count (FC) derived from the least component DNA of each polymorphic site;
(3) according to the FC and TC counts of each polymorphic site, using uses linear regression or robust linear regression to calculate the concentration of the least component DNA in the sample, or using the mean or median of FC and TC to calculate the concentration of the least component DNA in the sample.

Figure 1 is a flowchart of estimating a concentration of fetal DNA in a plasma cfDNA sample of a pregnant woman as described in Example 8.

**Example 9. Estimation of expected counts of individual alleles in a polymorphic site according to a concentration f of the sample having the least component in a mixture of two samples**

For a plasma cfDNA sample of a biological pregnant woman, the two samples here refer to maternal cfDNA and fetal cfDNA, respectively, where the least component is the fetal cfDNA component and the most component is the maternal cfDNA component; for a mixture of two independent genome samples, the least component refers to the DNA component of the sample with a small proportion and the most component is the DNA component with a large proportion; for a plasma cfDNA sample of a pregnant woman who is legally permitted to accept egg donation, the least component is the fetal cfDNA component and the most component is the maternal cfDNA component.

A polymorphic site is selected, and the total count (TC) derived from the DNA of the two samples of such polymorphic site is firstly estimated according to the method described in Example 5. If the concentration of the least component is f, the concentration of the other sample having the most component is 1-f. For the genotype of any polymorphic site, the theoretical expected counts of individual alleles of the polymorphic site are estimated according to the following steps:

(1) for alleles derived from each chromosome position in the sample having the most component, marking the relative value thereof as 1-f;
(2) for alleles derived from each chromosome position in the sample having the least component, marking the relative value thereof as f;
(3) calculating the relative total value of each allele and the relative total value of all alleles in the polymorphic site;
(4) for each allele, calculating the ratio of the relative total value of such allele to the relative total value of all alleles, and then multiplying the ratio by TC to obtain the theoretical expected count of the allele.

For example, for a plasma DNA sample of a biological pregnant woman, assuming that the fetal DNA concentration is f, for any polymorphic site, the total count of individual alleles thereof is marked as TC. Then, for a polymorphic site of genotype AA|AA, there are two chromosomal positions derived from the sample having the most component (mother's DNA), which are A and A (marks before the vertical bar), respectably, and there are two chromosomal positions derived from the sample having the least component (fetal DNA), which areA and A (marks after the vertical bar), respectably. Then the relative total value of allele A is (1-f)+(1-f)+f+f=2, and the relative total value of all alleles is (1-f)+(1-f)+f+f=2; the ratio is 2/2=1, so the theoretical expected value of allele A is TC*1=TC. For genotype AB|AC, the relative total value of all alleles is (1-f)+(1-f)+f+f=2; the relative total value of allele A is (1-f)+f=1, and the ratio is 1/2, so its theoretical expected value is 1/2×TC=TC/2; the relative total value of allele B is 1-f, and the ratio is (1-f)/2, so its theoretical expected value is (1-f)/2×TC; the relative total value of allele C is f, and the ratio is f/2, so its theoretical expected value is f/2×TC. For a polymorphic site of genotype AB AAB on a chromosome whose karyotype is disomy-trisomy, the relative total value of all alleles is (1-f)+(1-f)+f+f+f=2+f; the relative total value of allele A is (1-f)+f+f=1+f, and the ratio is (1+f)/(2+f), so its theoretical expected value is (1+f)/(2+f)×TC; the relative total value of allele B is 1-f+f=1, and the ratio is 1/(2+f), so its theoretical expected value is 1/(2+f)×TC. Theoretical expected counts for other genotypes can be obtained in a similar way.

For example, for a plasma DNA sample of a pregnant woman who is legally permitted to accept egg donation, assuming that the fetal DNA concentration is f, for any polymorphic site, the total count of individual alleles thereof is marked as TC. For genotype AB|AC, the relative total value of all alleles is (1-f)+(1-f)+f+f=2; the relative total value of allele A is (1-f)+f=1, and the ratio is 1/2, so its theoretical expected value is 1/2×TC=TC/2; the relative total value of allele B is 1-f, and the ratio is (1-f)/2, so its theoretical expected value is (1-f)/2×TC; the relative total value of allele C is f, and the ratio is f/2, so its theoretical expected value is f/2×TC. For genotype AA|BC, the relative total value of all alleles is (1-f)+(1-f)+f+f=2; the relative total value of allele A is (1-f)+(1-f)=2-2f, and the ratio is (2-2f)/2=1-f, so its theoretical expected value is (1-f)×TC; the relative total value of allele B is f, and the ratio is f/2, so its theoretical expected value is f/2×TC; the relative total value of allele C is f, and the ratio is f/2, so its theoretical expected value is f/2×TC. Theoretical expected counts for other genotypes can be obtained in a similar way.

**Example 10. Preforming a goodness-of-fit test for allele counts at a polymorphic site**

A polymorphic site is selected, and a goodness-of-fit test is performed for possible genotypes of the site according to the following steps:

(1) calculating the counts of individual alleles of the polymorphic site, and marking them as observed counts $O_1$, $O_2$, ..., $O_m$ in descending order;
(2) calculating the expected counts of individual alleles thereof according to the method described in Example 9, and marking them as $E_1$, $E_2$, ..., $E_n$, respectively in descending order;
(3) using the observed counts and expected counts of individual alleles to perform the a goodness-of-fit test.

The goodness-of-fit test in the above step (3) can be, but not limited to, a goodness-of-fit test as performed by using Fisher's exact test, binomial distribution test, chi-square test or G test.

For example, for a certain genotype, if the observed count values of individual alleles are $O_1$, $O_2$ and $O_3$, respectively, and the expected count values are $E_1$, $E_2$ and $E_3$, respectively, then the goodness-of-fit of the G test can be calculated as :

$$G = 2 \times \sum \left[ O_i \times \ln\left(\frac{O_i}{E_i}\right) \right] = 2 \times \sum \left[ O_1 \times \ln\left(\frac{O_1}{E_1}\right) + O_2 \times \ln\left(\frac{O_2}{E_2}\right) + O_3 \times \ln\left(\frac{O_3}{E_3}\right) \right] ,$$

or

$$AIC = G - 2 \times df = 2 \times \sum \left[ O_1 \times \ln\left(\frac{O_1}{E_1}\right) + O_2 \times \ln\left(\frac{O_2}{E_2}\right) + O_3 \times \ln\left(\frac{O_3}{E_3}\right) \right] - 2 \times df$$

where df is the degree of freedom.

Preferably, if the number of the observed counts of alleles is less than the number of the expected counts of alleles, the missing observed count(s) of alleles is/are set to be a small value, such as 0.1; if the number of the expected counts of alleles is less than the number of the observed counts of alleles, the expected value(s) of the missing position(s) is/are set to be a small value or background noise value, such as 5 or TC×α.

For example, if the two allele counts of a polymorphic site are observed to be 4105 and 577 respectively, the fetal DNA concentration f=0.25, and the noise threshold is set to be α=0.01, then $O_1$=4105, $O_2$=577, TC=4105+577=4682. In order to determine which genotype has the best fit for individual allele counts of the polymorphic site, a goodness-of-fit test is performed for the observed individual allele counts against theoretical counts of individual alleles for all possible genotypes at the polymorphic site. Results of the goodness-of-fit test for genotypes AA|AA, AA|AB and AB|AC of the polymorphic site are illustrated as follows:

Genotype AA|AA: the degree of freedom df--1, the expected counts of alleles are $E_1=TC\times(1-\alpha)=4682\times(1-0.01)=4635.18$, $E_2=TC\times\alpha=46.82$, respectively; then G=1901.045, AIC=G-2×df=1899.045. Or the degree of freedom df=0, the expected counts of alleles are $E_1=TC=4682$, $E_2=0$ (rounded off), respectively; then G=0.0, AIC=G-2×df=0.0.

Genotype AA|AB: the degree of freedom df--1, the expected counts of alleles are $E_1=TC\times(2-f)/2=4682\times(2-0.25)/2=4096.75$, $E_2=TC\times f/2=4682\times0.25/2=585.25$, respectively; then G=0.1334, AIC= G-2×*df* =-1.8666.

Genotype AB|AC: the degree of freedom df=2, since three alleles are expected to be present while there are only two observed counts of alleles, $O_3$ is set to be a very small value, such as setting $O_3$=0.1, and the expected counts of alleles are $E_1=TC\times1/2=4682\times1/2=2341$, $E_2=TC\times(1-f)/2=4682\times(1-0.25)/2=1755.75$, $E_3=TC\times f/2=4682\times0.25/2=585.25$, respectively, then G=3325.046, AIC= G-2× *df* =3321.046.

In addition, the goodness-of-fit test all can also be performed with the same number of allele counts. Since there may be at most three alleles for this polymorphic site, the maximal three values are retained for both the observed counts of alleles and the expected counts of alleles, wherein the observed counts of alleles can be complemented with a small value, while the expected counts of alleles can be complemented with a threshold. For example, for the fitted genotype AA|AB for the above two observed allele values, set $O_3$=0.1, $E_3=TC\times\alpha$ =46.82, *df*=2, so $E_1=TC\times(1-\alpha)\times(2-f)/2=4055.783$, $E_2=TC\times(1-a)\times f/2=579.398$; G=94.24, AIC= G-2×df=90.24.

**Example 11. Estimation of the possible genotype of a polymorphic site by using a concentration f of sample having the least component and allele counts of the polymorphic site in a mixture of samples**

For a polymorphic site, the genotype of the polymorphic site is estimated according to the following steps:

(1) preforming goodness-of-fit tests with observed counts of individual alleles against theoretical counts of individual alleles of each possible genotype, respectively, according to the method described in Example 10;
(2) selecting the genotype with the best goodness-of-fit test for the observed counts of alleles to be marked as the genotype of the polymorphic site.

**Example 12. Estimation of the count (FC) derived from the sample having the least component in a polymorphic site by using a concentration f of the sample having the least component and individual counts of alleles of the polymorphic site and the genotypes thereof in a mixture of samples**

In a mixture of two independent samples, the concentration of the sample having the least component is f, the concentration of the sample having the most component is 1-f, the individual counts of alleles are marked as R1, R2, R3 and R4, respectively, in descending order, and then the count (FC) derived from the least component of the polymorphic site is estimated according to the following steps:

(1) if the genotype of the polymorphic site is AA|AA, then FC is estimated to be NA;
(2) if the genotype of the polymorphic site is AA|AB, then FC is estimated to be 2.0×R2;
(3) if the genotype of the polymorphic site is AB|AA, then FC is estimated to be R1-R2;
(4) if the genotype of the polymorphic site is AB|AB, then FC is estimated to be NA;
(5) if the genotype of the polymorphic site is AB|AC, then FC is estimated to be R1-R2+R3 or 2.0×R3;
(6) if the genotype of the polymorphic site is AA|BB, then FC is estimated to be R2;
(7) if the genotype of the polymorphic site is AA|BC, then FC is estimated to be R2+R3 or 2.0×R2 or 2.0×R3;
(8) if the genotype of the polymorphic site is AB|CC, then determining whether f>1/3, if the determination result is yes, then FC is estimated to be R1; if the determination result is no, then FC is estimated to be R3 ;
(9) if the genotype of the polymorphic site is ABICD, then FC is estimated to be R3+R4 or 2.0×R3 or 2.0×R4;
(10) if the genotype of the polymorphic site is not any of the above genotypes, then FC is estimated to be NA.

**Example 13. Estimation of a concentration of the sample having the least component in a mixture of two samples by using allele counts at polymorphic sites**

Multiple polymorphic sites are selected, and the concentration of the sample having the least component in a mixture of two independent samples is estimated according to the following steps:

(1) setting a background noise value $\alpha$, a concentration precision value $\varepsilon$ and an initial concentration value $f_0$;
(2) estimating the genotype of each polymorphic site according to the method described in Example 11;
(3) estimating the total count (FC) derived from the sample DNA having the least component of each polymorphic site in the mixture, according to the method described in Example 7 or Example 12;

(4) estimating the total count (TC) derived from two samples of each polymorphic site according to the method described in Example 5;

(5) according to the FC and TC counts of individual polymorphic site, estimating the concentration of the least component DNA in the mixed sample according to the method described in Example 8;

(6) determining whether $|f-f_0|$ is less than $\varepsilon$, and if the determination result is yes, then the concentration of the least component in the mixture is f; if the determination result is no, then setting $f_0=f$, and then performing step (2).

For a plasma DNA sample from a pregnant woman who is legally permitted to accept egg donation, the least component is fetal DNA, and the most component is maternal DNA. Since the fetus does not inherit the genetic material on the chromosomes of the pregnant woman who is legally permitted to accept egg donation, each polymorphic site in the plasma DNA of the pregnant woman who is legally permitted to accept egg donation may be one of the nine genotypes (not considering cases where the mother and/or fetus have chromosomal aneuploidy or copy number variation of chromosomal fragments and/or the mother and/or fetus are chimera genotypes and/or the fetus has other genotypes corresponding to non-diploid karyotypes for various reasons), wherein the concentration of fetal DNA can be estimated by iteration according to the steps as described above.

For a plasma DNA sample of a biological pregnant woman, the least component is fetal DNA and the most component is maternal DNA. Since the fetus inherits the genetic material on the chromosome of the biological mother, each polymorphic site in the plasma DNA of the biological pregnant woman may be one of the five genotypes (not considering cases where the mother and/or fetus have chromosomal aneuploidy or copy number variation of chromosomal fragments and/or the mother and/or fetus are chimera genotypes and/or the fetus does not inherit the mother's genotype for various reasons), wherein the concentration of fetal DNA can be estimated by iteration according to the steps as described above.

Figure 2 is a flowchart for estimating a fetal DNA concentration in a plasma DNA sample from a pregnant woman legally permitted to accept egg donation as described in Example 13.

**Example 14. Estimation of a fetal DNA concentration using simulated sequencing of polymorphic sites in a plasma DNA sample of a pregnant woman**

The method and steps for estimating the concentration of fetal DNA in the sample by using the relative ratio method of allele counts are briefly illustrated below by taking the counts of individual alleles of five hypothetical polymorphic sites in simulated plasma cfDNA of a pregnant woman as an example.

**(1) Simulation of the sequencing results of multiple polymorphic sites on the reference genome**

Polymorphic sites on the reference genome are selected and marked as Id001-Id005. Assume that the results of allele counts of the five polymorphic sites simulated according to Example 3 are shown in Table 1. In the hypothetical plasma cfDNA of a pregnant woman, the reference genome is considered to be a chromosomal region where both the mother and the fetus have a normal disomy karyotype, so each polymorphic site theoretically contains at most 3 alleles. Here counts for up to five alleles are shown for each site (some of these allele counts represent systematic noise during sample processing, sequencing, etc.). It should be understood that each polymorphic site may be detected to contain multiple alleles, and count statistics should be performed for each allele.

Table 1: Counts of individual alleles for five hypothetical polymorphic sites

| Site No. | Allele count | | | | |
|---|---|---|---|---|---|
| | 1 | 2 | 3 | 4 | 5 |
| Id001 | 35 | 14127 | | | |
| Id002 | 4105 | 577 | 13 | 7 | 9 |
| Id003 | 54 | 3101 | 3148 | 23 | |
| Id004 | 3552 | 5809 | 27 | 11 | 17 |
| Id005 | 3028 | 1011 | 4007 | 6 | 6 |

**(2) estimating the count derived from fetal DNA in each polymorphic site count according to the method described in Example 6 and Example 7**

Since each polymorphic site in the plasma cfDNA of the pregnant woman can only theoretically have up to three alleles, the allele counts of individual polymorphic sites are sorted in descending order and the maximal three numbers are marked as R1, R2 and R3, respectively. The results are shown in Table 2.

Table 2: Counts of individual alleles for five hypothetical polymorphic sites as ranked.

| Site No. | R1 | R2 | R3 |
|---|---|---|---|
| Id001 | 14127 | 35 | 0 |
| Id002 | 4105 | 577 | 13 |
| Id003 | 3148 | 3101 | 54 |
| Id004 | 5809 | 3552 | 27 |
| Id005 | 4007 | 3028 | 1011 |

The noise threshold for sequencing is set to be $\alpha$=0.01. The amplification count (FC) theoretically derived from fetal DNA and the total count (TC) theoretically derived from mother and fetal DNA in each polymorphic site is calculated.

For site Id001, R2/(R1+R2)=35/(14127+35)=0.002<0.01, the number of alleles is estimated to be one, the genotype is estimated to be AA|AA, FC=NA, TC=R1=14127.

For site Id002, R2/(R1+R2)=577/(4105+577)=0.123≥0.01, R3/(R1+R2+R3)=13/(4105+577+13)=0.003<0.01, the number of alleles is estimated to be two. Since R1/(R1+R2)=0.877≥0.75, the genotype is estimated to be AA|AB, FC=2×R2=1154, TC=R1+R2=4682.

For site Id003, R2/(R1+R2)=0.496>0.01, R3/(R1+R2+R3)=0.009<0.01, the number of alleles is estimated to be two, and since R1/(R1+R2)=0.504<0.5+$\alpha$, the genotype is estimated to be AB|AB, FC=NA, TC=R1+R2=6249.

For site Id004, R2/(R1+R2)=0.379≥0.01, R3/(R1+R2+R3)=0.003<0.01, the number of alleles is estimated to be two, and since 0.5+$\alpha$≤R1/(R1+ R2)=0.621<0.75, the genotype is estimated to be AB|AA, FC=R1-R2=2257, TC=R1+R2=9361.

For site Id005, R2/(R1+R2)=0.430≥0.01, R3/(R1+R2+R3)=0.126≥0.01, the number of alleles is estimated to be two, and since R2/R1=0.756≥0.5, the genotype is estimated to be AB|AC, FC=R1-R2+R3=1990, TC=R1+R2+R3=8046.

**(3) estimating the concentration of fetal DNA**

R software and linear regression or robust linear regression are used to calculate the concentration of fetal DNA in the sample, or the mean or median of FC and TC is used to calculate the concentration of fetal DNA in the sample. The results are shown in Table 3.

(a) Enter the values of FC and TC

$$FC=c(NA,1154,NA,2257,1990)$$

$$TC=c(14127,4682,6249,9361,8046)$$

(b) Calculate the concentration of fetal DNA using linear regression

$$lmfit=lm(FC\sim TC+0)$$

$$f=lmfit\$coefficients["TC"]$$

(c) Calculate the concentration of fetal DNA using robust regression library(MASS)

$$rlmfit=rlm(FC\sim TC+0,maxit=1000)$$

$$f=rlmfit\$coefficients["TC"]$$

(d) Calculate the concentration of fetal DNA in the sample using the mean or median of FC and TC

$$(d1)f=median(FC/TC,na.rm = T)$$

$$(d2)f=median(FC[c(2,4,5)])/median(TC[c(2,4,5)])$$

$$(d3)f=mean(FC/TC,na.rm = T)$$

$$(d4)f=mean(FC[c(2,4,5)])/mean(TC[c(2,4,5)])$$

(e) See Table 3 for the calculation results of the fetal DNA concentration.

Table 3: Estimation of a fetal DNA concentration in a sample by different methods.

| Estimation method | Estimated fetal DNA concentration |
|---|---|
| Linear regression (b) | 0.2441 |
| Robust regression (c) | 0.2441 |
| Median of ratios (d1) | 0.2465 |
| Mean of ratios (d3) | 0.2450 |
| Ratio of medians (d2) | 0.2473 |
| Ratio of means (d4) | 0.2445 |

**Example 15. Estimation of a fetal DNA concentration using simulated sequencing of polymorphic sites in a plasma cfDNA sample of a pregnant woman legally permitted to accept egg donation**

Taking simulated allele counts of 9 hypothetical polymorphic sites in a plasma cfDNA of a pregnant woman legally permitted to accept egg donation as an example, the following is a brief description of the process and steps of the estimation of the fetal DNA concentration in the sample by using the iterative fitting genotype method of allele counts.

**(1) Simulating the sequencing results of counts of individual alleles of multiple polymorphic sites on the reference genome of the plasma cfDNA sample of the pregnant woman legally permitted to accept egg donation**

The polymorphic sites on the reference genome are selected and marked as Id001-Id009, respectively. Assume that the results of allele counts of the 9 polymorphic sites simulated according to Example 3 are shown in Table 4. In the hypothetical plasma cfDNA of the pregnant woman legally permitted to accept egg donation, the reference genome is considered to be a chromosomal region where both the mother and the fetus have a normal disomy karyotype, so each polymorphic site theoretically contains at most 4 alleles. Here counts for up to five alleles are shown for each site. It should be understood that each polymorphic site may be detected to contain multiple alleles, and count statistics should be performed for each allele.

Table 4: Allele counts for hypothetical nine polymorphic sites.

| Site No. | Allele count | | | | |
|---|---|---|---|---|---|
| | 1 | 2 | 3 | 4 | 5 |
| Id001 | 35 | 14127 | | | |
| Id002 | 4105 | 577 | 13 | 7 | 9 |
| Id003 | 54 | 3101 | 3148 | 23 | |
| Id004 | 11 | 5809 | 27 | 3552 | 17 |
| Id005 | 3028 | 1011 | 4007 | 6 | 6 |
| Id006 | 36 | 936 | 3322 | 28 | 16 |
| Id007 | 5422 | 52 | 974 | 938 | 27 |
| Id008 | 1498 | 4835 | 1537 | 4711 | 38 |
| Id009 | 36 | 3412 | 2237 | 3493 | 23 |

**(2) Iteratively estimating the concentration of fetal DNA according to the method described in Example 13**

Since each polymorphic site can only theoretically have up to four alleles in the plasma of the pregnant woman legally permitted to accept egg donation, the allele counts of individual polymorphic sites are sorted in descending order and the maximal four numbers are marked as R1, R2, R3 and R4, respectively; then the noise threshold of sequencing is set to be $\alpha$=0.01, the iteration precision value $\varepsilon$=0.001, and the initial estimated value of fetal concentration $f_0$=0.10; finally the concentration of fetal DNA is calculated as follows.

**Step (a)** estimates the genotype of the site and the amplification count (FC) theoretically derived from fetal DNA and the total count (TC) theoretically derived from mother and fetal DNA for each polymorphic site according to counts of individual alleles and $f_0$, by following the method described in Example 11 and Example 12.

For example, for site number Id0006, R1 to R4 are 3322, 936, 36 and 28 respectively, then Oi=3322, $O_2$=936, $O_3$=36 and $O_4$=28. Since R2/(R1+R2)$\geq$0.01 and R3/(R1+R2+R3)<0.01, the number of alleles detected to be higher than the noise threshold at this site is two.

The goodness-of-fit test for all possible genotypes at this site is performed as follows:

**AA|AA**:TC=R1+R2=4258,              $E_1$=(1-$\alpha$)$\times$TC=4215.42,              $E_2$=$\alpha$$\times$TC=42.58,

$$G_{AA|AA}=2 \times \left[ O_1 \times \ln\left(\frac{O_1}{E_1}\right) + O_2 \times \ln\left(\frac{O_2}{E_2}\right) \right] =4202.43$$

**AA|AB**:TC=R1+R2=4258,              $E_1$=(1-$f_0$/2)$\times$TC=4045.10,              $E_2$=$f_0$/2$\times$TC=212.90,

$$G_{AA|AB} = 2 \times \left[ O_1 \times \ln\left(\frac{O_1}{E_1}\right) + O_2 \times \ln\left(\frac{O_2}{E_2}\right) \right]=1463.58$$

**AB|AA**:TC=R1+R2=4258,              $E_1$=(1+$f_0$)/2$\times$TC=2341.90,              $E_2$=(1-$f_0$)/2$\times$TC=1916.10,

$$G_{AB|AA}=2 \times \left[ O_1 \times \ln\left(\frac{O_1}{E_1}\right) + O_2 \times \ln\left(\frac{O_2}{E_2}\right) \right]=981.61$$

**AB|AB**:TC=R1+R2=4258,              $E_1$=1/2$\times$TC=2129.00,              $E_2$=1/2$\times$TC=2129.00,

$$G_{AB|AB}= 2 \times \left[ O_1 \times \ln\left(\frac{O_1}{E_1}\right) + O_2 \times \ln\left(\frac{O_2}{E_2}\right) \right]=1417.62$$

**AB|AC**:TC=R1+R2+R3=4294,    $E_1$=1/2$\times$TC=2147.00,    $E_2$=(1-$f_0$)/2$\times$TC=1932.30,    $E_3$=$f_0$/2$\times$TC=214.70,

$$G_{AB|AC}= 2 \times \left[ O_1 \times \ln\left(\frac{O_1}{E_1}\right) + O_2 \times \ln\left(\frac{O_2}{E_2}\right) + O_3 \times \ln\left(\frac{O_3}{E_3}\right) \right]=1414.58$$

**AA|BB**:TC=R1+R2=4258,              $E_1$=(1-$f_0$)$\times$TC=3832.20,              $E_2$=$f_0$$\times$TC=425.80,

$$G_{AA|BB}= 2 \times \left[ O_1 \times \ln\left(\frac{O_1}{E_1}\right) + O_2 \times \ln\left(\frac{O_2}{E_2}\right) \right]=525.23$$

**AA|BC**:TC=R1+R2+R3=4294,    $E_1$=(1-$f_0$)$\times$TC=3864.60,    $E_2$=$f_0$/2$\times$TC=214.70,    $E_3$=$f_0$/2$\times$TC=214.70,

$$G_{AA|BC} \quad = 2 \times \left[ O_1 \times \ln\left(\frac{O_1}{E_1}\right) + O_2 \times \ln\left(\frac{O_2}{E_2}\right) + O_3 \times \ln\left(\frac{O_3}{E_3}\right)\right] = 1622.53$$

**AB|CC**:TC=R1+R2+R3=4294, $E_1$=(1-$f_0$)/2×TC=1932.30, $E_2$=(1-$f_0$)/2×TC=1932.30, $E_3$=$f_0$×TC=429.40,

$$G_{AB|CC} = 2 \times \left[ O_1 \times \ln\left(\frac{O_1}{E_1}\right) + O_2 \times \ln\left(\frac{O_2}{E_2}\right) + O_3 \times \ln\left(\frac{O_3}{E_3}\right)\right] = 2064.69$$

**AB|CD**:TC=R1+R2+R3+R4=4322, $E_1$=(1-$f_0$)/2×TC=1944.90, $E_2$=(1-$f_0$)/2×TC=1944.90, $E_3$=$f_0$/2×TC=216.10, $E_4$=$f_0$/2×TC=216.10, $G_{AB|CD}$=1944.34。

Since $G_{AA|BB}$<$G_{AB|AA}$<$G_{AB|AC}$<$G_{AB|AB}$<$G_{AA|AB}$<$G_{AA|BC}$<$G_{AB|CD}$<$G_{AB|CC}$<$G_{AA|AA}$, the genotype of site Id006 is estimated to be AA|BB. Then, FC=R2=936 and TC=R1+R2=4258 are estimated according to the method described in Example 12.

According to the same rules, FC and TC values are estimated for the above nine sites, respectively.

**Step (b)** uses the FC and TC values of each polymorphic site to estimate the fetal DNA concentration f according to the method described in Example 8.

**Step (c)** determines whether the absolute value of f-$f_0$ is less than $\varepsilon$, and if the determination result is yes, then outputs that the fetal DNA concentration is f, and ends the calculation; if the determination result is no, then sets $f_0$=f, and then performs the above step (a).

The results of the iterative execution of the above example are shown in Table 5 below.

Table 5: Iteration parameter estimates for the fetal DNA concentration.

| Iteration no. | Initial $f_0$ | Recalculated f | $|f-f_0|$ |
|---|---|---|---|
| 1 | 0.1 | 0.2385 | 0.1385 |
| 2 | 0.2385 | 0.2436 | 0.0051 |
| 3 | 0.2436 | 0.2436 | 0 |

The concentration of the fetal DNA in this example is therefore estimated to be f=0.2436.

**Example 16. Estimation of the genotype of a site to be analyzed by using a concentration of fetal DNA and allele counts of the site in a plasma DNA sample of a pregnant woman**

A set of polymorphic sites in the reference group and two polymorphic sites in the target group in the plasma cfDNA sample of the pregnant woman are simulated as described in Example 3. Assume that a set of polymorphic sites in the reference group are used to estimate the concentration of fetal DNA according to the method described in Example 14 to f=0.20, and the counts of individual alleles of the two polymorphic sites in the target group are A: 16994, 1896, 23; B: 9146, 7355, 1892, 58, respectively. If both the mother and fetus have normal disomy on the chromosome where site A and site B are located and there is no large fragment insertion or deletion variation affecting site A and site B, both site A and site B can only be one of the following five genotypes, namely AA|AA, AA|AB, AB|AA, AB|AB, and AB|AC. Taking the above-mentioned results of allele counts for site A and site B as an example, their most probable genotypes are respectively estimated according to the method described in Example 11.

All possible genotypes of site A and site B are tested for a goodness-of-fit by using the G test, and the results are shown in Table 6 below.

Table 6: Estimating genotypes of target loci using a goodness-of-fit test.

| Gene site | Possible genotype | Actual allele count | | | Expected allele count | | | G test | AIC value | Estimation result |
|---|---|---|---|---|---|---|---|---|---|---|
| | | $O_1$ | $O_2$ | $O_3$ | $E_1$ | $E_2$ | $E_3$ | | | |
| Site A | AA\|AA | 16994 | 1896 | | 18701.1 | 188.9 | | 5492.03 | 5490.03 | |
| | AA\|AB | 16994 | 1896 | | 17001 | 1889 | | 0.03 | -1.97 | * |
| | AB\|AA | 16994 | 1896 | | 11334 | 7556 | | 8524.15 | 8522.15 | |
| | AB\|AB | 16994 | 1896 | | 9445 | 9445 | | 13874.73 | 13872.73 | |
| | AB\|AC | 16994 | 1896 | 23 | 9456.5 | 7565.2 | 1891.3 | 14472.08 | 14470.08 | |
| Site B | AA\|AA | 9146 | 7355 | 1892 | 18025.14 | 183.93 | 183.93 | 50668.67 | 50666.67 | |
| | AA\|AB | 9146 | 7355 | 1892 | 16388.16 | 1820.91 | 183.93 | 18687.03 | 18685.03 | |
| | AB\|AA | 9146 | 7355 | 1892 | 10925.44 | 7283.63 | 183.93 | 5711.41 | 5709.41 | |
| | AB\|AB | 9146 | 7355 | 1892 | 9104.54 | 9104.54 | 183.93 | 5764 | 5762 | |
| | AB\|AC | 9146 | 7355 | 1892 | 9196.5 | 7357.2 | 1839.3 | 1.77 | -2.23 | * |

From the results in Table 6, it can be seen that site A has the best goodness-of-fit test result for genotype AA|AB and site B has the best goodness-of-fit test result for genotype AB|AC, so it is estimated that the genotype of site A is AA|AB and the genotype of site B is AB|AC.

**Example 17. Estimation of the karyotype of the target to be detected by using the concentration f of the sample having the least component in a sample mixture and the allele counts of a set of polymorphic sites in the target region**

Using multiple polymorphic sites in the target region and a comprehensive goodness-of-fit test to estimate the karyotype of the target chromosomal or sub-chromosomal fragment to be detected, the main steps are as follows:

(1) analyzing the sample to be tested, and listing all possible karyotypes of the sample in the target region;
(2) for each possible karyotype, listing all possible genotypes corresponding to the karyotype at each polymorphic site in the target region;
(3) for each polymorphic site in the target region, selecting a genotype with the best fit for each karyotype according to the method described in Example 11;
(4) Comprehensively analyzing the goodness-of-fit test results of all polymorphic sites in the target region for all karyotypes, and rendering the karyotype with the best comprehensive fit of polymorphic sites as the karyotype of the target (chromosomal or sub-chromosomal fragment) to be detected.

**Example 18. Estimation of the aneuploidy variation at the chromosome level or deletion or duplication variations at the sub-chromosomal level in the region to be analyzed by using the fetal DNA concentration f and the allele counts of a set of polymorphic sites in the chromosomal or sub-chromosomal region to be analyzed in a plasma DNA sample of a pregnant woman**

Two pregnant women's plasma cfDNA samples are simulated as described in Example 3, wherein a set of polymorphic sites in the reference group and a set of polymorphic sites in the target region derived from a specific chromosomal or sub-chromosomal fragment are simulated for each sample. Assume that a set of polymorphic sites in the reference genome are used to estimate the concentrations of fetal DNA in two samples according to the method described in Example 14 to be both f=0.20, and the counts of individual alleles for a set of polymorphic sites in the target region in sample 1 and sample 2 are listed in Table 7 below.

Table 7: Allele counts for a set of polymorphic sites on the target chromosome to be tested in two hypothetical samples.

| Sample No. | Site No. | Allele count | | | |
|---|---|---|---|---|---|
| | | 1 | 2 | 3 | 4 |
| Sample 1 | Id001 | 9565 | 14 | 4 | 0 |
| | Id002 | 5820 | 652 | 6 | 3 |
| | Id003 | 6718 | 4465 | 12 | 5 |
| | Id004 | 7838 | 7656 | 34 | 12 |
| | Id005 | 9465 | 7552 | 1898 | 33 |
| Sample 2 | Id001 | 7021 | 1574 | 7 | 3 |
| | Id002 | 10588 | 1185 | 1164 | 23 |
| | Id003 | 3408 | 2861 | 23 | 12 |
| | Id004 | 9059 | 6012 | 1505 | 34 |
| | Id005 | 9386 | 9373 | 1899 | 18 |

Assume that a set of polymorphic sites in the target region in sample 1 and sample 2 are derived from chromosome 21, and our goal is to detect whether the fetuses in sample 1 and sample 2 are trisomy 21, that is, whether the karyotype for chromosome 21 in these two samples is disomy-disomy (both mother and fetus have normal disomy for chromosome 21) or disomy-trisomy (a pregnant woman with normal disomy chromosome 21 is pregnant with a fetus with trisomy chromosome 21). For disomy-disomy, all polymorphic sites can only be one of the following 5 genotypes, namely AA|AA, AA|AB, AB|AA, AB|AB or AB|AC. For disomy-trisomy, all polymorphic sites can only be one of the following 10 genotypes, namely AA|AAA, AA|AAB, AAIABB, AAIABC, ABIAAA, AB AAB, ABIAAC, ABIABC, ABIACC or ABIACD. For a set of polymorphic sites per target region of chromosome 21 in sample 1 and sample 2, a goodness-of-fit test is performed by using the G test according to the disomy-disomy and disomy-trisomy karyotype, respectively, according to the method described in Example 17, and the results are shown in Table 8 below.

Table 8: the goodness-of-fit test results for individual allele counts at polymorphic sites in the target region according to the karyotype.

| Sample No. | Site No. | The goodness-of-fit test result with the best fit | | | | | | | |
| | | Fitting karyotype of disomy-disomy | | | | Fitting karyotype of disomy-trisomy | | | |
| | | Genotype | Total count | G value | AIC value | Genotype | Total count | G value | AIC value |
|---|---|---|---|---|---|---|---|---|---|
| Sample 1 | Id001 | AA\|AA | 9565 | 0 | 0 | AA\|AAA | 9565 | 0 | 0 |
| | Id002 | AA\|AB | 6472 | 0.039 | -1.961 | AA\|AAB | 6472 | 7.338 | 5.338 |
| | Id003 | AB\|AA | 11183 | 0.025 | -1.975 | AB\|AAA | 11183 | 60.564 | 58.564 |
| | Id004 | AB\|AB | 15494 | 2.138 | 0.138 | AB\|AAB | 15494 | 97.537 | 95.537 |
| | Id005 | AB\|AC | 18915 | 0.054 | -3.946 | AB\|AAC | 18915 | 154.291 | 150.291 |
| Sample 2 | Id001 | AA\|AB | 8595 | 543.745 | 541.745 | AA\|ABB | 8595 | 0.099 | -1.901 |
| | Id002 | AA\|AB | 12937 | 3131.201 | 3129.201 | AA\|ABC | 12937 | 0.193 | -3.807 |
| | Id003 | AB\|AB | 6269 | 47.789 | 45.789 | AB\|AAB | 6269 | 0.084 | -1.916 |
| | Id004 | AB\|AC | 16576 | 143.656 | 139.656 | AB\|AAC | 16576 | 0.077 | -3.923 |
| | Id005 | AB\|AC | 20658 | 245.48 | 241.48 | AB\|ABC | 20658 | 0.266 | -3.734 |

For sample 1, the individual allele counts for most polymorphic sites have a better fit to the genotypes in disomy-disomy than to genotypes in disomy-trisomy, so the karyotype of sample 1 is estimated to be disomy-disomy, that is, both mother and fetus are normal disomy.

For sample 2, the individual allele counts for all polymorphic sites have a better fit to the genotypes in trisomy-disomy than to the genotypes in disomy-disomy, so the karyotype of sample 2 is estimated to be a disomy-trisomy, that is, the mother has a normal disomy and the fetus has an abnormal trisomy 21.

When comprehensively considering the fitting results of multiple polymorphic sites, the karyotype with the best fit for most samples can be considered, or the G value, AIC value, modified G value and/or modified AIC value can be used for determination.

For example, if the disomy-disomy karyotype for sample 1 is fitted, then:

the integrated G value is $\Sigma G_i=0.0+0.039+0.025+2.138+0.054=2.256$

the integrated AIC value is $\Sigma AIC_i=0.0+(-1.961)+(-1.975)+0.138+(-3.946)=-7.744$

the integrated AIC/total count value is $\Sigma(AIC_i/TC_i)=0.0/9565+(-1.961/6472)+(-1.975/11183)+0.138/15494+(-3.946/18915)=-0.00068$

the integrated AIC/total count/f value is $\Sigma(AIC_i/TC_i/f=0.0/9565/0.2+(-1.961/6472/0.2)+(-1.975/11183/0.2)+0.138/15494/0.2+(-3.946/18915/0.2)=-0.0034$.

If the disomy-trisomy karyotype for sample 1 is fitted, then:

the integrated G value is $\Sigma G_i=319.73$
the integrated AIC value is $\Sigma AIC_i=309.73$
the integrated AIC/total count is $\Sigma(AIC_i/TC_i)=0.02017$
the integrated AIC/total count/f is $\Sigma(AIC_i/TC_i/f)=0.10087$.

For sample 1, the fits of integrated G value, integrated AIC value, integrated AIC/total count value, and integrated AIC/total count/f value to the disomy-disomy genotype all are smaller than the corresponding fits to the disomy-trisomy genotype, thus these values or values derived from them can also be used to determine the fitting quality of each allele of multiple polymorphic sites to different karyotypes.

When detecting for micro-deletion or micro-duplication variations at the sub-chromosomal level, one should consider that the mother may carry homozygous or heterozygous micro-deletions or micro-duplications at the sub-chromosomal level, so for each polymorphic site as affected, all possible genotypes should be taken into account and detected using a goodness-of-fit test. For example, the detection of micro-deletion mutations at the sub-chromosomal level requires detection of all possible genotype combinations of mothers and fetuses under conditions where mothers have homozygous micro-deletions, heterozygous micro-deletions or are normal and the fetuses have homozygous micro-deletions, heterozygous micro-deletions or are normal. Correspondingly, if micro-duplication mutations at the sub-chromosomal level are to be detected, it is necessary to detect all possible genotype combinations of mothers and fetuses under conditions where mothers have homozygous micro-duplications, heterozygous micro-duplications or are normal and the fetuses have homozygous micro-duplications, heterozygous micro-duplications or are normal.

**Example 19. Estimation of concentrations of fetal DNA in plasma DNA samples of pregnant woman using the high-throughput sequencing results of a set of polymorphic sites in the samples**

According to the method described in Example 1, for each sample in the amplicon sequencing data set (Barrett, Xiong et al. 2017, PLoS One 12:e0186771) of cfDNA indel markers in pregnant women's plasma, each indel marker (polymorphic site) was counted, and then according to the method described in Example 8, for each polymorphic site in each sample, the count (FC) derived from fetal DNA and the total count (TC) derived from the pregnant woman and fetal DNA were estimated, and the concentration of fetal DNA in each sample was estimated by using the FC and TC of each polymorphic site in each sample.

Figure 3 shows the analysis results of a plasma cfDNA sample of a pregnant woman in this data set. The count (FC) derived from fetal DNA and the total count (TC) derived from the pregnant woman and fetal DNA for each indel polymorphic site in the sample are represented as a point in the graph. A robust regression fitting (fitting model: FC~TC+0) was performed by using the FC and TC values of each polymorphic site in the sample and the rlm function in the MASS library of the R software package and the concentration of fetal DNA was estimated. The result of the rlm robust regression fitting was the straight line in the figure, and the fetal DNA concentration was estimated as the slope (the model coefficient for TC) of this line.

**Example 20. Estimation of DNA concentrations of the least components in mixed DNA samples using the high-throughput sequencing results of a set of polymorphic sites in the samples**

According to the method described in Example 2, for each sample in the mixed sample amplicon sequencing data set (Kim, Kim et al. 2019, Nat Commun 10:1047), counts of individual alleles in each polymorphic site were counted, and then, according to the method described in Example 8, for each polymorphic site of each sample, the count (FC) derived from the least component DNA and the total count (TC) derived from all DNA were estimated, and the concentration of the least component DNA in each sample was estimated using the FC and TC of each polymorphic site in each sample.

Figure 4a shows the analysis result of a mixed DNA sample in this data set. The count (FC) derived from the least component DNA and the total count (TC) derived from all DNA for each polymorphic site in the sample are represented as a point in the graph. A rlm robust regression (model: FC~TC+0) was performed by using the FC and TC values of each polymorphic site and the concentration of the least component DNA in the sample was estimated. The result of the rlm robust regression was the straight line fitted in the figure, and the least component DNA concentration was estimated as the slope (the model coefficient for TC) of this line. Figure 4b shows the analysis result of all mixed DNA samples in this data set. Multiple replicates were performed for four mixed samples at the library preparation or sequencing level, the expected least component DNA concentrations were 0.01, 0.02, 0.10, or 0.20 (x-axis), respectively, and the estimated least component DNA concentrations of each sample were y-axis. The dotted line in the figure indicates the position of the straight line y=x.

**Example 21. Computer simulation of variations at chromosomal level, sub-chromosomal level and short sequence level in plasma DNA samples of pregnant women**

To detect genetic variations at the chromosomal, sub-chromosomal, or short-sequence level, we have simulated variations where the karyotypes were disomy-monosomy and disomy-trisomy at the chromosomal level, and simulated variations of nullisomy-nullisomy, nullisomy-monosomy, monosomy-nullisomy, monosomy-monosomy, monosomy-disomy, disomy-monosomy, disomy-disomy, disomy-trisomy, trisomy-disomy, trisomy-trisomy, trisomy-tetrasomy, tetrasomy-trisomy and tetrasomy-tetrasomy at the sub-chromosomal level, and simulated all possible genotypes of any polymorphic site under the normal disomy-disomy karyotype at the short sequence level. The specific simulation process of different polymorphic sites in each sample is briefly described as follows:

1. Simulation of plasma DNA samples of pregnant women containing chromosomal monosomy.

In order to detect chromosomal monosomy aneuploidy variations at the chromosomal level, we have simulated plasma DNA samples of pregnant women containing chromosomal monosomy, wherein three pairs of chromosomes, numbered as No.1 (Chr01), No. 2 (Chr02) and No. 3 (Chr03), respectively, are simulated for both the mother and fetus in each sample. 100 polymorphic sites are simulated according to the method described in Example 3 on chromosomes 1, 2 and 3 in each sample. A concentration randomly selected from the following concentrations (0.02, 0.05, 0.10, 0.15, 0.20, 0.25, 0.30, 0.35, 0.40, 0.45) is used as the simulated fetal DNA concentration for each sample.

The simulated chromosome 1 is the reference chromosome in the sample, wherein the genotype of each polymorphic site is simulated as one of the normal disomy-disomy genotypes, and the total count of the individual alleles of each polymorphic site is 200.

The simulated chromosome 2 is a disomy-disomy chromosome in the sample, wherein the genotype of each polymorphic site is simulated as one of the normal disomy-disomy genotypes, and the total count of the individual alleles of each polymorphic site is 200.

The simulated chromosome 3 is a disomy-monosomy chromosome in the sample, wherein the genotype of each polymorphic site is simulated as one of the disomy-monosomy genotypes. Due to the absence of one fetal chromosome, the total count of the individual alleles of each polymorphic site is 200-100f.

Using the simulated allelic sequence of each sample as the input file, the ART simulation software (Huang, Li et al. 2012, Bioinformatics 28:593-594) is used to simulate the high-throughput sequencing results, where the fold parameter of the ART simulation software is set as 50 or 100.

2. Simulation of plasma DNA samples of pregnant women containing chromosomal trisomy.

In order to detect chromosomal trisomy aneuploidy variations at the chromosomal level, we have simulated plasma DNA samples of pregnant women containing chromosomal trisomy, wherein three pairs of chromosomes, numbered as No.1 (Chr01), No. 2 (Chr02) and No. 3 (Chr03), respectively, are simulated for both the mother and fetus in each sample. 100 polymorphic sites are simulated according to the method described in Example 3 on chromosomes 1, 2 and 3 in each sample. A concentration randomly selected from the following concentrations (0.02, 0.05, 0.10, 0.15, 0.20, 0.25, 0.30, 0.35, 0.40, 0.45) is used as the simulated fetal DNA concentration for each sample.

The simulated chromosome 1 is the reference chromosome in the sample, wherein the genotype of each polymorphic site is simulated as one of the normal disomy-disomy genotypes, and the total count of the individual alleles of each polymorphic site is 200.

The simulated chromosome 2 is a disomy-disomy chromosome in the sample, wherein the genotype of each polymorphic site is simulated as one of the normal disomy-disomy genotypes, and the total count of the individual alleles of each polymorphic site is 200.

The simulated chromosome 3 is a disomy-trisomy chromosome in the sample, wherein the genotype of each polymorphic site is simulated as one of the disomy-trisomy genotypes. Due to the presence of one extra fetal chromosome, the total count of the individual alleles of each polymorphic site is 200+100f.

Using the simulated allelic sequence of each sample as the input file, the ART simulation software is used to simulate the high-throughput sequencing results, where the fold parameter of the ART simulation software is set as 50 or 100.

3. Simulation of plasma DNA samples of pregnant women containing sub-chromosomal micro-deletions.

In order to detect micro-deletion variations at the sub-chromosomal level, we have simulated plasma DNA samples of pregnant women containing chromosomal micro-deletions, wherein 7 pairs of chromosomes, numbered as No.1 (Chr01), No. 2 (Chr02), No. 3 (Chr03), No. 4 (Chr04), No. 5 (Chr05), No. 6 (Chr06) and No. 7 (Chr07), respectively, are simulated for both the mother and fetus in each sample. 100 polymorphic sites are simulated according to the method described in Example 3 on chromosomes 1 to 7 in each sample. A concentration randomly selected from the following concentrations (0.02, 0.05, 0.10, 0.15, 0.20, 0.25, 0.30, 0.35, 0.40, 0.45) is used as the simulated fetal DNA concentration for each sample. Here, each micro-deletion region is regarded as a whole chromosome, and the polymorphic site is selected from the micro-deletion region, in which in a single genome, a pair of chromosomes where one chromosome is normal and one chromosome contained a micro-deletion are marked as monosomy, while a pair of chromosomes where two chromosomes both contain a micro-deletion are marked as nullisomy.

The simulated chromosome 1 is the reference chromosome in the sample, wherein the genotype of each polymorphic site is simulated as one of the normal disomy-disomy genotypes, and the total count of the individual alleles of each polymorphic site is 200.

The simulated chromosome 2 is a disomy-disomy chromosome in the sample, wherein the genotype of each polymorphic site is simulated as one of the normal disomy-disomy genotypes, and the total count of the individual alleles of each polymorphic site is 200.

The simulated chromosome 3 is a disomy-monosomy chromosome in the sample, wherein the genotype of each polymorphic site is simulated as one of the disomy-monosomy genotypes. Since one fetal chromosome contains a micro-deletion, the total count of the individual alleles of each polymorphic site is 200-100f.

The simulated chromosome 4 is a monosomy-disomy chromosome in the sample, wherein the genotype of each polymorphic site is simulated as one of the monosomy-disomy genotypes. Since one fetal chromosome contains a micro-deletion, the total count of the individual alleles of each polymorphic site is 100+100f.

The simulated chromosome 5 is a monosomy-monosomy chromosome in the sample, wherein the genotype of each polymorphic site is simulated as one of the monosomy-monosomy genotypes. Since one maternal chromosome and one fetal chromosome both contain a micro-deletion, the total count of the individual alleles of each polymorphic site is 100.

The simulated chromosome 6 is a monosomy-nullisomy chromosome in the sample, wherein the genotype of each polymorphic site is simulated as one of the monosomy-nullisomy genotypes. Since one maternal chromosome and the pair of fetal chromosomes all contain a micro-deletion, the total count of the individual alleles of each polymorphic site is 100-100f.

The simulated chromosome 7 is a nullisomy-nullisomy chromosome in the sample, wherein the genotype of each polymorphic site is simulated as one of the nullisomy-nullisomy genotypes. Since the pair of maternal chromosomes and the pair of fetal chromosomes all contain a micro-deletion, the total count of the individual alleles of each polymorphic site is 0, that is, the simulation produces no specific amplification sequence or the simulation produces some random sequences which cannot be located to any chromosome.

Using the simulated allelic sequence of each sample as the input file, the ART simulation software is used to simulate the high-throughput sequencing results, where the fold parameter of the ART simulation software is set as 50 or 100.

4. Simulation of plasma DNA samples of pregnant women containing sub-chromosomal micro-duplications.

In order to detect micro-duplication variations at the sub-chromosomal level, we have simulated plasma DNA samples of pregnant women containing sub-chromosomal micro-duplications, wherein 7 pairs of chromosomes, numbered as No. 1 (Chr01), No. 2 (Chr02), No. 3 (Chr03), No. 4 (Chr04), No. 5 (Chr05), No. 6 (Chr06) and No. 7 (Chr07), respectively, are simulated for both the mother and fetus in each sample. 100 polymorphic sites are simulated according to the method described in Example 3 on chromosomes 1 to 7 in each sample. A concentration randomly selected from the following concentrations (0.02, 0.05, 0.10, 0.15, 0.20, 0.25, 0.30, 0.35, 0.40, 0.45) is used as the simulated fetal DNA concentration for each sample. Here, each micro-duplication region is regarded as a pair of chromosomes, and the polymorphic site is selected from the micro-duplication region, thus in a single genome, a pair of chromosomes where one chromosome is normal and one chromosome contains a micro-duplication are marked as trisomy, while a pair of chromosomes where two chromosomes both contain a micro-duplication are marked as tetrasomy.

The simulated chromosome 1 is the reference chromosome in the sample, wherein the genotype of each polymorphic site is simulated as one of the normal disomy-disomy genotypes, and the total count of the individual alleles of each polymorphic site is 200.

The simulated chromosome 2 is a disomy-disomy chromosome in the sample, wherein the genotype of each polymorphic site is simulated as one of the normal disomy-disomy genotypes, and the total count of the individual alleles of each polymorphic site is 200.

The simulated chromosome 3 is a disomy-trisomy chromosome in the sample, wherein the genotype of each polymorphic site is simulated as one of the disomy-trisomy genotypes. Since one fetal chromosome contains a micro-duplication, the total count of the individual alleles of each polymorphic site is 200+100f.

The simulated chromosome 4 is a trisomy-disomy chromosome in the sample, wherein the genotype of each polymorphic site is simulated as one of the trisomy-disomy genotypes. Since one maternal chromosome contains a micro-duplication, the total count of the individual alleles of each polymorphic site is 300-100f.

The simulated chromosome 5 is a trisomy-trisomy chromosome in the sample, wherein the genotype of each polymorphic site is simulated as one of the trisomy-trisomy genotypes. Since one maternal chromosome and one fetal chromosome both contain a micro-duplication, the total count of the individual alleles of each polymorphic site is 300.

The simulated chromosome 6 is a trisomy-tetrasomy chromosome in the sample, wherein the genotype of each polymorphic site is simulated as one of the trisomy-tetrasomy genotypes. Since one maternal chromosome and the pair of fetal chromosomes all contain a micro-duplication, the total count of the individual alleles of each polymorphic site is 300+100f.

The simulated chromosome 7 is a tetrasomy-tetrasomy chromosome in the sample, wherein the genotype of each polymorphic site is simulated as one of the tetrasomy-tetrasomy genotypes. Since the pair of maternal chromosomes and the pair of fetal chromosomes all contain a micro-duplication, the total count of the individual alleles of each polymorphic site is 400.

Using the simulated allelic sequence of each sample as the input file, the ART simulation software is used to simulate the high-throughput sequencing results, where the fold parameter of the ART simulation software is set as 50 or 100.

5. Simulation of plasma DNA samples of pregnant women containing variations at the short sequence level.

In order to detect variations at the short sequence level, we have simulated plasma DNA samples of pregnant women containing variation sites at the short sequence level, wherein 2 pairs of chromosomes, numbered as No.1 (Chr01), and No. 2 (Chr02), respectively, are simulated for both the mother and fetus in each sample. 100 polymorphic sites are simulated according to the method described in Example 3 on chromosome 1 and chromosome 2 in each sample. A concentration randomly selected from the following concentrations (0.02, 0.05, 0.10, 0.15, 0.20, 0.25, 0.30, 0.35, 0.40, 0.45) is used as the simulated fetal DNA concentration for each sample.

The simulated chromosome 1 is the reference chromosome in the sample, wherein the genotype of each polymorphic site is simulated as one of the normal disomy-disomy genotypes, and the total count of the individual alleles of each polymorphic site is 200.

The simulated chromosome 2 is a disomy-disomy chromosome in the sample, and the total count of the individual alleles simulated for each site is 200. For any simulated site, one of the alleles is selected to be marked as wild-type (normal type, represented by a capital letter A), and the remaining alleles are marked as mutant types (represented by a lowercase letter a, b, c or d, respectively), so each simulated site can only be one of the following 14 genotypes, namely AA|AA, AA|Aa, Aa|AA, Aa|Aa, Aa|Ab, Aa|aa, Aa|ab, aa|aa, aa|ab, ab|aa, ab|aa, ab|ab or ab|ac, respectively. 100 sites to be detected on chromosome 2 are randomly simulated, and one of the 14 genotypes is randomly selected for each site, and then sequences of individual alleles thereof are simulated in proportion according to the set fetal DNA concentration and the method described in Example 3.

Using the simulated allelic sequence of each sample as the input file, the ART simulation software is used to simulate the high-throughput sequencing results, where the fold parameter of the ART simulation software is set as 50 or 100.

6. Simulation of single genome samples.

In order to detect variations at the chromosomal or sub-chromosomal level of single genomes, we have simulated non-pregnant women's genomic DNA samples (such as pre-implantation embryonic genomic DNA samples), wherein 5 chromosomes, numbered as No. 1 (Chr01) to No. 5 (Chr05), respectively, are simulated for each sample. 100 polymorphic sites are simulated according to the method described in Example 3 on chromosome 1 to chromosome 5 in each sample. Here, normal chromosomes are marked as disomy, each micro-deletion region is regarded as a whole chromosome, and each micro-duplication region is regarded as a pair of chromosomes, and the polymorphic site is selected from the micro-deletion/micro-deletion region. Among them, in a single genome, a pair of chromosomes where one chromosome is normal and one chromosome contains a micro-deletion are marked as monosomy, while a pair of chromosomes where two chromosomes both contain a micro-deletion are marked as nullisomy, and a pair of chromosomes where one chromosome is normal and one chromosome contains a micro-duplication are marked as trisomy, while a pair of chromosomes where two chromosomes both contain a micro-duplication are marked as tetrasomy.

The simulated chromosome 1 is a normal disomy chromosome in the sample, wherein the genotype of each polymorphic site is simulated as one of the normal disomy genotypes (AA or AB), and the total count of the individual alleles of each polymorphic site is 200.

The simulated chromosome 2 is a nullisomy or homozygous micro-deletion chromosome in the sample, wherein the genotype of each polymorphic site is simulated as a normal nullisomy or homozygous micro-deletion genotype (ØØ), and the total count of the individual alleles of each polymorphic site is 0, so the simulation produces no specific amplification sequence or the simulation produces some random sequences which cannot be located to any chromosome.

The simulated chromosome 3 is a monosomy or heterozygous micro-deletion chromosome in the sample, wherein the genotype of each polymorphic site is simulated as a monosomy or heterozygous micro-deletion genotype (AØ) and the total count of the individual alleles of each polymorphic site is 100.

The simulated chromosome 4 is a trisomy or heterozygous micro-duplication chromosome in the sample, wherein the genotype of each polymorphic site is simulated as one of the trisomy or heterozygous micro-duplication genotypes (AAA, AAB or ABC), and the total count of the individual alleles of each polymorphic site is 300.

The simulated chromosome 5 is a tetrasomy or homozygous micro-duplication chromosome in the sample, wherein the genotype of each polymorphic site is simulated as a tetrasomy or homozygous micro-duplication genotype (AAAA, AAAB, AABB, AABC or ABCD), and the total count of the individual alleles of each polymorphic site is 400.

Using the simulated allelic sequence of each sample as the input file, the ART simulation software is used to simulate the high-throughput sequencing results, where the fold parameter of the ART simulation software is set as 50 or 100.

**Example 22. Detection of fetal chromosomal monosomy abnormalities using fetal DNA concentrations in plasma DNA samples of pregnant women and allele counts of sites to be analyzed**

According to the method described in Example 21, the plasma DNA samples of pregnant women containing chromosomal monosomy are simulated, wherein chromosomes 1, 2 and 3 are the reference chromosome, the chromosome with a normal disomy-disomy karyotype and the chromosome with an abnormal disomy-monosomy karyotype, respectively.

Analyzing the sequencing data of the simulated samples: firstly using the allele counts of individual polymorphic sites on the reference chromosome 1 to estimate the concentration f of fetal DNA in the samples according to the method described in Example 8; then according to the fetal DNA concentration f in the samples and the allele counts of individual polymorphic sites on chromosome 2 or 3, estimating the karyotype of chromosome 2 or 3, respectively, according to the method described in Example 17. In order to detect whether the fetuses have chromosomal monosomy abnormalities on chromosome 2 or 3, we need to consider whether the individual allele counts for individual polymorphism sites on chromosome 2 or 3 have better comprehensive goodness-of-fit test results for genotypes with a disomy-disomy karyotype or a genotype with a disomy-monosomy karyotype.

Figure 5 shows detection of monosomy abnormalities in fetal chromosomes in simulated samples by using a goodness-of-fit test. Figure 5a shows detection of fetal monosomy abnormalities in chromosomes of a normal disomy-disomy karyotype in simulated samples by using comprehensive goodness-of-fit test results. Among it, the AIC values on the y-axis are the corrected AIC values, which are obtained by dividing the AIC values of the G-test at the site by the fetal concentration and then further dividing it by the total allele count of the site. Figure 5b shows detection of fetal monosomy abnormalities in chromosomes of a disomy-monosomy karyotype in simulated samples by using comprehensive goodness-of-fit test results. For the normal chromosome (chromosome 2 of a disomy-disomy karyotype), almost all polymorphic sites have a good fit for the genotype of a disomy-disomy karyotype, but are not fitted well for the genotype of a disomy-monosomy karyotype. For the abnormal chromosome (chromosome 3 of a disomy-monosomy karyotype), almost all polymorphic sites have a good fit for the genotype of a disomy-monosomy karyotype, but are not fitted well for the genotype of a disomy-disomy karyotype. Therefore, the test results are that no chromosomal monosomy abnormality is found on fetal chromosome 2 and that a chromosomal monosomy abnormality is found on fetal chromosome 3.

**Example 23. Detection of fetal chromosomal trisomy abnormalities using fetal DNA concentrations in plasma DNA samples of pregnant women and allele counts of sites to be analyzed**

According to the method described in Example 21, the plasma DNA samples of pregnant women containing chromosomal trisomy are simulated, wherein chromosomes 1, 2 and 3 are the reference chromosome, the chromosome with a normal disomy-disomy karyotype and the chromosome with an abnormal disomy-trisomy karyotype, respectively.

Analyzing the sequencing data of the simulated samples: firstly using the allele counts of individual polymorphic sites on the reference chromosome 1 to estimate the concentration f of fetal DNA in the samples according to the method described in Example 8; then according to the fetal DNA concentration f in the samples and the allele counts of individual polymorphic sites on chromosome 2 or 3, estimating the karyotype of chromosome 2 or 3, respectively, according to the method described in Example 17. In order to detect whether the fetuses have chromosomal trisomy abnormalities on chromosome 2 or 3, we need to consider whether the individual allele counts for individual polymorphism sites on chromosome 2 or 3 have better comprehensive goodness-of-fit test results for genotypes with a disomy-disomy karyotype or a genotype with a disomy-trisomy karyotype.

Figure 6 shows detection of trisomy abnormalities in fetal chromosomes in simulated samples by using a goodness-of-fit test. Figure 6a shows detection of fetal trisomy abnormalities in chromosomes of a normal disomy-disomy karyotype in simulated samples by using comprehensive goodness-of-fit test results. Among it, the AIC values on the y-axis are the corrected AIC values, which are obtained by dividing the AIC values of the G-test at the site by the fetal concentration and then further dividing it by the total allele count of the site. Figure 6b shows detection of fetal trisomy abnormalities in chromosomes of a disomy-trisomy karyotype in simulated samples by using comprehensive goodness-of-fit test results. For the normal chromosome (chromosome 2 of a disomy-disomy karyotype), almost all polymorphic sites have a good fit for the genotype of a disomy-disomy karyotype, but are not fitted well for the genotype of a disomy-trisomy karyotype. For the abnormal chromosome (chromosome 3 of a disomy-trisomy karyotype), almost all polymorphic sites have a good fit for the genotype of a disomy-trisomy karyotype, but are not fitted well for the genotype of a disomy-disomy karyotype. Therefore, the test results are that no chromosomal trisomy abnormality is found on fetal chromosome 2 and that a chromosomal trisomy abnormality is found on fetal chromosome 3.

**Example 24. Detection of fetal chromosomal micro-deletion abnormalities using fetal DNA concentrations in plasma DNA samples of pregnant women and allele counts of sites to be analyzed**

According to the method described in Example 21, the plasma DNA samples of pregnant women containing chromosomal micro-deletions are simulated, wherein chromosomes 1 to 7 are the reference chromosome, the chromosome wherein both the mother and fetus are normal (the chromosome with a normal disomy-disomy karyotype), the chromosome wherein the mother is normal while the fetus has a chromosome with a micro-deletion (the chromosome with a disomy-monosomy karyotype), the chromosome wherein the mother has a chromosome with a micro-deletion while the fetus is normal (the chromosome with a monosomy-disomy karyotype), the chromosome wherein both the mother and fetus have a chromosome with a micro-deletion (the chromosome with a monosomy-monosomy karyotype), the chromosome wherein the mother has a chromosome with a micro-deletion while the fetus has a pair of chromosomes with a micro-deletion (the chromosome with a monosomy-nullisomy karyotype), and the chromosome wherein both the mother and fetus have a pair of chromosomes with a micro-deletion (the chromosome with a nullisomy-nullisomy karyotype), respectively.

Analyzing the sequencing data of the simulated samples: firstly using the allele counts of individual polymorphic sites on the reference chromosome 1 to estimate the concentration f of fetal DNA in the samples according to the method described in Example 8; then according to the fetal DNA concentration f in the samples and the allele counts of individual polymorphic sites on chromosomes 2 to 7, estimating the respective karyotypes of chromosomes 2 to 7, respectively, according to the method described in Example 17. In order to detect whether the fetuses have chromosomal micro-deletion abnormalities on certain chromosome No., we need to use the individual allele counts for individual polymorphism sites on the chromosome to perform a comprehensive goodness-of-fit test for each possible maternal and/or fetal micro-deletion karyotype, and then, according to the karyotype that has the best comprehensive fit for the individual allele counts of all polymorphic sites, determined whether there is a micro-deletion abnormality in the fetal chromosome.

Figure 7 shows detection of micro-deletion abnormalities in fetal chromosomes in simulated samples by using a goodness-of-fit test. Figure 7a shows detection of fetal chromosomal micro-deletion abnormalities in chromosomes of a monosomy-disomy karyotype (the mother has a heterozygous micro-deletion, while the fetus is normal) in simulated samples by using comprehensive goodness-of-fit test results. Among it, the AIC values on the y-axis are the corrected AIC values, which are obtained by dividing the AIC values of the G-test at the site by the fetal concentration and then further dividing it by the total allele count of the site. Figure 7b is a partial enlargement of Figure 7a. Figure 7c shows detection of fetal chromosomal micro-deletion abnormalities in chromosomes of a monosomy-monosomy karyotype (the mother and fetus both have a heterozygous micro-deletion) in simulated samples by using comprehensive goodness-of-fit test results. Figure 7d is a partial enlargement of Figure 7c. For the chromosome of a monosomy-disomy karyotype wherein the fetus is normal, almost all polymorphic sites have a good fit for the genotype of a monosomy-disomy karyotype, but are not fitted well for the genotypes of other possible karyotypes. For the chromosome of a monosomy-monosomy karyotype wherein the fetus contains a micro-deletion, almost all polymorphic sites have a good fit for the genotype of a monosomy-monosomy karyotype, but are not fitted well for the genotypes of other possible karyotypes. Therefore, the test result of Figure 7a and Figure 7b is that no micro-deletion abnormality is found on such fetal chromosome No., and the test result of Figure 7c and Figure 7d is that a micro-deletion abnormality is found on such fetal chromosome No..

**Example 25. Detection of fetal chromosomal micro-duplication abnormalities using fetal DNA concentrations in plasma DNA samples of pregnant women and allele counts of sites to be analyzed**

According to the method described in Example 21, the plasma DNA samples of pregnant women containing chromosomal micro-duplications are simulated, wherein chromosomes 1 to 7 are the reference chromosome, the chromosome wherein both the mother and fetus are normal (the chromosome with a normal disomy-disomy karyotype), the chromosome wherein the mother is normal while the fetus has a chromosome with a micro-duplication (the chromosome with a disomy-trisomy karyotype), the chromosome wherein the mother has a chromosome with a micro-duplication while the fetus is normal (the chromosome with a trisomy-disomy karyotype), the chromosome wherein both the mother and fetus have a chromosome with a micro-duplication (the chromosome with a trisomy-trisomy karyotype), the chromosome wherein the mother has a chromosome with a micro-duplication while the fetus has a pair of chromosomes with a micro-duplication (the chromosome with a trisomy-tetrasomy karyotype), and the chromosome wherein both the mother and fetus have a pair of chromosomes with a micro-duplication (the chromosome with a tetrasomy-tetrasomy karyotype), respectively.

Analyzing the sequencing data of the simulated samples: firstly using the allele counts of individual polymorphic sites on the reference chromosome 1 to estimate the concentration f of fetal DNA in the samples according to the method described in Example 8; then according to the fetal DNA concentration f in the samples and the allele counts of individual polymorphic sites on chromosomes 2 to 7, estimating the respective karyotypes of chromosomes 2 to 7, respectively, according to the method described in Example 17. In order to detect whether the fetuses have chromosomal micro-duplication abnormalities on certain chromosome No., we need to use the individual allele counts for individual polymorphism sites on the chromosome to perform a comprehensive goodness-of-fit test for each possible maternal and/or fetal micro-duplication karyotype, and then, according to the karyotype that has the best comprehensive fit for the individual allele counts of all polymorphic sites, determined whether there is a micro-duplication abnormality in the fetal chromosome.

Figure 8 shows detection of micro-duplication abnormalities in fetal chromosomes in simulated samples by using a goodness-of-fit test. Figure 8a shows detection of fetal chromosomal micro-duplication abnormalities in chromosomes of a trisomy-disomy karyotype (the mother has a heterozygous micro-duplication, while the fetus is normal) in simulated samples by using comprehensive goodness-of-fit test results. Among it, the AIC values on the y-axis are the corrected AIC values, which are obtained by dividing the AIC values of the G-test at the site by the fetal concentration and then further dividing it by the total allele count of the site. Figure 8b is a partial enlargement of Figure 8a. Figure 8c shows detection of fetal chromosomal micro-duplication abnormalities in chromosomes of a trisomy-trisomy karyotype (the mother and fetus both have a heterozygous micro-duplication) in simulated samples by using comprehensive goodness-of-fit test results. Figure 8d is a partial enlargement of Figure 8c. For the chromosome of a trisomy-disomy karyotype wherein the fetus is normal, almost all polymorphic sites have a good fit for the genotype of a trisomy-disomy karyotype, but are not fitted well for the genotypes of other possible karyotypes. For the chromosome of a trisomy-trisomy karyotype wherein the fetus contains a micro-duplication, almost all polymorphic sites have a good fit for the genotype of a trisomy-trisomy karyotype, but are not fitted well for the genotypes of other possible karyotypes. Therefore, the test result of Figure 8a and Figure 8b is that no micro-duplication abnormality is found on such fetal chromosome No., and the test result of Figure 8c and Figure 8d is that a micro-duplication abnormality is found on such fetal chromosome No..

**Example 26. Detection of wild-mutant types of sites to be analyzed using fetal DNA concentrations in plasma DNA samples of pregnant women and allele counts of sites to be analyzed**

According to the method described in Example 21, the plasma DNA samples of pregnant women containing specific short-sequence site variations were simulated, wherein chromosomes 1 to 2 were the reference chromosome and the chromosome containing specific short-sequence site variations, respectively. Specifically, each polymorphic site in chromosome 1 was selected from different chromosomal regions, while multiple polymorphic sites in chromosome 2 were selected from the same specific site, which, however, pertains to the results of independent amplifications performed with the same and/or different primers, that is, the simulated polymorphic sites on chromosome 2 represent distinct independent replicates of a particular site.

In order to detect wild-mutant types at specific sites, we have adopted two schemes: (1) directly perform a goodness-of-fit test on all possible wild-mutant genotypes and comprehensively analyze the results of the goodness-of-fit test; and (2) firstly estimate the genotypes of the sites to be tested without distinguishing the wild-mutant alleles, and then determine the wild-mutant types of individual alleles in the estimated genotypes, thereby determining the wild-mutant types of individual alleles in the mother and/or fetus.

(1) Perform a goodness-of-fit test for all possible wild-mutant genotypes. (a) Estimating the concentration f of fetal DNA in the sample according to the method described in Example 8 by using the allele counts of individual polymorphic sites on the reference chromosome 1. (b) Listing all possible wild-mutant genotypes at this specific site on chromosome 2, i.e. AA|AA, AA|Aa, Aa|AA, Aa|Aa, Aa|Ab, Aa|aa, Aa|ab, aa|aa, aa|aa, aa|ab, ab|aa, ab|aa, ab|ab and ab|ac, where A represents the wild-type allele and a, b, and c represent the respective mutant alleles. (c) For each wild-mutant genotype, estimating the theoretical counts of individual alleles according to the concentration f of fetal DNA in the sample as estimated in step (a) above. (d) For each wild-mutant genotype, determine its actual count according to the nucleic acid sequences of individual alleles. (e) Performing a goodness-of-fit test for each wild-mutant genotype with respect to independent replicates for each site. (f) Comprehensively analyzing the results of the goodness-of-fit test, and selecting the wild-mutant type with the comprehensively best fit for all replicated sites as the estimated genotype of the specific site. (g) Determining the wild-mutant type for each allele of the mother and/or fetus based on the estimated wild-mutant genotype.

(2) Firstly estimate the genotypes without distinguishing the wild-mutant alleles, and then determine the wild-mutant types of individual alleles of the mother and/or fetus according to the wild-mutant nucleic acid sequences of individual alleles.

Analyzing the sequencing data of the simulated samples: firstly using the allele counts of individual polymorphic sites on the reference chromosome 1 to estimate the concentration f of fetal DNA in the samples according to the method described in Example 8; then according to the fetal DNA concentration f in the samples and the allele counts of individual specific short-sequence sites to be detected on chromosome 2, estimating the genotypes of individual specific short-sequence sites on chromosome 2 according to the method described in Example 11, respectively. In order to detect whether the fetus has genetic variations at the short sequence level, such as point mutations, short indel mutations, etc. that cause some monogenic diseases, the genotype is firstly estimated for each replicated site to be detected in accordance with the method described in Example 11 without considering whether individual allele sequences belong to the wild-type sequences, and then whether the site has any variation in the mother and fetus is determined according to whether the sequences of individual alleles are normal wild-type sequences.

Figure 9 shows detection of wild-mutant types of fetal short sequence sites in simulated samples by using a goodness-of-fit test. Figure 9a shows detection of the genotype of a simulated short-sequence site where the mother has a heterozygous mutation, while the fetus is normal by using goodness-of-fit test results (different dots represent different independent replicates of the target site of interest to be detected). Among it, the AIC values on the y-axis are the corrected AIC values, which are obtained by dividing the AIC values of the G-test at the site by the fetal concentration and then further dividing it by the total allele count of the site. Figure 9b is a partial enlargement of Figure 9a. The results indicated a genotype where the mother was heterozygous and the fetus was homozygous (AB|AA). Further analysis showed that allele A was a wild-type and allele B was a mutant, so it was determined that the mother was heterozygous for the mutation and the fetus was normal with respect to this site. Figure 9c shows detection of a genotype of a simulated short-sequence site where both the mother and fetus has a heterozygous mutation by using goodness-of-fit test results. Figure 9d is a partial enlargement of Figure 9c. The results indicated a genotype where both the mother and fetus were heterozygous (AB|AC). Further analysis showed that allele A was a wild-type and alleles B and C both were a mutant, so it was determined that both the mother and fetus were heterozygous for the mutation with respect to this site, and the fetus either had a *de novo* mutation or inherited an allelic mutation derived from the father.

**Example 27. Estimation of the genotype of a site to be analyzed using the concentration f of the sample having the least component in a sample mixture and the relative distribution diagram of allele counts of the site**

For a site to be detected, the genotype of the site is estimated according to the following steps:

(1) analyzing the sample to be tested, and listing all possible genotypes of the target site of interest;
(2) calculating theoretical values of relative counts of individual alleles in each possible genotype, and for each genotype, selecting at least one non-maximal theoretical value of relative counts of alleles to be plotted against the maximal theoretical value of relative counts of alleles to mark theoretical positions of all possible genotypes;
(3) calculating relative counts of individual alleles of the target DNA site, and selecting at least one non-maximal relative count of alleles to be plotted against the maximal relative count of alleles to mark actual positions of relative counts of alleles of the target DNA site;

(4) inferring the genotype thereof according to the theoretical position distribution and actual position distribution of the target DNA site in the relative count map of alleles.

Figure 10 shows the theoretical distribution of polymorphic sites derived from a normal karyotype chromosome on the relative distribution diagram of alleles in a plasma DNA sample of a pregnant woman. Figure 10a shows all possible genotypes for and theoretical values of the relative counts of individual allele for the polymorphic sites on the chromosome of a normal disomy-disomy karyotype. Figure 10b shows the distribution of the second maximal relative count (RR2) of alleles relative to the maximal relative count (RR1) of alleles at individual polymorphic sites on the chromosome of a normal disomy-disomy karyotype. The results showed that each polymorphic site was distributed in different positions on the relative distribution diagram of allele counts due to difference in genotype, and its genotype could be inferred according to its specific distribution position.

**Example 28. Estimation of the karyotype of a target to be detected using the concentration f of the sample having the least component in a sample mixture and the relative distribution diagram of allele counts of a set of polymorphic sites in the target region**

We use the relative distribution diagram of allele counts of individual polymorphic sites in the target region to detect aneuploidy at the chromosome level or deletion or duplication variation at the sub-chromosomal level, and the main steps are:

(1) analyzing the sample to be tested and listing all possible karyotypes of the sample on the target chromosomal or sub-chromosomal fragment;
(2) for each possible karyotype, listing all possible genotypes of the target DNA sites in the target group on the chromosome or sub-chromosome for such karyotype in the sample, and then for each genotype, selecting at least one non-maximal theoretical value of relative counts of alleles to be plotted against the maximal theoretical value of relative counts of alleles to mark a theoretical position of such genotype;
(3) for each target DNA site in the target group, calculating relative counts of individual alleles and selecting at least one non-maximal relative count of alleles to be plotted against the maximal relative count of alleles to mark the actual location of such site;
(4) inferring the karyotype of the target chromosomal or sub-chromosomal fragment to be detected according to the theoretical position distribution and actual position distribution of all target DNA sites in the relative count map of alleles.

Figure 11 shows the theoretical distribution in the relative distribution diagram of alleles at each polymorphic site on the chromosome where the mother is normal and the fetal has an aneuploid variation in a plasma DNA sample of a pregnant woman. Figure 11a shows all possible genotypes and the theoretical values of relative counts of their respective alleles at polymorphic sites on the chromosomes with a disomy-disomy karyotype and a disomy-monosomy karyotype. Figure 11b shows the theoretical distribution of the second maximal relative count (RR2) of alleles relative to the maximal relative count (RR1) of alleles at each polymorphic site on chromosomes with a disomy-disomy karyotype and a disomy-monosomy karyotype. Figure 11c shows all possible genotypes and the theoretical values of relative counts of their respective alleles at each polymorphic site on the chromosomes with a disomy-disomy karyotype and a disomy-trisomy karyotype. Figure 11d shows the theoretical distribution of the second or fourth maximal relative count (RR2 or RR24) of alleles relative to the maximal relative count (RR1) of alleles at each polymorphic site on chromosomes with a disomy-disomy karyotype and a disomy-trisomy karyotype.

Figure 12 shows the theoretical distribution in the relative distribution diagram of alleles at each polymorphic site on the sub-chromosome wherein the mother or fetus has a micro-deletion or micro-duplication variation in a plasma DNA sample of a pregnant woman. Figure 12a shows all possible genotypes and the theoretical values of relative counts of their respective alleles at polymorphic sites on the chromosome wherein the mother or fetus has a micro-deletion karyotype. Figure 12b shows the theoretical distribution of the second maximal relative count (RR2) of alleles relative to the maximal relative count (RR1) of alleles at each polymorphic site on chromosomes wherein the mother or fetus has a micro-deletion karyotype. Figure 12c shows all possible genotypes and the theoretical values of relative counts of their respective alleles at each polymorphic site on the sub-chromosome wherein the mother has a micro-duplication and the fetus is normal. Figure 12d shows the theoretical distribution of the second or third maximal relative count (RR2 or RR3) of alleles relative to the maximal relative count (RR1) of alleles at each polymorphic site on the sub-chromosome where the mother has a micro-duplication and the fetus is normal.

**Example 29. Estimation of the wild-mutant type of a site to be analyzed using the concentration f of the sample having the least component in a sample mixture and the wild-type of the site and the relative counts of individual non-wild-type alleles thereof**

We use the count of the wild-type allele and the counts of individual non-wild-type alleles of the site to be analyzed to detect the wild-mutant type of the site, and the main steps are:

(1) analyzing the sample to be tested, and listing the wild-type sequence and all possible genotypes of the target site of interest;
(2) calculating theoretical values of relative counts of the wild-type allele and other non-wild-type alleles for each possible genotype, and for each genotype, selecting at least one theoretical value of relative counts of non-wild-type alleles to be plotted against the theoretical value of the relative count of the wild-type allele to mark theoretical positions of all possible genotypes;
(3) calculating relative counts of the wild-type allele and other non-wild-type alleles of the target DNA site of interest, and selecting at least one relative count of non-wild-type alleles to be plotted against the relative count of the wild-type allele to mark the actual position of the genotype of the target DNA site;
(4) inferring the wild-mutant type thereof according to the theoretical position distribution and actual position distribution of the target DNA site in the relative count map of alleles.

Figure 13 shows the relative distribution diagram of individual allele counts of all possible genotypes of the site to be detected on the chromosome of a normal disomy-disomy in a plasma DNA sample of a pregnant woman. Figure 13a shows all possible genotypes and the theoretical values of relative counts of their respective alleles at the site to be detected on the chromosome of a normal disomy-disomy. Figure 13b shows a theoretical distribution diagram of the maximal relative count (RR2) of non-wild-type alleles relative to the relative count (RR1) of the wild-type allele of the site to be detected on the chromosome of a normal disomy-disomy. Among them, A represents the wild-type allele, and a, b or c represents the non-wild-type (mutant) allele.

**Example 30. Detection of fetal chromosomal monosomy abnormalities using fetal DNA concentrations in plasma DNA samples of pregnant women and the relative distribution diagram of allele counts of sites to be analyzed**

According to the method described in Example 21, the plasma DNA samples of pregnant women containing chromosomal monosomy are simulated, wherein chromosomes 1, 2 and 3 are the reference chromosome, the chromosome with a normal disomy-disomy karyotype and the chromosome with an abnormal disomy-monosomy karyotype, respectively.

Analyzing the sequencing data of the simulated samples: firstly using the allele counts of individual polymorphic sites on the reference chromosome 1 to estimate the concentration f of fetal DNA in the samples according to the method described in Example 8; then according to the fetal DNA concentration f in the samples and the allele counts of individual polymorphic sites on chromosome 2 or 3, estimating the karyotype of chromosome 2 or 3, respectively, according to the method described in Example 28. In order to detect whether the fetuses have chromosomal monosomy abnormalities on chromosome 2 or 3, we need to detect whether chromosome 2 or 3 has a normal disomy-disomy karyotype (both the mother and fetus have disomy) or an abnormal disomy-monosomy karyotype (the mother has normal disomy and the fetus has abnormal monosomy). Therefore, we firstly marked the theoretical positions of all disomy-disomy and disomy-monosomy genotypes on the relative distribution diagram of allele counts, respectively, and then determined the karyotype for the chromosome to be analyzed according to the distribution on the relative distribution diagram of allele counts at individual polymorphic sites on the chromosome.

Figure 14 shows the detection of monosomy variations in fetal chromosomes by using the relative distribution diagram of the counts of individual alleles at polymorphic sites. Figure 14a is a plot of the relative counts of alleles for all polymorphic sites on a simulated normal disomy-disomy chromosome. Figure 14b is a plot of the relative counts of alleles for all polymorphic sites on a simulated disomy-monosomy chromosome. The results showed that almost all the relative counts of polymorphic sites in Figure 14a were distributed around the corresponding disomy-disomy genotype clusters, while almost none were distributed around the corresponding disomy-monosomy genotype clusters. However, in Figure 14b, almost all relative counts of polymorphic sites were distributed around the corresponding disomy-monosomy genotype clusters, while almost none were distributed around the corresponding disomy-disomy genotype clusters. Therefore, the karyotype of the chromosome to be analyzed in Figure 14a was of the disomy-disomy type, that is, the chromosome of the fetus was normal; and the karyotype of the chromosome to be analyzed in Figure 14b was of the disomy-monosomy type, that is, the chromosome of the fetus was an abnormal monosomy.

**Example 31. Detection of fetal chromosomal trisomy abnormalities using fetal DNA concentrations in plasma DNA samples of pregnant women and the relative distribution diagram of allele counts of sites to be analyzed**

According to the method described in Example 21, the plasma DNA samples of pregnant women containing chromosomal trisomy are simulated, wherein chromosomes 1, 2 and 3 are the reference chromosome, the chromosome with a normal disomy-disomy karyotype and the chromosome with an abnormal disomy-trisomy karyotype, respectively.

Analyzing the sequencing data of the simulated samples: firstly using the allele counts of individual polymorphic sites on the reference chromosome 1 to estimate the concentration f of fetal DNA in the samples according to the method described in Example 8; then according to the fetal DNA concentration f in the samples and the allele counts of individual polymorphic sites on chromosome 2 or 3, estimating the karyotype of chromosome 2 or 3, respectively, according to the method described in Example 28. In order to detect whether the fetuses have chromosomal trisomy abnormalities on chromosome 2 or 3, we need to detect whether chromosome 2 or 3 has a normal disomy-disomy karyotype (both the mother and fetus have disomy) or an abnormal disomy-trisomy karyotype (the mother has normal disomy and the fetus has abnormal trisomy). Therefore, we firstly marked the theoretical positions of all disomy-disomy and disomy-trisomy genotypes on the relative distribution diagram of allele counts, respectively, and then determined the karyotype for the chromosome to be analyzed according to the distribution on the relative distribution diagram of allele counts at individual polymorphic sites on the chromosome.

Figure 15 shows the detection of trisomy variations in fetal chromosomes by using the relative distribution diagram of the counts of individual alleles at polymorphic sites. Figure 15a is a plot of the relative counts of alleles for all polymorphic sites on a simulated normal disomy-disomy chromosome. Figure 15b is a plot of the relative counts of alleles for all polymorphic sites on a simulated disomy-trisomy chromosome. The results showed that almost all the relative counts of polymorphic sites in Figure 15a were distributed around the corresponding disomy-disomy genotype clusters, while almost none were distributed around the corresponding disomy-trisomy genotype clusters. However, in Figure 15b, almost all relative counts of polymorphic sites were distributed around the corresponding disomy-trisomy genotype clusters, while almost none were distributed around the corresponding disomy-monosomy genotype clusters. Therefore, the karyotype of the chromosome to be analyzed in Figure 15a was of the disomy-disomy type, that is, the chromosome of the fetus was normal; and the karyotype of the chromosome to be analyzed in Figure 15b was of the disomy-trisomy type, that is, the chromosome of the fetus was an abnormal trisomy.

**Example 32. Detection of fetal chromosomal micro-deletion abnormalities using fetal DNA concentrations in plasma DNA samples of pregnant women and the relative distribution diagram of allele counts of sites to be analyzed**

According to the method described in Example 21, the plasma DNA samples of pregnant women containing chromosomal micro-deletions are simulated, wherein chromosomes 1 to 7 are the reference chromosome, the chromosome wherein both the mother and fetus are normal (the chromosome with a normal disomy-disomy karyotype), the chromosome wherein the mother is normal while the fetus has a chromosome with a micro-deletion (the chromosome with a disomy-monosomy karyotype), the chromosome wherein the mother has a chromosome with a micro-deletion while the fetus is normal (the chromosome with a monosomy-disomy karyotype), the chromosome wherein both the mother and fetus have a chromosome with a micro-deletion (the chromosome with a monosomy-monosomy karyotype), the chromosome wherein the mother has a chromosome with a micro-deletion while the fetus has a pair of chromosomes with a micro-deletion (the chromosome with a monosomy-nullisomy karyotype), and the chromosome wherein both the mother and fetus have a pair of chromosomes with a micro-deletion (the chromosome with a nullisomy-nullisomy karyotype), respectively.

Analyzing the sequencing data of the simulated samples: firstly using the allele counts of individual polymorphic sites on the reference chromosome 1 to estimate the concentration f of fetal DNA in the samples according to the method described in Example 8; then according to the fetal DNA concentration f in the samples and the allele counts of individual polymorphic sites on chromosomes 2 to 7, estimating the karyotypes of chromosomes 2 to 7, respectively, according to the method described in Example 28. In order to detect whether the fetuses have chromosomal micro-deletion abnormalities on certain chromosome No., we need to detect whether such chromosome has a normal disomy-disomy karyotype (both the mother and fetus have disomy) or one of abnormal karyotypes containing a micro-deletion (such chromosome of the mother and/or the fetus has a micro-deletion). Therefore, we firstly marked the positions of all genotypes in cases, where the mother and fetal chromosomes may contain micro-deletions, on the relative distribution diagram of allele counts, respectively, and then determined the karyotype for the chromosome to be analyzed according to the distribution on the relative distribution diagram of allele counts at individual polymorphic sites on the chromosome.

Figure 16 shows the detection of micro-deletion variations in fetal chromosomes by using the relative distribution diagram of the counts of individual alleles at polymorphic sites. Figure 16a is a plot of the relative counts of alleles for all polymorphic sites on a simulated monosomy-disomy chromosome. Figure 16b is a plot of the relative counts of alleles for all polymorphic sites on a simulated monosomy-monosomy chromosome. The results showed that almost all the relative counts of polymorphic sites in Figure 16a were distributed around the corresponding monosomy-disomy genotype clusters, while almost none were distributed around the genotype clusters of other karyotypes. However, in Figure 16b, almost all relative counts of polymorphic sites were distributed around the corresponding monosomy-monosomy genotype clusters, while almost none were distributed around the genotype clusters of other karyotypes. Therefore, the karyotype of the chromosome to be analyzed in Figure 16a was of the monosomy-disomy type, that is, the chromosome of the fetus was normal and contained no micro-deletion; and the karyotype of the chromosome to be analyzed in Figure 16b was of the monosomy-monosomy type, that is, one chromosome of the fetus contained a micro-deletion variation.

**Example 33. Detection of fetal chromosomal micro-duplication abnormalities using fetal DNA concentrations in plasma DNA samples of pregnant women and the relative distribution diagram of allele counts of sites to be analyzed**

According to the method described in Example 21, the plasma DNA samples of pregnant women containing chromosomal micro-duplications are simulated, wherein chromosomes 1 to 7 are chromosomes 1 to 7 are the reference chromosome, the chromosome wherein both the mother and fetus are normal (the chromosome with a normal disomy-disomy karyotype), the chromosome wherein the mother is normal while the fetus has a chromosome with a micro-duplication (the chromosome with a disomy-trisomy karyotype), the chromosome wherein the mother has a chromosome with a micro-duplication while the fetus is normal (the chromosome with a trisomy-disomy karyotype), the chromosome wherein both the mother and fetus have a chromosome with a micro-duplication (the chromosome with a trisomy-trisomy karyotype), the chromosome wherein the mother has a chromosome with a micro-duplication while the fetus has a pair of chromosomes with a micro-duplication (the chromosome with a trisomy-tetrasomy karyotype), and the chromosome wherein both the mother and fetus have a pair of chromosomes with a micro-duplication (the chromosome with a tetrasomy-tetrasomy karyotype), respectively.

Analyzing the sequencing data of the simulated samples: firstly using the allele counts of individual polymorphic sites on the reference chromosome 1 to estimate the concentration f of fetal DNA in the samples according to the method described in Example 8; then according to the fetal DNA concentration f in the samples and the allele counts of individual polymorphic sites on chromosomes 2 to 7, estimating the karyotypes of chromosomes 2 to 7, respectively, according to the method described in Example 28. In order to detect whether the fetuses have chromosomal micro-duplication abnormalities on certain chromosome No., we need to detect whether such chromosome has a normal disomy-disomy karyotype (both the mother and fetus have disomy) or one of abnormal karyotypes containing a micro-duplication (such chromosome of the mother and/or the fetus has a micro-duplication). Therefore, we firstly marked the positions of all genotypes in cases, where the mother and fetal chromosomes may contain micro-duplications, on the relative distribution diagram of allele counts, respectively, and then determined the karyotype for the chromosome to be analyzed according to the distribution on the relative distribution diagram of allele counts at individual polymorphic sites on the chromosome. Since the total number of all genotypes that may contain micro-duplications in the mother and fetal chromosomes is tens or hundreds, while marking all these genotypes on the relative distribution diagram of allele counts is very unfavorable for classification analysis of relative counts of alleles at individual polymorphic sites, here we only marked the distribution of genotypes where the fetus was normal and did not contain micro-duplications. If the relative counts of alleles at individual polymorphic sites on the chromosome to be tested are not observed to be clustered at the position corresponding to the normal genotype of the fetus but clustered at other positions, it means that the chromosome in the sample contains fetal micro-duplication variations or other types of variations.

Figure 17 shows the detection of micro-duplication variations in fetal chromosomes by using the relative distribution diagram of the counts of individual alleles at polymorphic sites. Figure 17a is a plot of the relative counts of alleles for all polymorphic sites on a simulated trisomy-disomy chromosome. Figure 17b is a plot of the relative counts of alleles for all polymorphic sites on a simulated trisomy-trisomy chromosome. The results showed that almost all the relative counts of polymorphic sites in Figure 17a were distributed around the corresponding genotype cluster wherein the fetus was normal. However, in Figure 17b, all relative counts of polymorphic sites were clearly divided into several clusters but were not clustered around the genotype cluster wherein the fetus was normal. Therefore, in the chromosomes to be analyzed in Figure 17a, the fetal chromosome was normal and did not contain a micro-duplication; while in the chromosomes to be analyzed in Figure 17b, either at least one of the chromosomes of the fetus contained a micro-duplication variation, or the chromosome had other types of variations.

**Example 34. Detection of wild-mutant types of sites to be analyzed using fetal DNA concentrations in plasma DNA samples of pregnant women and the relative distribution diagram of allele counts of sites to be analyzed**

According to the method described in Example 21, the plasma DNA samples of pregnant women containing specific short-sequence site variations were simulated, wherein chromosomes 1 to 2 were the reference chromosome and the chromosome containing specific short-sequence site variations, respectively. Specifically, each polymorphic site in chromosome 1 was selected from different chromosomal regions, while multiple polymorphic sites in chromosome 2 were selected from the same specific site, which, however, pertains to the results of independent amplifications performed with the same and/or different primers, that is, the simulated polymorphic sites on chromosome 2 represent distinct independent replicates of a particular site.

Analyzing the sequencing data of the simulated samples: firstly using the allele counts of individual polymorphic sites on the reference chromosome 1 to estimate the concentration f of fetal DNA in the samples according to the method described in Example 8; then according to the fetal DNA concentration f in the samples and the allele counts of individual specific short-sequence sites to be detected on chromosome 2, estimating the wild-mutant types of individual specific short-sequence sites on chromosome 2 according to the method described in Example 29, respectively. In order to detect whether the fetus has short genetic variations, such as point mutations, short indel mutations, etc. that cause some monogenic diseases, all possible genotypes (wild-type alleles are marked as capital letter A, and mutants are marked as small letters a-c according to allele counts in descending order) of the fetus and mother needs to be considered for each site, including genotypes where four copies of genes of the mother and fetus all are of non-wild-type variations (aa|aa, aalab, ablaa, ablab or ablac), genotypes where two copies of genes of the mother are of non-wild-type variations and the fetus is of a heterozygous variation of wild-type and mutant (aa|Aa or ab|Aa), a genotype where the mother is of a heterozygous variation of wild-type and mutant and the fetus is normal (Aa|AA), genotypes where both the mother and fetus are of a heterozygous variation of wild-type and mutant (AalAa or AalAb), genotypes where the mother is of a heterozygous variation of wild-type and mutant and the fetus is of non-wild-type variations (Aalaa or Aalab), a genotype where the mother is normal and the fetal is of a heterozygous variation of wild-type and mutant (AA|Aa), and a genotype where the mother and fetus both are of a normal wild-type (AA|AA). Among them, 20-fold biological replicates at the sequencing level were performed for each simulated site to be detected.

Figure 18 shows the detection of variations of the fetus at the short sequence level by using the relative distribution diagram of the counts of individual alleles at polymorphic sites. Figure 18a is a plot of relative counts of alleles for a polymorphic site in the simulated ablaa genotype. The genotype of the polymorphic site was estimated to be ablaa type, i.e. a type where the mother is of a heterozygous variation that is a double mutant and the fetus is of a heterozygous variation that is a wild-mutant type, according to the clustered distribution of biological replicates at the sequencing level of the polymorphic site on the relative count distribution diagram. Figure 18b is a plot of relative counts of alleles for a polymorphic site in the simulated Aalab genotype. The genotype of the polymorphic site was estimated to be Aalab type type, i.e. a type where the mother is of a heterozygous variation that is a wild-mutant type and the fetus is of a heterozygous variation that is a double mutant, according to the clustered distribution of biological replicates at the sequencing level of the polymorphic site on the relative count distribution diagram.

**Example 35. Detection of genetic variations in a single genome sample using allele counts and the relative distribution diagram of sites to be analyzed**

We use the relative distribution diagram of allele counts of individual polymorphic sites in the target region to detect the aneuploidy at the chromosome level or the deletion and duplication variations at the sub-chromosomal level of the target to be detected in a single genome sample, and the main steps are:

(1) calculating the relative counts of individual alleles of each target DNA site in the target group;
(2) for each target DNA site, plotting a distribution diagram A of the second maximal relative count of alleles to the maximal relative count of alleles or plotting a distribution diagram B of the maximal relative count of alleles to the relative position of the target DNA site on the chromosome or sub-chromosome;
(3) estimating the karyotype of the target to be detected in a single genome sample by using the relative distribution diagram A and/or distribution diagram B of allele counts of individual target DNA sites in the target group.

A single genome sample was simulated according to the method described in Example 21, wherein chromosomes 1 to 5 are disomy, nullisomy (or homozygous micro-deletion), monosomy (or heterozygous micro-deletion), trisomy (or heterozygous micro-duplication), and tetrasomy (or homozygous micro-duplication), respectively.

In order to detect whether a single genome sample has a chromosomal or sub-chromosomal level variation, the following five situations need to be considered: (1) there is deletion of both of the pair of chromosomes (nullisomy) or micro-deletion of the same region in both of the pair of chromosomes (homozygous micro-deletion); (2) one of the pair of chromosomes is normal while the other is absent (monosomy) or a micro-deletion is present in the other chromosome (heterozygous micro-deletion); (3) both of the pair of chromosomes are normal; (4) there are three chromosomes (trisomy), or one of the pair of chromosomes is normal and there are a micro-duplication in the other chromosome (heterozygous micro-duplication) (5) there are four chromosomes (tetrasomy), or there are micro-duplication in the same region in both of the pair of chromosomes (homozygous micro-duplication).

Figure 19 shows the detection of the karyotype of a target chromosome or sub-chromosome in a single-genome sample by using the relative counts of individual alleles at polymorphic sites. For each polymorphic site on the target region (chromosomal or sub-chromosomal region), the second maximal relative count of alleles is plotted against the maximal relative count of alleles (relative count map A), or the maximal relative count of alleles is plotted against the relative position of the site on the simulated chromosome (relative count position map B). The results show that the genotypes of chromosomes with different karyotypes have different characteristic distributions on the relative count map A or relative count position map B, and the karyotype (variation type) of the target chromosome or sub-chromosome can be detected according to these characteristic distributions.

In addition, all methods described herein can be performed in any suitable order unless otherwise indicated herein or otherwise clearly contradicted by context. The use of any and/or all examples and/or exemplary language provided in certain Examples herein is intended merely to better illuminate the invention and does not pose a limitation on the scope of the invention otherwise claimed. No language in the specification should be construed as indicating any non-claimed element as a necessity to the practice of the invention.

Groups of alternative elements or Examples of the invention disclosed herein are not to be construed as limiting. Each group member may be referred to or claimed alone or in any combination with other members of the group or other elements found herein. One or more members of a group may be included in, or deleted from, such group for reasons of convenience and/or patentability.

Although the present technology has been described in sufficient detail with reference to one or more specific embodiments, those of ordinary skill in the art will recognize that changes may be made to the embodiments specifically disclosed in this application, and such modifications and improvements are within the scope and spirit of the technology. Thus, the subject matter of the invention is not to be restricted except in the scope of the appended claims. Furthermore, when interpreting the specification and claims, all terms should be interpreted in the broadest possible manner consistent with the context.

**Claims**

1. A method for calculating a concentration of the least component DNA in a sample, **characterized in that** the method comprises the steps of:

   (a1) setting a noise threshold $\alpha$ of the sample;
   (a2) for each target DNA site, firstly using counts of its individual alleles to estimate its genotype, and then estimating the count (FC) derived from the least component DNA and total count (TC) based on its estimated genotype; and
   (a3) using the count (FC) of the least component DNA and total count (TC) for each target DNA site to estimate the concentration of the least component DNA.

2. The method according to claim 1, **characterized in that** step (a2) comprises the steps of:

   (a2-i) sorting the allele counts of the target DNA site in descending order, wherein the maximal three allele counts are marked in sequence as R1, R2 and R3, respectively;
   (a2-ii) estimating the genotype of the target DNA site using counts of individual alleles for the target DNA site; and
   (a2-iii) based on the estimated genotype of the target DNA site and the individual allele counts for the target DNA site, estimating the count (FC) derived from the least component DNA and total count (TC).

3. The method according to claim 2, **characterized in that** step (a2-ii) comprises the steps of:

   (a2-ii-1) using the counts of individual alleles for the target DNA site to determine the number of alleles that are detected to be higher than the noise threshold in the target DNA site; if the determination result is 1, performing the following step (a2-ii-2); if the determination result is 2, performing the following step (a2-ii-3); if the determination result is greater than 2, performing the following step (a2-ii-4);
   (a2-ii-2) estimating the genotype of the target DNA site as AA|AA, and then performing the following step (a2-ii-5);
   (a2-ii-3) estimating the genotype of the target DNA site based on the number, that is 2, of alleles detected to be higher than the noise threshold and the maximal two allele counts for the target DNA site, and then performing the following step (a2-ii-5);
   (a2-ii-4) estimating the genotype of the target DNA site based on the number, that is greater than 2, of alleles detected to be higher than the noise threshold and at least two maximal allele counts for the target DNA site, and then performing the following step (a2-ii-5); and

(a2-ii-5) outputting the estimated genotype of the target site.

4. The method according to claim 3, **characterized in that** step (a2-ii-3) comprises the steps of:

(a2-ii-3-1) determining whether the value of R1/(R1+R2) is less than $0.5+\alpha$, and if the determination result is yes, estimating the genotype of the target DNA site as AB|AB, and then performing the following step (a2-ii-3-3); if the determination result is no, then performing the following step (a2-ii-3-2);
(a2-ii-3-2) determining whether the value of R1/(R1+R2) is less than 0.75, and if the determination result is yes, estimating the genotype of the target DNA site as AB|AA, and then performing the following step (a2-ii-3-3); if the determination result is no, estimating the genotype of the target DNA site as AAIAB, and then performing the following step (a2-ii-3-3); and
(a2-ii-3-3) outputting the estimated genotype of the target site.

5. The method according to claim 3, **characterized in that** step (a2-ii-4) comprises the steps of:

(a2-ii-4-1) determining whether R2/R1 is greater than or equal to 0.5 and/or whether R1/(R1+R2) is greater than or equal to 1/2 and less than or equal to 2/3 and/or whether R2/(R1+R2) is a value that is greater than or equal to 1/3 and less than or equal to 1/2, and if the determination result is yes, estimating the genotype of the target DNA site as AB|AC, and then performing the following step (a2-ii-4-3); if the determination result is no, then performing the following step (a2-ii-4-2);
(a2-ii-4-2) marking the allele count for this site an an outlier, and then either estimating the genotype of this target site to be NA and performing the following step (a2-ii-4-3), or setting the number of alleles detected to be higher than the noise threshold in the target DNA site to be 2, then estimating the genotype of the target site as described in step (a2-ii-3), and performing the following step (a2-ii-4-3); and
(a2-ii-4-3) outputting the estimated genotype of the target site.

6. The method according to claim 2, **characterized in that** step (a2-iii) comprises the steps of:

(a2-iii-1) if the estimated genotype of the target site is AA|AA, estimating the count (FC) derived from the least component DNA as NA, and the total count (TC) as R1 or R1+R2 or R1+R2+R3, and then performing the following step (a2-iii-7);
(a2-iii-2) if the estimated genotype of the target site is AB AB, estimating the count (FC) derived from the least component DNA as NA, and the total count (TC) as R1+R2 or R1+R2 +R3, and then performing the following step (a2-iii-7);
(a2-iii-3) if the estimated genotype of the target site is AB|AA, estimating the count (FC) derived from the least component DNA as R1-R2, and the total count (TC) as R1+R2 or R1+R2+R3, and then performing the following step (a2-iii-7);
(a2-iii-4) if the estimated genotype of the target site is AA|AB, estimating the count (FC) derived from the least component DNA as 2 times R2, and the total count (TC) as R1+R2 or R1+R2+R3, and then performing the following step (a2-iii-7);
(a2-iii-5) if the estimated genotype of the target site is AB|AC, estimating the count (FC) derived from the least component DNA as R1-R2+R3 or 2 times R3 or 2 times (R1-R2), and the total count (TC) as R1+R2+R3, and then performing the following step (a2-iii-7);
(a2-iii-6) if the estimated genotype of the target site is not one of the above-mentioned genotypes, estimating the count (FC) derived from the least component DNA as NA, and the total count (TC) as R1 or R1+R2 or R1+R2+R3, and then performing the following step (a2-iii-7); and
(a2-iii-7) outputting the estimated count (FC) derived from the least component DNA and total count (TC).

7. A method for calculating a concentration of the least component DNA in a sample, **characterized in that** the method comprises the steps of:

(b 1) setting a noise threshold $\alpha$, an initial concentration estimation value $f_0$ and an iteration error precision value $\varepsilon$ of the sample;
(b2) for each target DNA site, using counts of its individual alleles and the concentration value $f_0$ of the least component DNA in the sample to estimate its genotype;
(b3) for each target DNA site, estimating the count (FC) derived from the least component DNA and total count (TC) based on its estimated genotype;
(b4) using the count (FC) of the least component DNA and total count (TC) of individual target sites to estimate the concentration f of the least component DNA; and

(b5) determining whether the absolute value of $f-f_0$ is less than $\varepsilon$, and if the determination result is no, then setting $f_0=f$, and then performing step (b2); if the determination result is yes, estimating the least component DNA concentration in the sample as f.

**8.** The method according to claim 7, **characterized in that** step (b2) comprises the steps of:

(b2-i) listing all possible genotypes of the target DNA site according to the source of the sample;

(b2-ii) for each possible genotype of the target DNA site, using the concentration value $f_0$ of the least component DNA in the sample and the total count (TC) of individual alleles of the target DNA site to calculate theoretical counts of individual alleles thereof;

(b2-iii) for each possible genotype of the target DNA site, using the counts of individual alleles of the target DNA site and theoretical counts of individual alleles thereof to perform a goodness-of-fit test; and

(b2-iv) analyzing results of the goodness-of-fit test of the target DNA site for all possible genotypes, and selecting the genotype with the best fit for each allele count of the target DNA site as the estimated target DNA site genotype.

**9.** The method according to claim 7, **characterized in that** in step (b3), for each target DNA site, estimating the count (FC) derived from the least component DNA and total count (TC) based on its estimated genotype, wherein the maximal four allele counts are marked in sequence in descending order as R1, R2, R3, and R4, comprises the following steps:

(b3-1) if the estimated genotype of the target site is AA|AA, estimating the count (FC) derived from the least component DNA as NA, and the total count (TC) as R1 or R1+R2 or R1+R2+R3 or R1+R2+R3+R4, and then performing the following step (b3-11);

(b3-2) if the estimated genotype of the target site is AB|AB, estimating the count (FC) derived from the least component DNA as NA, and the total count (TC) as R1+R2 or R1+R2+R3 or R1+R2+R3+R4, and then performing the following step (b3-11);

(b3-3) if the estimated genotype of the target site is AB|AA, estimating the count (FC) derived from the least component DNA as R1-R2, and the total count (TC) as R1+R2 or R1+R2+R3 or R1+R2+R3+R4, and then performing the following step (b3-11);

(b3-4) if the estimated genotype of the target site is AA|AB, estimating the count (FC) derived from the least component DNA as 2 times R2, and the total count (TC) as R1+R2 or R1+R2+R3 or R1+R2+R3+R4, and then performing the following step (b3-11);

(b3-5) if the estimated genotype of the target site is AB|AC, estimating the count (FC) derived from the least component DNA as R1-R2+R3 or 2 times R3 or 2 times (R1-R2), and the total count (TC) as R1+R2+R3 or R1+R2+R3+R4, and then performing the following step (b3-11);

(b3-6) if the estimated genotype of the target site is AA|BB, estimating the count (FC) derived from the least component DNA as R2, and the total count (TC) as R1+R2 or R1+R2+R3 or R1+R2+R3+R4, and then performing the following step (b3-11);

(b3-7) if the estimated genotype of the target site is AA|BC, estimating the count (FC) derived from the least component DNA as R2+R3 or 2 times R2 or 2 times R3, and the total count (TC) as R1+R2+R3 or R1+R2+R3+R4, and then performing the following step (b3-11);

(b3-8) if the estimated genotype of the target site is AB|CC, then determining whether the current estimated value $f_0$ is greater than or equal to 1/3, and if the determination result is yes, estimating the count (FC) derived from the least component DNA as R1, and the total count (TC) as R1+R2+R3 or R1+R2+R3+R4, and then performing the following step (b3-11); if the determination result is no, estimating the count (FC) derived from the least component DNA as R3, and the total count (TC) as R1+R2+R3 or R1+R2+R3+R4, and then performing the following step (b3-11);

(b3-9) if the estimated genotype of the target site is AB|CD, estimating the count (FC) derived from the least component DNA as R3+R4 or 2 times R3 or 2 times R4, and the total count (TC) as R1+R2+R3+R4, and then performing the following step (b3-11);

(b3-10) if the estimated genotype of the target site is not one of the above-mentioned genotypes, estimating the count (FC) derived from the least component DNA as NA, and the total count (TC) as R1 or R1+R2 or R1+R2+R3 or R1+R2+R3+R4, and then performing the following step (b3-11); and

(b3-11) outputting the estimated count (FC) derived from the least component DNA and total count (TC).

**10.** The method according to claim 1 or claim 7, **characterized in that** in step (a3) or step (b4), the concentration of the least component DNA is estimated by fitting a regression model.

**11.** The method according to claim 1 or claim 7, **characterized in that** in step (a3) or step (b4), the concentration of the least component DNA in the sample is calculated by using linear regression and/or robust linear regression and/or the mean of FC and TC and/or the median of FC and TC, according to FC and TC counts.

**12.** The method according to any one of claims 1-11, wherein said sample is a plasma sample of a pregnant woman, and said least component DNA is fetal DNA.

**13.** A method for detecting genetic variations in a sample, **characterized in that** the method comprises the following steps in sequence:

(1) receiving a biological sample to be tested and preparing nucleic acids;
(2) enriching or amplifying target DNA sites, wherein at least one of the target DNA sites has more than one allele in the sample;
(3) sequencing the amplified target DNA sites;
(4) for each target DNA site, counting the counts of its individual alleles; and
(5) determining the karyotype or genotype or wild-mutant type of the target to be detected in the sample by using a goodness-of-fit test of allele counts and/or a relative distribution diagram of allele counts for target DNA sites.

**14.** The method according to claim 13, **characterized in that** in step (5), the goodness-of-fit test of allele counts for the target DNA sites is used for determining the karyotype or genotype or wild-mutant type of the target to be detected in the sample, and the determining comprises the following steps in sequence:

(c1) dividing each target DNA site into reference sites or target sites according to its location on a chromosome, wherein reference sites form a reference group, and target sites forms a target group;
(c2) calculating the concentration of the least component DNA in the sample using the allele counts for individual target DNA sites in the reference group; and
(c3) estimating the karyotype or genotype or wild-mutant type of the target to be detected in the sample by means of the goodness-of-fit test using the allele counts for individual target DNA sites in the target group and the concentration of the least component DNA in the sample.

**15.** The method according to claim 13, **characterized in that** in step (5), the relative distribution diagram of allele counts for target DNA sites is used for determining the karyotype or genotype or wild-mutant type of the target to be detected in the sample, and said determining comprises the following steps in sequence:

(d1) dividing each target DNA site into reference sites or target sites according to its location on the chromosome, wherein reference sites forms a reference group, and target sites forms a target group;
(d2) calculating the concentration of the least component DNA in the sample using the allele counts for individual target DNA sites in the reference group; and
(d3) estimating the karyotype or genotype or wild-mutant type of the target to be detected in the sample by means of the relative distribution diagram of allele counts,
using the allele counts for individual target DNA sites in the target group and the concentration of the least component DNA in the sample.

**16.** The method according to claim 13, **characterized in that** in step (5), the relative distribution diagram of allele counts for target DNA sites is used for determining the karyotype of the target to be detected in the sample, wherein the sample to be detected is a single genome sample, and the determining comprises the following steps in sequence:

(e1) calculating the relative counts of individual alleles of each target DNA site;
(e2) for each target DNA site, plotting a distribution diagram A of its second maximal relative count of alleles to its maximal relative count of alleles or plotting a distribution diagram B of its maximal relative count of alleles to the relative position of the target DNA site on the chromosome or sub-chromosome;
(e3) estimating the karyotype of the target to be detected in a single genome sample by using the relative distribution diagram A and/or distribution diagram B of allele counts of individual target DNA sites.

**17.** The method according to claim 14 or the method according to claim 15, **characterized in that** in step (c2) or step (d2), the method according to any one of claims 1-12 is used to calculate the concentration of the least component DNA in the sample.

18. The method according to claim 14, **characterized in that** in step (c3), the genotype of the target to be detected in the sample is estimated by means of the goodness-of-fit test using the allele counts for individual target DNA sites in the target group and the concentration of the least component DNA in the sample, and the estimating comprises the following steps in sequence:

(c3-a1) for each target DNA site in the target group, listing all possible genotypes thereof;
(c3-a2) for each target DNA site in the target group, calculating theoretical counts of individual alleles for each possible genotype thereof, according to the least component DNA concentration in the sample and the total count of individual alleles at this site;
(c3-a3) for each target DNA site in the target group, using the individual allele counts and theoretical counts of the target DNA site to perform the goodness-of-fit test for each possible genotype thereof; and
(c3-a4) for each target DNA site in the target group, selecting the best-fitting genotype as the genotype of the target DNA site, according to the goodness-of-fit test results for all possible genotypes thereof.

19. The method according to claim 14, **characterized in that** in step (c3), the karyotype of the target to be detected in the sample is estimated by means of the goodness-of-fit test using the allele counts for individual target DNA sites in the target group and the concentration of the least component DNA in the sample, and the estimating comprises the following steps in sequence:

(c3-b1) analyzing the sample to be tested, and listing all possible karyotypes of the target chromosomal or sub-chromosomal fragment to be detected;
(c3-b2) for each possible karyotype, listing all possible genotypes for each target DNA site in the target group;
(c3-b3) for each target DNA site in the target group, firstly using the individual allele counts thereof to perform the goodness-of-fit test for all possible genotypes thereof, and then for each possible karyotype, selecting a genotype with the best fit for such karyotype; and
(c3-b4) comprehensively analyzing the goodness-of-fit test results of all target DNA sites for each karyotype, and selecting a karyotype with the best comprehensive fit for all target DNA sites as the karyotype of the target chromosomal or sub-chromosomal fragment to be detected.

20. The method according to claim 14, **characterized in that** in step (c3), the wild-mutant type of the target to be detected in the sample is estimated by means of the goodness-of-fit test using the allele counts for individual target DNA sites in the target group and the concentration of the least component DNA in the sample, and the estimating comprises the following steps in sequence:

(c3-c1a) for each target DNA site in the target group, listing all possible wild-mutant genotypes thereof;
(c3-c2a) for each target DNA site in the target group, calculating theoretical counts of individual alleles for each possible wild-mutant genotype thereof, according to the least component DNA concentration in the sample and the total count of individual alleles at this site;
(c3-c3a) for each target DNA site in the target group, using the individual allele counts and theoretical counts of the target DNA site to perform the goodness-of-fit test for each possible wild-mutant genotype thereof; and
(c3-c4a) comprehensively analyzing all target DNA sites in the target group, and selecting a wild-mutant genotype with the best fit for all target sites as the wild-mutant genotype of the target to be detected.

21. The method according to claim 14, **characterized in that** in step (c3), the wild-mutant type of the target to be detected in the sample is estimated by means of the goodness-of-fit test using the allele counts for individual target DNA sites in the target group and the concentration of the least component DNA in the sample, and the estimating comprises the following steps in sequence:

(c3-c1b) for each target DNA site in the target group, estimating its genotype by means of the goodness-of-fit test, according to the individual allele counts thereof and the least component DNA concentration in the sample; and
(c3-c2b) determining the wild-mutant type of each allele of the target to be detected in each component of the sample, according to the genotype and the sequence of each allele for each target DNA site in the target group.

22. The method according to claim 14, **characterized in that** said goodness-of-fit test in step (c3) is performed by using a chi-square test, G test, Fisher's exact test, binomial distribution test, variants thereof or combinations thereof.

23. The method according to claim 14, **characterized in that** said goodness-of-fit test in step (c3) is the goodness-of-fit test performed by using calculated values, G values, AIC values, corrected G values, corrected AIC values, variants of G values or AIC values, or combinations thereof, of the G test.

24. The method according to claim 14, **characterized in that** the goodness-of-fit test in step (c3) is the goodness-of-fit test performed by using the method according to claim 8.

25. The method according to claim 15, **characterized in that** in step (d3), the genotype of the target to be detected in the sample is estimated by means of the relative distribution diagram of allele counts, using the allele counts for individual target DNA sites in the target group and the concentration of the least component DNA in the sample, and the estimating comprises the following steps in sequence:

(d3-a1) for each target DNA site in the target group, listing all possible genotypes thereof;
(d3-a2) for each possible genotype of the target DNA sites in the target group, firstly calculating theoretical values of relative counts of alleles thereof according to the concentration of the least component DNA in the sample, and then selecting at least one non-maximal theoretical value of relative counts of alleles to be plotted against the maximal theoretical value of relative counts of alleles to mark a theoretical position of the genotype;
(d3-a3) for each target DNA site in the target group, firstly calculating relative counts of alleles thereof, and then selecting at least one non-maximal relative count of alleles to be plotted against the maximal relative count of alleles to mark the actual position of the target DNA site on the relative count map of alleles; and
(d3-a4) inferring the genotype of the target to be detected according to the theoretical position distribution and actual position distribution of each target DNA site in the target group in the relative count map of alleles.

26. The method according to claim 15, **characterized in that** in step (d3), the karyotype of the target to be detected in the sample is estimated by means of the relative distribution diagram of allele counts, using the allele counts for individual target DNA sites in the target group and the concentration of the least component DNA in the sample, and the estimating comprises the following steps in sequence:

(d3-b 1) analyzing the sample to be tested, and listing all possible karyotypes of the target chromosomal or sub-chromosomal fragment to be detected;
(d3-b2) for each possible karyotype, listing all possible genotypes for each target DNA site in the target group;
(d3-b3) for each possible genotype of the target DNA sites in the target group, firstly calculating theoretical values of relative counts of alleles thereof according to the concentration of the least component DNA in the sample, and then selecting at least one non-maximal theoretical value of relative counts of alleles to be plotted against the maximal theoretical value of relative counts of alleles to mark a theoretical position of the genotype;
(d3-b4) for each target DNA site in the target group, firstly calculating relative counts of alleles thereof, and then selecting at least one non-maximal relative count of alleles to be plotted against the maximal relative count of alleles to mark the actual position of the target DNA site on the relative count map of alleles; and
(d3-b5) inferring the karyotype of the target to be detected according to the theoretical position distribution and actual position distribution in each karyotype of each target DNA site in the target group in the relative count map of alleles.

27. The method according to claim 15, **characterized in that** in step (d3), the wild-mutant type of the target to be detected in the sample is estimated by means of the relative distribution diagram of allele counts, using the allele counts for individual target DNA sites in the target group and the concentration of the least component DNA in the sample, and the estimating comprises the following steps in sequence:

(d3-c1) for each target DNA site in the target group, listing the wild-type sequence and all possible wild-mutant genotypes thereof;
(d3-c2) for each possible wild-mutant genotype, calculating theoretical values of relative counts of its wild-type allele and other non-wild-type alleles, and selecting at least one theoretical value of relative counts of non-wild-type alleles to be plotted against the theoretical value of the relative count of the wild-type allele to mark a theoretical position of its wild-mutant genotype;
(d3-c3) for each target DNA site in the target group, calculating relative count values of its wild-type allele and other non-wild-type alleles, and selecting at least one relative count of non-wild-type alleles to be plotted against the relative count of the wild-type allele to mark the actual position of the target DNA site on the relative count map of alleles;
(d3-c4) inferring the wild-mutant type thereof according to the theoretical position distribution and actual position distribution of all target DNA sites in the target group in the relative count map of alleles.

28. A system for detecting genetic variations in a sample comprising devices and/or computer program products and/or modules for implementing any step in the method of any one of claims 1 to 27.

29. A kit for detecting genetic variations in a sample, said kit comprising primers for performing any of the steps in the method of any one of claims 1 to 27.

Figure 1

Figure 2

Figure 3

Figure 4

Figure 5

Figure 6

Figure 7

Figure 8

Figure 9

**a**

| Genotype | Allele count | | | Allele relative count | | |
|---|---|---|---|---|---|---|
| | R1 | R2 | R3 | RR1 | RR2 | RR3 |
| AA\|AA | $R_mR_mR_fR_f$ | 0 | 0 | 1 | 0 | 0 |
| AA\|AB | $R_mR_mR_f$ | $R_f$ | 0 | $(2-f)/2$ | $f/2$ | 0 |
| AB\|AA | $R_mR_fR_f$ | $R_m$ | 0 | $(1+f)/2$ | $(1-f)/2$ | 0 |
| AB\|AB | $R_mR_f$ | $R_mR_f$ | 0 | $1/2$ | $1/2$ | 0 |
| AB\|AC | $R_mR_f$ | $R_m$ | $R_f$ | $1/2$ | $(1-f)/2$ | $f/2$ |

$R_m$ and $R_f$: allele counts derived from the mother and fetal genomes
f: fetal DNA concentration

**b**

Figure 10

**a**

| Genotype | | Allele relative count | |
|---|---|---|---|
| | | RR1 | RR2 |
| Disomy-Disomy | AA\|AA | 1 | 0 |
| | AA\|AB | (2-f)/2 | f/2 |
| | AB\|AA | (1+f)/2 | (1-f)/2 |
| | AB\|AB | 1/2 | 1/2 |
| | AB\|AC | 1/2 | (1-f)/2 |
| Disomy-Monosomy | AA\|AØ | 1 | 0 |
| | AA\|BØ | 1-f/(2-f) | f/(2-f) |
| | AB\|AØ | 1/(2-f) | 1-1/(2-f) |
| | AB\|CØ | 1-1/(2-f) | 1-1/(2-f) |

**c**

| Genotype | | Allele relative count | | | |
|---|---|---|---|---|---|
| | | RR1 | RR2 | RR3 | RR4 |
| Disomy-Disomy | AA\|AA | 1 | 0 | 0 | 0 |
| | AA\|AB | (2-f)/2 | f/2 | 0 | 0 |
| | AB\|AA | (1+f)/2 | (1-f)/2 | 0 | 0 |
| | AB\|AB | 1/2 | 1/2 | 0 | 0 |
| | AB\|AC | 1/2 | (1-f)/2 | f/2 | 0 |
| Disomy-Trisomy | AA\|AAA | 1 | 0 | 0 | 0 |
| | AA\|AAB | 2/(2+f) | f/(2+f) | 0 | 0 |
| | AA\|ABB | (2-f)/(2+f) | 2f/(2+f) | 0 | 0 |
| | AB\|AAA | (1+2f)/(2+f) | (1-f)/(2+f) | 0 | 0 |
| | AB\|AAB | (1+f)/(2+f) | 1/(2+f) | 0 | 0 |
| | AA\|ABC | (2-f)/(2+f) | f/(2+f) | f/(2+f) | 0 |
| | AB\|AAC | (1+f)/(2+f) | (1-f)/(2+f) | f/(2+f) | 0 |
| | AB\|ABC | 1/(2+f) | 1/(2+f) | f/(2+f) | 0 |
| | AB\|ACC | 1/(2+f) | (1-f)/(2+f) | 2f/(2+f) | 0 |
| | AB\|ACD | 1/(2+f) | (1-f)/(2+f) | f/(2+f) | f/(2+f) |

**b**

**d**

Figure 11

71

Figure 12

**a**

| Genotype | Allele relative count | | |
|---|---|---|---|
| | Wildtype RR1 | Mutant RR2 | Mutant RR3 |
| AA\|AA | 1 | 0 | 0 |
| AA\|Aa | (2−f)/2 | f/2 | 0 |
| Aa\|AA | (1+f)/2 | (1−f)/2 | 0 |
| Aa\|Aa | 1/2 | 1/2 | 0 |
| Aa\|aa | (1−f)/2 | (1+f)/2 | 0 |
| Aa\|Ab | 1/2 | (1−f)/2 | f/2 |
| Aa\|ab | (1−f)/2 | 1/2 | f/2 |
| aa\|Aa | f/2 | (2−f)/2 | 0 |
| aa\|aa | 0 | 1 | 0 |
| aa\|ab | 0 | (2−f)/2 | f/2 |
| ab\|Aa | f/2 | 1/2 | (1−f)/2 |
| ab\|aa | 0 | (1+f)/2 | (1−f)/2 |
| ab\|ab | 0 | 1/2 | 1/2 |
| ab\|ac | 0 | 1/2 | (1−f)/2 |

**b**

Figure 13

Figure 14

Figure 15

**a** Monosomy-Disomy Chromosome

**b** Monosomy-Monosomy Chromosome

Figure 16

Figure 17

a  Mother Double Mutant – Fetus Wild-Mutant type

b  Mother Wild-Mutant type – Fetus Double Mutant

Figure 18

Figure 19

## INTERNATIONAL SEARCH REPORT

| International application No. |
|---|
| **PCT/CN2021/125359** |

**A.    CLASSIFICATION OF SUBJECT MATTER**

C12Q 1/68(2018.01)i

According to International Patent Classification (IPC) or to both national classification and IPC

**B.    FIELDS SEARCHED**

Minimum documentation searched (classification system followed by classification symbols)

C12Q

Documentation searched other than minimum documentation to the extent that such documents are included in the fields searched

Electronic data base consulted during the international search (name of data base and, where practicable, search terms used)

VEN, CNABS, PubMed, ISI Web of Knowledge, CNKI: applicant/inventor, 胎儿, DNA, 等位基因, 母亲, 浓度, 计算, 阈值, 基因型, 血浆, allele, genotype, pregnant, fetal, chromosome, 染色体, 评估, estimat+, , concentration, 比例, proportion

**C.    DOCUMENTS CONSIDERED TO BE RELEVANT**

| Category* | Citation of document, with indication, where appropriate, of the relevant passages | Relevant to claim No. |
|---|---|---|
| X | WO 2009013496 A1 (THE CHINESE UNIVERSITY OF HONG KONG) 29 January 2009 (2009-01-29)<br>    description paragraphs 16, 37, 65, 79, 95, 101, 104, 116-118 | 1-29 |
| A | US 2013267425 A1 (THE CHINESE UNIVERSITY OF HONG KONG) 10 October 2013 (2013-10-10)<br>    entire document | 1-29 |
| A | CN 108138226 A (AFFYMETRIX, INC) 08 June 2018 (2018-06-08)<br>    entire document | 1-29 |
| A | CN 103849684 A (THE CHINESE UNIVERSITY OF HONG KONG) 11 June 2014 (2014-06-11)<br>    entire document | 1-29 |
| A | CN 111951890 A (BEIJING BOHAO YUNTIAN TECHNOLOGY CO., LTD. et al.) 17 November 2020 (2020-11-17)<br>    entire document | 1-29 |
| A | CN 109971846 A (MEDTIMES GENE DETECTION CENTER INC.) 05 July 2019 (2019-07-05)<br>    entire document | 1-29 |

☑ Further documents are listed in the continuation of Box C.     ☑ See patent family annex.

| *    Special categories of cited documents: | "T"    later document published after the international filing date or priority date and not in conflict with the application but cited to understand the principle or theory underlying the invention |
|---|---|
| "A"    document defining the general state of the art which is not considered to be of particular relevance | |
| "E"    earlier application or patent but published on or after the international filing date | "X"    document of particular relevance; the claimed invention cannot be considered novel or cannot be considered to involve an inventive step when the document is taken alone |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | |
| "P"    document published prior to the international filing date but later than the priority date claimed | "&"    document member of the same patent family |

| Date of the actual completion of the international search | Date of mailing of the international search report |
|---|---|
| **06 January 2022** | **19 January 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| | International application No. |
|---|---|
| | **PCT/CN2021/125359** |

### C. DOCUMENTS CONSIDERED TO BE RELEVANT

| Category* | Citation of document, with indication, where appropriate, of the relevant passages | Relevant to claim No. |
|---|---|---|
| A | WO 2020104394 A1 (SISTEMAS GENOMICOS, S. L.) 28 May 2020 (2020-05-28) entire document | 1-29 |
| A | WO 2017051996 A1 (SK TELECOM CO., LTD.) 30 March 2017 (2017-03-30) entire document | 1-29 |
| A | 杜含笑 等 (DU, Hanxiao et al.). "单基因遗传病无创产前检测的研究与展望 (Non-official translation: Research and Outlook for Noninvasive Prenatal Testing for Monogenic Diseases)" 临床检验杂志 (Chinese Journal of Clinical Laboratory Science), Vol. 36, No. 11, 30 November 2018 (2018-11-30), pp. 805-808 | 1-29 |
| A | 赵馨 等 (ZHAO, Xin et al.). "无创产前诊断在胎儿染色体结构异常中的研究进展 (Non-official translation: Research Advances in Noninvasive Prenatal Diagnosis of Fetal Structural Chromosome Abnormalities)" 中国产前诊断杂志(电子版) (Chinese Journal of Prenatal Diagnosis(Electronic Version)), Vol. 8, No. 4, 31 December 2016 (2016-12-31), pp. 1-4 | 1-29 |
| T | GAO, S. "Noninvasive detection of fetal genetic variations through polymorphic site sequencing of maternal plasma DNA" *THE JOURNAL OF GENE MEDICINE*, 30 November 2021 (2021-11-30), article e3400, pages 1-10 | 1-29 |

Form PCT/ISA/210 (second sheet) (January 2015)

**INTERNATIONAL SEARCH REPORT**
Information on patent family members

International application No.

**PCT/CN2021/125359**

| Patent document cited in search report | | | Publication date (day/month/year) | Patent family member(s) | | | Publication date (day/month/year) |
|---|---|---|---|---|---|---|---|
| WO | 2009013496 | A1 | 29 January 2009 | WO | 2009013492 | A1 | 29 January 2009 |
| | | | | KR | 20190114041 | A | 08 October 2019 |
| | | | | KR | 102128960 | B1 | 02 July 2020 |
| | | | | CA | 3076159 | A1 | 29 January 2009 |
| | | | | CA | 3029497 | A1 | 29 January 2009 |
| | | | | CA | 2694007 | A1 | 29 January 2009 |
| | | | | CA | 2694007 | C | 26 February 2019 |
| | | | | HR | P20160493 | T1 | 15 July 2016 |
| | | | | JP | 2017148073 | A | 31 August 2017 |
| | | | | JP | 6383837 | B2 | 29 August 2018 |
| | | | | KR | 20200100860 | A | 26 August 2020 |
| | | | | IL | 233261 | D0 | 31 August 2014 |
| | | | | IL | 233261 | A | 31 July 2016 |
| | | | | CN | 106834481 | A | 13 June 2017 |
| | | | | CN | 106886688 | A | 23 June 2017 |
| | | | | KR | 20190114039 | A | 08 October 2019 |
| | | | | KR | 102222378 | B1 | 04 March 2021 |
| | | | | HU | E054639 | T2 | 28 September 2021 |
| | | | | CA | 2693081 | A1 | 29 January 2009 |
| | | | | CA | 2693081 | C | 26 January 2016 |
| | | | | CN | 103853916 | A | 11 June 2014 |
| | | | | CN | 107083424 | A | 22 August 2017 |
| | | | | KR | 20100075826 | A | 05 July 2010 |
| | | | | KR | 101646978 | B1 | 09 August 2016 |
| | | | | KR | 20190143494 | A | 30 December 2019 |
| | | | | KR | 102197512 | B1 | 04 January 2021 |
| | | | | SI | 2514842 | T1 | 30 June 2016 |
| | | | | CA | 3009992 | A1 | 29 January 2009 |
| | | | | CA | 3009992 | C | 19 October 2021 |
| | | | | KR | 20100058503 | A | 03 June 2010 |
| | | | | KR | 101916456 | B1 | 07 November 2018 |
| | | | | US | 2009087847 | A1 | 02 April 2009 |
| | | | | US | 8706422 | B2 | 22 April 2014 |
| | | | | HK | 1224033 | A1 | 11 August 2017 |
| | | | | CA | 3076142 | A1 | 29 January 2009 |
| | | | | CA | 3127930 | A1 | 29 January 2009 |
| | | | | HK | 1199067 | A1 | 19 June 2015 |
| | | | | NZ | 600407 | A | 20 December 2013 |
| | | | | DK | 2514842 | T3 | 30 May 2016 |
| | | | | PL | 2514842 | T3 | 31 August 2016 |
| | | | | US | 2009029377 | A1 | 29 January 2009 |
| | | | | CN | 107083425 | A | 22 August 2017 |
| | | | | ES | 2869347 | T3 | 25 October 2021 |
| | | | | HK | 1144024 | A1 | 21 January 2011 |
| | | | | PT | 2183693 | E | 14 January 2014 |
| | | | | EA | 201600280 | A1 | 29 July 2016 |
| | | | | EA | 035451 | B1 | 18 June 2020 |
| | | | | EA | 035451 | B9 | 09 September 2020 |
| | | | | EP | 3892736 | A1 | 13 October 2021 |
| | | | | CN | 103902809 | A | 02 July 2014 |

Form PCT/ISA/210 (patent family annex) (January 2015)

**INTERNATIONAL SEARCH REPORT**
Information on patent family members

International application No.

**PCT/CN2021/125359**

| Patent document cited in search report | | | Publication date (day/month/year) | Patent family member(s) | | | Publication date (day/month/year) |
|---|---|---|---|---|---|---|---|
| US | 2013267425 | A1 | 10 October 2013 | AU | 2018204344 | A1 | 05 July 2018 |
| | | | | AU | 2018204344 | B2 | 27 September 2018 |
| | | | | WO | 2013150503 | A1 | 10 October 2013 |
| | | | | CN | 104640997 | A | 20 May 2015 |
| | | | | US | 2020063207 | A1 | 27 February 2020 |
| | | | | CN | 107841543 | A | 27 March 2018 |
| | | | | US | 2016201134 | A1 | 14 July 2016 |
| | | | | US | 10501797 | B2 | 10 December 2019 |
| | | | | TR | 201815541 | T4 | 21 November 2018 |
| | | | | CA | 2869371 | A1 | 10 October 2013 |
| | | | | CA | 2869371 | C | 12 January 2021 |
| | | | | EP | 2834376 | A1 | 11 February 2015 |
| | | | | EP | 2834376 | A4 | 18 November 2015 |
| | | | | EP | 2834376 | B1 | 15 March 2017 |
| | | | | EP | 3178945 | A1 | 14 June 2017 |
| | | | | EP | 3178945 | B1 | 17 October 2018 |
| | | | | ES | 2623089 | T3 | 10 July 2017 |
| | | | | HK | 1245851 | A1 | 31 August 2018 |
| | | | | HK | 1204658 | A1 | 27 November 2015 |
| | | | | AU | 2013245272 | A1 | 23 October 2014 |
| | | | | AU | 2013245272 | B2 | 05 April 2018 |
| | | | | HK | 1201300 | A1 | 28 August 2015 |
| | | | | JP | 2015521028 | A | 27 July 2015 |
| | | | | JP | 6073461 | B2 | 01 February 2017 |
| CN | 108138226 | A | 08 June 2018 | BR | 112018007710 | A2 | 23 October 2018 |
| | | | | AU | 2016341845 | A1 | 10 May 2018 |
| | | | | IL | 258795 | D0 | 28 June 2018 |
| | | | | EP | 3901279 | A1 | 27 October 2021 |
| | | | | EP | 3901279 | A4 | 27 October 2021 |
| | | | | WO | 2017070096 | A1 | 27 April 2017 |
| | | | | EP | 3362580 | A1 | 22 August 2018 |
| | | | | EP | 3362580 | A4 | 27 March 2019 |
| | | | | EP | 3362580 | B1 | 17 February 2021 |
| | | | | JP | 2019500706 | A | 10 January 2019 |
| | | | | JP | 6858783 | B2 | 14 April 2021 |
| | | | | US | 2018305748 | A1 | 25 October 2018 |
| | | | | RU | 2706203 | C1 | 14 November 2019 |
| | | | | KR | 20180071299 | A | 27 June 2018 |
| CN | 103849684 | A | 11 June 2014 | WO | 2009013492 | A1 | 29 January 2009 |
| | | | | KR | 20190114041 | A | 08 October 2019 |
| | | | | KR | 102128960 | B1 | 02 July 2020 |
| | | | | CA | 3076159 | A1 | 29 January 2009 |
| | | | | CA | 3029497 | A1 | 29 January 2009 |
| | | | | CA | 2694007 | A1 | 29 January 2009 |
| | | | | CA | 2694007 | C | 26 February 2019 |
| | | | | HR | P20160493 | T1 | 15 July 2016 |
| | | | | JP | 2017148073 | A | 31 August 2017 |
| | | | | JP | 6383837 | B2 | 29 August 2018 |
| | | | | KR | 20200100860 | A | 26 August 2020 |
| | | | | IL | 233261 | D0 | 31 August 2014 |

Form PCT/ISA/210 (patent family annex) (January 2015)

International application No.

**PCT/CN2021/125359**

| Patent document cited in search report | | | Publication date (day/month/year) | Patent family member(s) | | | Publication date (day/month/year) |
|---|---|---|---|---|---|---|---|
| | | | | IL | 233261 | A | 31 July 2016 |
| | | | | CN | 106834481 | A | 13 June 2017 |
| | | | | CN | 106886688 | A | 23 June 2017 |
| | | | | KR | 20190114039 | A | 08 October 2019 |
| | | | | KR | 102222378 | B1 | 04 March 2021 |
| | | | | HU | E054639 | T2 | 28 September 2021 |
| | | | | CA | 2693081 | A1 | 29 January 2009 |
| | | | | CA | 2693081 | C | 26 January 2016 |
| | | | | CN | 103853916 | A | 11 June 2014 |
| | | | | CN | 107083424 | A | 22 August 2017 |
| | | | | KR | 20100075826 | A | 05 July 2010 |
| | | | | KR | 101646978 | B1 | 09 August 2016 |
| | | | | KR | 20190143494 | A | 30 December 2019 |
| | | | | KR | 102197512 | B1 | 04 January 2021 |
| | | | | SI | 2514842 | T1 | 30 June 2016 |
| | | | | CA | 3009992 | A1 | 29 January 2009 |
| | | | | CA | 3009992 | C | 19 October 2021 |
| | | | | KR | 20100058503 | A | 03 June 2010 |
| | | | | KR | 101916456 | B1 | 07 November 2018 |
| | | | | US | 2009087847 | A1 | 02 April 2009 |
| | | | | US | 8706422 | B2 | 22 April 2014 |
| | | | | HK | 1224033 | A1 | 11 August 2017 |
| | | | | CA | 3076142 | A1 | 29 January 2009 |
| | | | | CA | 3127930 | A1 | 29 January 2009 |
| | | | | HK | 1199067 | A1 | 19 June 2015 |
| | | | | NZ | 600407 | A | 20 December 2013 |
| | | | | DK | 2514842 | T3 | 30 May 2016 |
| | | | | PL | 2514842 | T3 | 31 August 2016 |
| | | | | US | 2009029377 | A1 | 29 January 2009 |
| | | | | CN | 107083425 | A | 22 August 2017 |
| | | | | ES | 2869347 | T3 | 25 October 2021 |
| | | | | HK | 1144024 | A1 | 21 January 2011 |
| | | | | PT | 2183693 | E | 14 January 2014 |
| | | | | EA | 201600280 | A1 | 29 July 2016 |
| | | | | EA | 035451 | B1 | 18 June 2020 |
| | | | | EA | 035451 | B9 | 09 September 2020 |
| | | | | EP | 3892736 | A1 | 13 October 2021 |
| | | | | CN | 103902809 | A | 02 July 2014 |
| CN | 111951890 | A | 17 November 2020 | None | | | |
| CN | 109971846 | A | 05 July 2019 | US | 2019338362 | A1 | 07 November 2019 |
| WO | 2020104394 | A1 | 28 May 2020 | UY | 38479 | A | 30 June 2020 |
| | | | | EP | 3884502 | A1 | 29 September 2021 |
| WO | 2017051996 | A1 | 30 March 2017 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LO, CORBETTA et al.** *Lancet,* 1997, vol. 350, 485-487
- **ADVANI, BARRETT et al.** *Prenat Diagn,* 2017, vol. 37, 1067-1075
- **BREVEGLIERI, D'AVERSA et al.** *Mol Diagn Ther,* 2019, vol. 23, 291-299
- **ANDARI ; BUSSAMRA et al.** *Ceska Gynekol,* 2020, vol. 85, 41-48
- **GUSEH.** *Hum Genet,* 2020, vol. 139, 1141-1148
- **CHIU ; CHAN et al.** *Proc Natl Acad Sci U S A,* 2008, vol. 105, 20458-20463
- **FAN ; BLUMENFELD et al.** *Proc Natl Acad Sci U S A,* 2008, vol. 105, 16266-16271
- **LIAO ; CHAN et al.** *PLoS One,* 2012, vol. 7, e38154
- **ZIMMERMANN ; HILL et al.** *Prenat Diagn,* 2012, vol. 32, 1233-1241
- **ADVANI ; BARRETT et al.** *Prenat Diagn,* 2017, vol. 37, 1067-1075
- **HU ; WANG et al.** *Human Genomics,* 2019, vol. 13, 14
- **SREBNIAK ; KNAPEN et al.** *Mol Genet Genomic Med,* 2020, vol. 8, e1062
- **LUN ; TSUI et al.** *Proc Natl Acad Sci U S A,* 2008, vol. 105, 19920-19925
- **LO ; CHAN et al.** *Sci Transl Med,* 2010, vol. 2, 61-91
- **LV, WEI et al.** *Clinical Chemistry,* 2015, vol. 61, 172-181
- **VERMEULEN ; GEEVEN et al.** *Am J Hum Genet,* 2017, vol. 101, 326-339
- **ALLEN, YOUNG et al.** *Noninvasive Prenatal Testing (NIPT),* 2018, 157-177
- **YIN ; DU et al.** *J Hum Genet,* 2018, vol. 63, 1129-1137
- **CUTTS ; VAVOULIS et al.** *Blood,* 2019, vol. 134, 1190-1193
- **ZHANG ; LI et al.** *Nat Med,* 2019, vol. 25, 439-447
- **KIM ; KIM et al.** *Nat Commun,* 2019, vol. 10, 1047
- **HUANG ; LI et al.** *Bioinformatics,* 2012, vol. 28, 593-594
- **BARRETT ; XIONG et al.** *PLoS One,* 2017, vol. 12, e0186771